# EUROPEAN PATENT APPLICATION

(11) **EP 3 569 246 A1**
(43) Date of publication of application: **20.11.2019**
(21) Application number: 18020211.1
(22) Date of filing: 16.05.2018
(51) Int. Cl.: A61K 41/00

(54) **MAGNETIC NANOPARTICLES SEQUENTIALLY IRRADIATED BY LASER RADIATION FOR MEDICAL OR COSMETIC APPLICATIONS**

(71) Applicant: Nanobacterie, 75116 Paris (FR)
(72) Inventor: Alphandéry, Edouard, 75116 PARIS (FR)

(57) **Abstract**

This invention relates magnetosomes for use in a laser radiation medical treatment, wherein the magnetosomes are administered to a body part of an individual and:
- In a first step, the magnetosomes are irradiated by a laser radiation, and
- In a second step, the magnetosomes are irradiated by a laser radiation of lower power than in the first step or no laser i rradi ati on of the magnetosomes is performed, and
the sequence of the first step and second step is repeated at least once.

## Description

### FIELD OF THE INVENTION

The invention relates to magnetic nanoparticles, in particular magnetosomes, being introduced to a body part of an individual and then irradiated sequentially by a laser radiation, leading to some improvement compared with a method in which magnetic nanoparticles, in particular magnetosomes, are continuously irradiated by laser radiation.

### STATE OF THE ART

Magnetosomes have been shown to efficiently destroy tumors when they are introduced to tumors and heated under the application of an alternating magnetic field (E. Alphandéry et al, ACSNano, V. 5, P. 6279-6296, 2011). Magnetosomes were also shown to be more efficient than their chemically synthesized counterparts, due to their better crystallization, larger sizes, and/or chain arrangement. It appears however that the application of the alternating magnetic field can be difficult to achieve since it requires the use of a large and expensive induction system in order to reach a sufficiently high strength to heat magnetosomes in the body part.

In this patent application, we therefore develop a method to heat the magnetic nanoparticles, in particular magnetosomes, with laser radiation, using a more compact and cheaper excitation source (a laser) than an induction system. Moreover, by sequentially irradiating magnetic nanoparticles, in particular magnetosomes, by laser radiation, we are able to increase the efficacy and/or reduce the toxicity of the treatment.

### DESCRIPTION OF THE INVENTION

The invention relates to magnetic nanoparticles, in particular magnetosomes, for use in a laser radiation medical treatment, wherein the magnetic nanoparticles, in particular the magnetosomes, are administered to a body part of an individual and:
- In a first step, the magnetic nanoparticles, in particular the magnetosomes, are irradiated by a laser radiation, and
- In a second step, the magnetic nanoparticles, in particular the magnetosomes, are irradiated by a laser radiation of lower power than in the first step or no laser irradiation of the magnetic nanoparticles, in particular the magnetosomes, is performed, and
the sequence of the first step and the second step is repeated at least once.

The invention relates to a method for treating an individual by laser radiation, comprising administering an effective amount of magnetosomes to a body part of the individual and:
- In a first step, irradiating the magnetic nanoparticles, in particular the magnetosomes, with a laser radiation, and
- In a second step, irradiating the magnetic nanoparticles, in particular the magnetosomes, by a laser radiation of lower power that in the first step, or performing no laser irradiation of the magnetosomes, wherein the sequence of the first step and the second step is repeated at least once.

In one embodiment of the invention, magnetic nanoparticle(s), in particular magnetosome(s), or the magnetic nanoparticle(s), in particular the magnetosome(s), represent(s) an assembly comprising more than 1, 10, 10², 10³, 10⁵, 10¹⁰, 10²⁰, or 10⁵⁰ magnetic nanoparticle(s), in particular magnetosome(s).

In still another embodiment of the invention, magnetic nanoparticle(s), in particular magnetosome(s), or the magnetic nanoparticles, in particular the magnetosome(s), represent(s) an assembly comprising less than 1, 10, 10², 10³, 10⁵, 10¹⁰, 10²⁰, or 10⁵⁰ magnetic nanoparticle(s), in particular magnetosome(s).

In still another embodiment of the invention, magnetic nanoparticle(s), in particular magnetosome(s), or the magnetic nanoparticle(s), in particular the magnetosome(s), represent(s) an assembly comprising more than 10⁻⁵⁰, 10⁻⁴⁰, 10⁻³⁰, 10⁻²⁰, 10⁻¹⁰, 10⁻⁵, 10⁻³, 1, or 10² mg of: i), magnetic nanoparticle(s), in particular magnetosome(s), or, ii), mg of iron comprised in magnetic nanoparticle(s), in particular magnetosome(s), or, iii), mg of magnetic nanoparticle(s), in particular magnetosome(s), per cm³ or, iv), mg of magnetic nanoparticle(s), in particular magnetosome(s), per cm³ of body part or, v), mg of iron comprised in magnetic nanoparticle(s), in particular magnetosome(s), per cm³ or, vi), mg of iron comprised in magnetic nanoparticle(s), in particular magnetosome(s), per cm³ of body part.

In still another embodiment of the invention, magnetic nanoparticle(s), in particular magnetosome(s), or the magnetic nanoparticle(s), in particular the magnetosome(s), represent(s) an assembly comprising less than 10⁻⁵⁰, 10⁻⁴⁰, 10⁻³⁰, 10⁻²⁰, 10⁻¹⁰, 10⁻⁵, 10⁻³, 1, or 10² mg of : i), magnetic nanoparticle(s), in particular magnetosome(s), or, ii), mg of iron comprised in magnetic nanoparticle(s), in particular magnetosome,(s), or, iii), mg of magnetic nanoparticle(s), in particular magnetosome(s), per cm³ or, iv), mg of magnetic nanoparticle(s), in particular magnetosome(s), per cm³ of body part, or, v), mg of iron comprised in magnetic nanoparticle(s), in particular magnetosome(s), per cm³ or, vi), mg of iron comprised in magnetic nanoparticle(s), in particular magnetosome(s), per cm³ of body part.

In some cases, magnetic nanoparticle(s), in particular magnetosome(s), or the magnetic nanoparticle(s), in particular the magnetosome(s), can be, represent, be associated with, correspond to, the magnetic nanoparticles, in particular magnetosomes, bound to, linked to, associated with the compound as defined in this invention.

In one embodiment of the invention, the magnetic nanoparticles, in particular magnetosomes, are nanoparticles characterized by at least one of the following properties: i), the presence of a core, preferentially magnetic, preferentially mineral, preferentially composed of iron oxide, most preferentially composed of maghemite or magnetite, or an intermediate composition between maghemite and magnetite, ii), the presence of a coating that surrounds the core of the magnetic nanoparticle, in particular magnetosome, and preferentially prevents aggregation of the magnetic nanoparticle, in particular magnetosome, preferentially enabling the administration in an organism or in the body part of the magnetic nanoparticle, in particular the magnetosome, or stabilizing the core of the magnetic nanoparticle, in particular the magnetosome, where coating thickness may preferably lie between 0.1 nm and 10 µm, between 0.1 nm and 1 µm, between 0.1 nm and 100 nm, between 0.1 nm and 10 nm, or between 1 nm and 5 nm, iii), magnetic properties leading to diamagnetic, paramagnetic, superparamagnetic, ferromagnetic, or ferrimagnetic behavior or properties, iv), a coercivity larger than 0.01, 0.1, 1, 10, 100, 10³, 10⁴, 10⁵, 10⁹, or 10²⁰ Oe, v), a ratio between remanent and saturating magnetization larger than 0.01, 0.1, 0.2, 0.3, 0.4, 0.5, 0.75, 0.9, or 0.99, vi), a saturating magnetization larger than 0.1, 1, 5, 10, or 50 emu/g, vii), magnetic properties such as coercivity, remanent and saturating magnetization, preferentially measured or observed at a temperature larger than 0.1 K, 1 K, 10 K, 20 K, 50 K, 100 K, 200 K, 300 K, 350 K, or 3000 K, viii), a crystallinity, *i.e.* magnetic nanoparticles, in particular magnetosomes, preferentially possessing at least 1, 2, 5, 10, or 100 crystalline plane(s), preferentially observable or measured by electron microscopy, ix), the presence of a single domain, x), a size that is larger than 0.1, 0.5, 1.5, 10, 15, 20, 25, 30, 50, 60, 70, 80, 100, 120, 150, or 200 nm, xi), a size lying between 0.1 nm and 10 µm, between 0.1 nm and 1 µm, between 0.1 nm and 100 nm, between 1 nm and 100 nm, or between 5 nm and 80 nm, xii), a non-pyrogenicity or apyrogenicity, which preferentially means that magnetic nanoparticles, in particular magnetosomes, possess an endotoxin concentration lower than 10000, 1000, 100, 50, 10, 5, 2, or 1 EU (endotoxin unit) per mg of magnetic nanoparticle, in particular magnetosome, or per mg of iron comprised in magnetic nanoparticle, in particular magnetosome, or which means that magnetic nanoparticles, in particular magnetosomes, do not trigger fever or do not trigger an increase in whole body temperature of an organism individual of more than 100, 50, 10, 6, 5, 3, 2, or 1 °C, preferentially following administration of magnetic nanoparticles, in particular magnetosomes, to a living organism or body part, xiii), a synthesis by a synthetizing living organism, preferentially by magnetotactic bacteria, leading to the production of magnetic nanoparticles, in particular magnetosomes, preferentially extracted from magnetotactic bacteria, preferentially only or mostly or in majority comprising the mineral magnetic core of the magnetic nanoparticles, in particular the magnetosomes, xiv), the presence of less than 50, 25, 15, 10, 5, 2, or 1% of organic or carbon material originating from the synthetizing living organism, xv), a cubic, spherical, cubo-octahedral, cigar-shaped, or elongated geometry, xvi), a ratio between length(s) and width(s), between the longest and largest edge(s) of the magnetic nanoparticles, in particular magnetosomes, or between two different edge(s), dimension(s), preferentially crossing dimension(s), or diameter(s) of the magnetic nanoparticles, in particular magnetosomes, that is/are comprised between 10⁻³ and 10³, 10⁻² and 10², 10⁻¹ and 10, 0.5 and 5, 0.2 and 2, or between 0.1 and 1.1, or xvii), the presence of more than 99, 95, 80, 70, 60, 50, or 25% of mineral material originating from the synthetizing living organism, or xvi), a specific absorption rate (SAR) that is larger than 1, 10, 1000, or 10⁴ Watt per gram of magnetic nanoparticle, in particular magnetosome, where the SAR is preferentially measured under the irradiation by the laser radiation.

In another embodiment of the invention, the magnetic nanoparticles, in particular magnetosomes, are nanoparticles characterized by at least one of the following properties: i), a coercivity lower than 0.01, 0.1, 1, 10, 100, 10³, 10⁴, 10⁵, 10⁹, or 10²⁰ Oe, ii), a ratio between remanent and saturating magnetization lower than 0.01, 0.1, 0.2, 0.3, 0.4, 0.5, 0.75, 0.9, or 0.99, iii), a saturating magnetization lower than 0.1, 1, 5, 10, 50, 200, 1000, or 5000 emu/g, iv), magnetic properties preferentially measured or observed at a temperature lower than 0.1 K, 1 K, 10 K, 20 K, 50 K, 100 K, 200 K, 300 K, 350 K, or 3000 K, viii), a size that is lower than 0.1, 0.5, 1.5, 10, 15, 20, 25, 30, 50, 60, 70, 80, 100, 120, 150, or 200 nm, ix), the presence of more than 50, 25, 15, 10, 5, 2, or 1% of organic or carbon material originating from the synthetizing living organism, x), the presence of less than 99, 95, 80, 70, 60, 50, or 25% of mineral material originating from the synthetizing living organism, or xi), a specific absorption rate (SAR) that is lower than 1, 10, 1000, or 10⁴ Watt per gram of magnetic nanoparticle, in particular magnetosome, preferentially measured under the irradiation by the laser radiation.

In some cases, it can be necessary to image the body part, preferentially to follow the evolution or growth of the body part following the treatment, using an imaging technique such as magnetic resonance imaging (MRI), computing tomography (CT), scanner, positron emission tomography (PET), radiography, or echography.

In some cases, the composition of the magnetic nanoparticle, in particular magnetosome, can prevent efficient imaging of the body part.

In some other cases, the concentration of the magnetic nanoparticle, in particular magnetosome, can be too large to enable efficient imaging of the body part.

In still some other cases, the magnetic nanoparticle, in particular magnetosome, can act like a screen or hide the body part and can prevent efficient imaging of the body part.

In one embodiment of the invention, the composition of the magnetic nanoparticle, in particular the magnetosome, is adjusted or changed to enable imaging of the body part. In some cases, the iron oxide composition is replaced by a composition comprising another substance selected among the families of lithium, beryllium, scandium, titanium, vanadium, chromium, manganese, iron, nickel, copper, zinc, boron, carbon, nitrogen, oxygen, fluorine, or helium, or among alkali metals, alkaline earth metals, coinage metals, triels, tetrela, pentels, pnictogens, chalcogens, halogens, or nobel gases.

In another embodiment of the invention, the concentration of the magnetic nanoparticle, in particular magnetosome, is decreased, preferentially below 10⁻²⁰, 10⁻⁹, 10⁻⁶, 10⁻³, 10⁻¹, 1, 10, 10³, 10⁶, 10⁹, or 10²⁰ mg per cm³ or mg per cm³ of body part, to enable efficient imaging of the body part.

In still another embodiment of the invention, the concentration of the magnetic nanoparticle, in particular magnetosome, is comprised between a minimum value and a maximum value.

In some cases, the minimum value is sufficiently large to enable the production of heat by the magnetic nanoparticle, in particular magnetosome, or the dissociation of the compound from the magnetic nanoparticle, in particular magnetosome, preferentially under the irradiation by the laser radiation. This minimum value can in some cases be larger than 10⁻²⁰, 10⁻⁹, 10⁻⁶, 10⁻³, 10⁻¹, 1, 10, 10³, 10⁶, 10⁹, or 10²⁰ mg per cm³ or mg per cm³ of body part.

In some other cases, the maximum value is sufficiently low to enable imaging of the body part. It can in some cases be larger than 10⁻²⁰, 10⁻⁹, 10⁻⁶, 10⁻³, 10⁻¹, 1, 10, 10³, 10⁶, 10⁹, or 10²⁰ mg per cm³ or mg per cm³ of body part.

In one embodiment of the invention, the SAR of the magnetic nanoparticle, in particular magnetosome, can be designated as SAR_{M} or SARᵣₑₐₗ, as defined later in this invention.

In one embodiment of the invention, the magnetic nanoparticles, in particular magnetosomes, are arranged in chains comprising more than 1, 2, 3, 4, 5, 6, 7, 8, 9, 10, 15, 20, 25, 30, 35, or 40 magnetic nanoparticle(s), in particular magnetosome(s). Magnetic nanoparticle(s), in particular magnetosome(s), can be arranged in chains inside magnetotactic bacteria or outside magnetotactic bacteria, preferentially after their extraction or isolation from magnetotactic bacteria.

In one embodiment of the invention, magnetic nanoparticles, in particular magnetosomes, are not arranged in chains.

In one embodiment of the invention, magnetic nanoparticles, in particular magnetosomes, are purified to remove more than 10, 50, or 90% of endotoxins and/or other biological or carbonaceous material such as proteins and lipids originating from magnetotactic bacteria. Such purification can use detergent such as NaOH or KOH. Purified magnetic nanoparticles, in particular magnetosomes, can be recoated with a synthetic coating such as a substance comprising a function selected in the group comprising carboxylic acids, phosphoric acids, sulfonic acids, esters, amides, ketones, alcohols, phenols, thiols, amines, ether, sulfides, acid anhydrides, acyl halides, amidines, amides, nitriles, hydroperoxides, imines, aldehydes, or peroxides. The coating can be made of carboxy-methyl-dextran, citric acid, phosphatidylcholine (DOPC), or oleic acid. In some cases, the coating can enable the dispersion of the magnetic nanoparticles, in particular magnetosomes, in a matrix or solvent such as water. Purified magnetic nanoparticles, in particular magnetosomes, (recoated or not) are preferentially non-pyrogenic. They preferentially comprise less than 10⁸, 10⁵, 10³, or 10 EU (endotoxin unit) per: i), mm³ or, ii), mL of magnetic nanoparticles, in particular magnetosomes, iii), mL of magnetic nanoparticle, in particular magnetosome, suspension, or iv), mL or cm³ of body part. Purified magnetic nanoparticles, in particular magnetosomes, (recoated or not) can be re-suspended in a liquid or re-dispersed in a matrix to result in a homogenous dispersion or in a high stability.

In one embodiment of the invention, a suspension of magnetic nanoparticles, in particular magnetosomes, is stable, which preferentially means that it is stable at a concentration larger than 1, 5, 10, 50, 100, 200, 500, or 1000 mg of magnetic nanoparticles, in particular magnetosomes, per mL of solvent, *i.e.* the optical density of this suspension, preferentially measured at 480 nm or another fixed wavelength, does not decrease by more than 1, 5, 10, 50, 75, or 90 %, preferentially within more than 1,5, 10, 10³, 10⁷, or 10²⁰ second(s) following homogenization or mixing of this suspension.

In another embodiment of the invention, an assembly of magnetic nanoparticles, in particular magnetosomes, is homogenously distributed. This preferentially means that magnetic nanoparticles, in particular magnetosomes, occupy more than 10⁻⁶, 10⁻³, 10⁻¹, 1, 5, 10, 50, or 75 % of the volume in which magnetic nanoparticles, in particular magnetosomes, are administered, mixed, inserted, or introduced. This percentage preferentially represents the ratio between the volume of the body part, in which magnetic nanoparticles, in particular magnetosomes, are administered, mixed, inserted, or introduced, measured before magnetic nanoparticle, in particular magnetosome, administration, mixing, insertion, or introduction, to/in the body part divided by the volume of the body part occupied by the magnetic nanoparticles, in particular magnetosomes, measured after magnetic nanoparticle, in particular magnetosome, administration, mixing, insertion, or introduction, to/in the body part, preferentially measured less than 10⁵⁰, 10²⁰, 10¹⁰, 10⁵, 10², 10, or 1 minute(s) following magnetic nanoparticle, in particular magnetosome, administration, mixing, insertion, or introduction to/in the body part.

In one embodiment of the invention, magnetic nanoparticles, in particular magnetosomes, are synthesized by a living organism, designated as synthetizing living organism, which consists or comprises at least 1, 2, 5, 10, 10³, 10⁶, or 10⁹ eukaryotic or prokaryotic cell(s).

In one embodiment of the invention, magnetic nanoparticles, in particular magnetosomes, are synthesized intracellularly, *i.e*. inside an eukaryotic or prokaryotic cell.

In another embodiment of the invention, magnetic nanoparticles, in particular magnetosomes, are synthesized extracellularly, *i.e.* outside an eukaryotic or prokaryotic cell.

In still another embodiment of the invention, magnetic nanoparticles, in particular magnetosomes, are synthesized or produced or crystallized or assembled or transformed into a nanoparticle by a living organism, a cell, compartment, organelle, or other biological material, such as protein, lipid, enzyme, DNA, or RNA, which is preferentially produced by or originates from an eukaryotic or prokaryotic cell. In some other cases, a chemical synthesis can be used to produce a chemical substance or compound that mimics, copies, or reproduces the compartment, organelle, or other biological material, wherein this chemical synthesis or chemical substance can be used or can result in the production of the magnetic nanoparticle, in particular the magnetosome. In some cases, the compartment, organelle, or other biological material, can be a lysosome, an endosome, a vesicle, preferentially biological material that has the capacity or the function either to dissolve or transform crystallized iron into free iron or to transform free iron into crystalized iron. In some cases, this transformation is partial and preferentially results in the destruction or formation of partly crystallized assembly of iron atoms or ions, or preferentially results in a mixture of crystallized iron and non-crystallized iron. In some cases, crystallized iron can be defined as an assembly of iron atoms or ions that leads to the presence of crystallographic planes, preferentially observable using a technique such as transmission or scanning electron microscopy as a characterization method, and free iron can preferentially be defined as one of several iron atoms or ions that do not lead to the presence of crystallographic planes, preferentially highlighted by the absence of diffraction patterns, using for example transmission or scanning electron microscopy as a characterization method.

In one embodiment of the invention, magnetic nanoparticles, in particular magnetosomes, are synthesized by a living organism when at least 1, 2, 5, 10 or 100 step(s) of their production, such as crystallization of iron oxide, stabilization of the iron oxide mineral, organization of the minerals of the magnetic nanoparticles, in particular magnetosome, for example in chains or aggregates, involves or is due to a living organism, a cell, compartment, organelle, or other biological material, such as protein, lipid, enzyme, DNA, or RNA, which is preferentially produced by or originates from an eukaryotic or prokaryotic cell. In this case, this can mean that magnetic nanoparticles, in particular magnetosomes, are not synthesized chemically or are different from chemical nanoparticles.

In another embodiment of the invention, magnetic nanoparticles, in particular magnetosomes, are not synthesized by a living organism when less than 1, 2, 5, 10 or 100 step(s) of their production, such as crystallization of iron oxide, stabilization of the iron oxide mineral, organization of the minerals of magnetic nanoparticles, in particular magnetosomes, for example in chains or aggregates, involves or is due to a living organism, a cell, compartment, organelle, or other biological material, such as protein, lipid, enzyme, DNA, or RNA, which is preferentially produced by or originates from an eukaryotic or prokaryotic cell. In this case, this can mean that magnetic nanoparticles, in particular magnetosomes, are synthesized chemically or are the same as or are similar to chemical nanoparticles. In one embodiment of the invention, magnetic nanoparticles, in particular magnetosomes, are synthesized by a living organism or prokaryotic cell, which is preferentially a bacterium, most preferentially a magnetotactic bacterium such as such as *Magnetospirillum magneticum* strain AMB-1, *magnetotactic coccus* strain MC-1, three facultative anaerobic vibrios strains MV-1, MV-2 and MV-4, the *Magnetospirillum magnetotacticum* strain MS-1, the *Magnetospirillum gryphiswaldense* strain MSR-1, a facultative anerobic magnetotactic spirillum, *Magnetospirillum magneticum* strain MGT-1, and an obligate anaerobe, *Desulfovibrio magneticus* RS-1.

In some cases, a magnetotactic bacterium can be defined as an organism that is able to produce a magnetic nanoparticle, in particular magnetosome. In some cases, the magnetotactic bacterium can use the magnetic nanoparticle, in particular magnetosome, to swim in the direction of the earth magnetic field.

In one embodiment of the invention, a magnetotactic bacterium is defined as a bacterium able to synthesize magnetic nanoparticles, in particular magnetosomes, wherein these magnetic nanoparticles, in particular magnetosomes, are preferentially characterized by at least one of the following properties: i), they are produced intracellularly, ii), they are magnetic, iii), they comprise a mineral, iv), their core is preferentially composed of a metallic oxide such as iron oxide, v), their core is surrounded by biological material such as lipids, proteins, endotoxins, which is preferentially removed, most preferentially removed by another organism than the magnetotactic bacterium such as a human, v), they are arranged in chains, or vi), they produce heat under the application of a laser or irradiation by a laser.

In one embodiment of the invention, the magnetic nanoparticle, in particular the magnetosome, comprises the mineral part synthesized by magnetotactic bacteria, *i.e.* preferentially the crystallized iron oxide produced by these bacteria. In this case, magnetic nanoparticles, in particular magnetosomes, or the mineral parts of magnetic nanoparticles, in particular magnetosomes, preferentially do not comprise proteins, lipids, endotoxins, or biological materials comprising carbon or carbonaceous material, or more than 0.1, 1, 10, 30, 50, or 75% of carbon, which is/are produced by these bacteria.

In one embodiment of the invention, the magnetic nanoparticle, in particular magnetosome, comprises the mineral part synthetized by magnetotactic bacteria and a substance, which is a carbonaceous biological material synthesized by a magnetotactic bacterium. Such substance can preferentially be used in the laser medical treatment. It may trigger an immune response, preferentially against pathological cells, or target the pathological cells or have a pharmacologic or metabolic or cosmetic effect. When such substance comprises toxic carbonaceous material synthesized by a magnetotactic bacterium, such as endotoxins or other types of immunogenic or toxic proteins, lipids, DNA, RNA, synthesized by these bacteria, such substance is preferentially removed or destroyed, most preferentially selectively to avoid the removal of other substances, which are preferentially non-toxic and/or of interest or potential efficacy for/in the laser medical treatment. When such substance comprises carbonaceous material synthesized by a magnetotactic bacterium, which can be used in the laser medical treatment, which preferentially targets or destroys pathological cells, which is preferentially non-toxic towards healthy cells, such substance is preferentially maintained in/on the magnetic nanoparticle, in particular magnetosome, or at the magnetic nanoparticle, in particular magnetosome, surface or is bound or linked or associated to/with the magnetic nanoparticle, in particular magnetosome,, preferentially before or without the irradiation by the laser radiation, preferentially before the administration of the magnetic nanoparticle, in particular magnetosome, in/to the body part.

In some cases, the substance described in the previous embodiment is the compound.

In one embodiment of the invention, pathological cells are cells that are old, preferentially older than 10⁻⁹, 10⁻⁶, 10⁻³, 1, 10³, 10⁶ minutes, or cells that belong to an old individual, preferentially cells that belong to an individual older than 1, 5, 10, 25, 50, 75, 80, 90, or 100 years, or cells that have entered into an apoptotic or necrotic state, or cells that have a larger or smaller size than a healthy cell, preferentially by a factor of more than 1.1, 1.2, 1.5, 2, 5, 10, 10³, or cells that possess organelle(s) that do(es) not work properly, mitochondria producing less energy than they do in a healthy cell, or ribosome linking more or less amino acids than they do in a healthy cell, or cellular membrane not enabling endocytosis or trans-membrane diffusion as they usually do in a healthy cell.

In another embodiment of the invention, pathological cells are cells that lead to an ugly or unusual appearance, where an unusual appearance can be defined as an appearance or condition that is different from that of 1, 5, 10, 50, 75, or 90 % of all individuals or of a representative sample of the whole population. An unusual appearance can also be associated to a skin disease, to the presence of plaque, redness, pimple, itch, or blister, on the skin.

In still some other cases, pathological cells can be defined as cells that are responsible for diseases, such as cancer, malfunction of the body part, or cells that lead to the death of an individual or to fever or to an increase in the temperature of the individual, preferentially by more than 0.1, 1, 2, 3, 4, 5, 10, 50, or 100 °C, preferentially above the physiological temperature, preferentially within a whole individual or a portion of an individual. In still some other cases, pathological cells can be defined as cells that do not divide at a normal speed, or cells that divide at a speed which is 1.1, 2, 5, 10, 10³, 10⁶, or 10⁹ larger than the speed of division of a healthy cell. In still some other cases, pathological cells are defined by their presence in large quantity in the body part, by the presence of more than 1, 10, 10³, 10⁶, 10⁹, or 10²⁰ pathological cells per cm³ of body part. In still some other cases, pathological cells are defined by their increasing number, preferentially by a factor of more than 1.1, 10, 10³, 10⁶, 10⁹, or 10²⁰ between a time considered before the individual is suffering from a disease, preferentially less than 1, 2, 10, 10³, 10⁶, 10⁹, or 10⁴⁰ hours before the individual is suffering from a disease, preferentially the disease responsible for the appearance of the pathological cells, and a time after or during the disease of the individual, preferentially more than 1, 2, 10, 10³, 10⁶, 10⁹, or 10⁴⁰ hours after the starting time of the disease of the individual.

In one embodiment of the invention, pathological cells are bacteria, preferentially pathological bacteria, tumor cells, preferentially benign or malign tumor cells.

In one embodiment of the invention, pathological cells can be defined as cells that are in contact, in interaction, with foreign material not belonging to the individual, such as viruses. In some cases, pathological cells can be defined as cells in which viruses have penetrated, or are replicating. In some cases, pathological cells can be assimilated to viruses or to other organisms or entities that colonize cells or target cells or destroy cells or use cells or enter in interaction with cells, preferentially either to enable their own reproduction, multiplication, survival, or death.

In one embodiment of the invention, a pathological cell is defined as a healthy cell that has undergone a transformation, modification, or a healthy cell that is dead, preferentially due to the presence of a virus or to other organisms or entities that colonize cells or use cells or enter in interaction with cells for their own reproduction, multiplication, or survival.

In one embodiment of the invention, the presence in the body part of viruses or of other organisms or entities that colonize cells or use cells or enter in interaction with cells for their reproduction, multiplication, or survival, can be used to deduce the presence of pathological cells in the body part or is responsible for the presence of pathological cells in the body part.

In one embodiment of the invention, the magnetic nanoparticles, in particular the magnetosomes, are assimilated to chemical nanoparticles, *i.e.* to nanoparticles that have not been synthesized by the synthetizing living organism, most preferentially by a magnetotactic bacterium, but possess at least 1, 2, 3, 4, 5, 6, 7, 8, 9, or 10 common property(ies) with the magnetosomes, where these common properties are preferentially a ferrimagnetic behavior, a large size, a crystallization, a mono-domain, a maghemite or magnetite composition, a chain arrangement, a cubic, spherical, cubo-octahedral, cigar-shaped, or elongated geometry, or a ratio between two different dimensions of the magnetosomes that is comprised between 10⁻³ and 10³.

In one embodiment of the invention, the magnetic nanoparticles can be superparamagnetic or ferrimagnetic nanoparticles, where the magnetic properties of the magnetic nanoparticles are preferentially measured at a temperature larger than 0.1, 1, 5, 10, 50, 100, 200, 300, or 400 K. In some cases, they can be comprised of a metal or metal oxide or of a majority of metallic atoms or of metallic and oxygen atoms. In some other cases, they can be comprised of iron oxide such as maghemite or magnetite.

In one embodiment of the invention, magnetic nanoparticles, in particular magnetosomes, are defined by at least one of the properties that result from the application of the laser on them, such as duration of the first or second step, maximum or minimum temperature or maximum or minimum percentage of dissociation, resulting from the sequential irradiation by the laser radiation of the magnetic nanoparticle, in particular magnetosome, or body part.

In some cases, the irradiation by the laser or laser radiation of the magnetosomes can mean that the magnetosomes are irradiated by the laser radiation.

In one embodiment of the invention, the irradiation by the laser radiation is or is due to an electromagnetic wave. The laser radiation can be an electromagnetic wave. The electromagnetic wave or laser radiation is preferentially emitted or generated by a laser equipment or apparatus. The electromagnetic wave is preferentially associated with: i), the propagation of electric and magnetic field waves, ii), waves that do not involve the movement of particles with a mass, or iii), waves comprising photons, or iv), light waves. Preferentially, the magnetic field produced by the electromagnetic wave has a strength lower than 10¹⁰, 10⁵, 10², 10, 1, 10⁻¹, 10⁻², 10⁻⁵, or 10⁻¹⁰ T. Preferentially, the electric field produced by the electromagnetic field has a strength lower than 10¹⁰, 10⁵, 10², 10, 1, 10⁻¹, 10⁻², 10⁻⁵, or 10⁻¹⁰ V.m⁻¹. In some cases, the electromagnetic wave oscillates in space or time at a frequency that is lower than 10¹⁰, 10⁵, 10³, 10, 1, 10⁻¹, 10⁻³, 10⁻⁵, or 10⁻¹⁰ GHz. In some other cases, the electromagnetic wave oscillates in space or time at a frequency that is larger than 10¹⁰, 10⁵, 10³, 10, 1, 10⁻¹, 10⁻³, 10⁻⁵, or 10⁻¹⁰ GHz. In some cases, the laser radiation is monochromatic, *i.e.* it comprises photons with a single wavelength. In some other cases, the laser radiation is polychromatic, *i.e.* it comprises photons with multiple wavelengths, or with more than 1, 2, 5, 10, or 10³ wavelength(s).

In one embodiment of the invention, the laser equipment, which can in some cases be designated as laser, is a device that emits or generates laser radiation, preferentially through a process of optical amplification based on stimulated emission of electromagnetic radiation. It is preferentially combined or used in combination with: i), a system such as an optical fiber that can carry or transport laser radiation from this equipment to the body part, ii), a system such as a lens, preferentially convergent or divergent, or a combination of lenses that can focalize or focus or de-focalize or de-focus the laser radiation preferentially on the body part or a specific location of the body part. The combination or association of the laser equipment with a system of light transportation and/or light focusing is designated as laser apparatus in this patent application.

The laser equipment or apparatus is preferentially a: i), gas laser, preferentially a helium-neon, argon, krypton, xenon ion, nitrogen, carbon dioxide or monoxide, or excimer laser, or ii), a chemical laser, preferentially a hydrogen fluoride, deuterium fluoride, chemical oxygen-iodine, or All gas-phase iodine laser, or iii), a dye laser, or iv), a metal-vapor laser, preferentially a helium-cadmium, helium-mercury, helium-selenium, helium-silver, a strontium vapor, a Neon copper, a Copper vapor, gold vapor, or manganese vapor laser, or v), a solid-state laser, preferentially, a ruby, Nd:YAG, NdCrYAG, Er:YAG, Neodymium YLF, Neodymium doped Yttrium orthovanadate, Neodymium doped yttrium calcium oxoborate, Neodymium glass, Titanium sapphire, Thulium YAG, Ytterbium YAG, Ytterbium:₂O₃, Ytterbium doped glass, Holmium YAG, Cerium doped lithium strontium (or calcium) aluminum fluoride, Promethium 147 doped phosphate glass, Chromium doped chrysoberyl (alexandrite), Erbium doped and erbium-ytterbium codoped glass, Trivalent uranium doped calcium fluoride, or divalent samarium doped calcium fluoride laser, or v), a semi-conductor laser, preferentially a Semiconductor diode, GaN, InGaN, AlGaInP, AlGaAs, InGaAsP, lead salt, Vertical cavity surface emitting, Quantum cascade, or Hybrid silicon laser, or vi), Free electron, Gas dynamic, "Nickel-like" Samarium, Raman, or Nuclear pumped laser. The laser equipment preferentially works in one of the following different ranges of wavelengths: X-rays, ultraviolet, visible, near infrared, mid infrared, far infrared. In some cases, the continuous wave laser power can be comprised between 10⁻³ mW and 10³ kW. In some other cases, the laser pulse energy is preferentially comprised between 1 mJ and 1 kJ.

In one embodiment of the invention, the wavelength λ is a monochromatic laser radiation. It is preferentially characterized by a spread or distribution in wavelengths, Δλ, which is lower than 10³λ, 10λ, λ/1.01, λ/2, λ/10, or λ/10³.

In another embodiment of the invention, each of the n different wavelengths λᵢ (1 < i < n) of a polychromatic laser radiation is characterized by a spread or distribution in wavelengths, which is such that Δλᵢ is lower than the absolute value of (λᵢ - λⱼ), where λᵢ and λⱼ are two different wavelengths of the polychromatic laser radiation, preferentially lower by more than 10⁹, 10⁶, 10³, 10, or 10⁻¹ nm.

In one embodiment of the invention, the laser radiation is characterized by or possesses or is associated with or comprises a laser power, laser power density, laser intensity, laser strength, or laser wavelength. In some cases, the laser radiation is continuous. In some other cases, the laser radiation is pulsed. In some cases, the word laser radiation can designate or mean laser, laser equipment, laser apparatus, laser radiation beam, laser beam, laser energy, laser intensity, laser power, laser power density, laser strength, laser frequency, laser wavelength, or laser pulsation. In still some other cases, the laser power density can be defined as the laser power divided by the volume, surface, or length, preferentially of the body part, which is preferentially irradiated by laser radiation or onto which laser radiation is preferentially applied. In some cases, the laser power or laser power density or another parameter of the laser is the laser power or power density or other parameter of the laser measured outside or at some distance, preferentially at a distance that is larger than 10⁻²⁰, 10⁻¹⁰, 10⁻⁵, 1, 10⁵, 10¹⁰, or 10²⁰ nm, of or from the equipment or apparatus generating the laser radiation. In some cases, the laser power or laser power density or another parameter of the laser is preferentially measured in the body part or region of magnetic nanoparticle, in particular magnetosome.

In one embodiment of the invention, the laser radiation is not or is different from a magnetic field, an alternating magnetic field, preferentially of strength larger 10⁻⁹, 10⁻⁶, 10⁻³, 10⁻¹, 1, 10³, or 10⁶ T, preferentially of frequency larger than 10⁻⁹, 10⁻⁶, 10⁻³, 10⁻¹, 1, 10 kHz, of an acoustic wave, of an ultrasound, of X-rays, of gamma rays, or of waves associated with or inducing or comprising the movement or oscillation, preferentially spatially or temporally, of particles with a non-zero mass or with a mass larger than 10⁻⁸⁰, 10⁻⁴⁰, 10⁻²⁰, 10⁻¹⁰, 10⁻⁵, 10⁻¹, or 1 gram.

In one embodiment of the invention, a laser radiation medical treatment, which could be designated as medical treatment, treatment, or laser treatment, uses the laser radiation or the irradiation by the laser radiation, preferentially applied on magnetic nanoparticle, in particular magnetosome, or body part, to trigger a medical, pharmaceutical, immunological, metabolic, diagnostic, medical device, drug, biological, or cosmetic effect. In some cases, a medical treatment can be the treatment of an illness or disease, such as an infectious disease, a cancer, or a therapeutic treatment. It can be the treatment of a disease due to the malfunction of an organ or body part. It can lead to or be associated with the destruction, disappearance, of pathological cells, preferentially of more than 1, 5, 10, 50, 75, 80, or 90 % of pathological cells, where this percentage can be equal to the ratio between the number of pathological cells, preferentially comprised in the body part, after the medical treatment, and the number of pathological cells, preferentially comprised in the body part, before the medical treatment. The presence of pathological cells, preferentially in the body part, can be due to the malfunction of a body part of an individual. In some cases, the medical treatment can be a diagnostic of a disease or a cosmetic treatment.

In one embodiment of the invention, the laser medical treatment is the treatment of a disease due to or associated with the presence of viruses, bacteria, or other types of organisms or pathological cells than those present in the treated individual before the beginning or appearance of the disease in the individual.

In one embodiment of the invention, the medical treatment is the treatment of anemia, preferentially the anemia or lack of a substance comprised in the body part, preferentially the anemia or lack in or of iron or of a substance comprised in the compound. In some cases, the anemia can be defined as a concentration in a substance comprised in an individual, which is more than 1.001, 1.01, 1.1, 2, 5, 10, 10², 10⁵, 10¹⁰, or 10²⁰ times lower in the individual suffering from anemia than in the healthy individual.

In some cases, anemia of a substance comprised in the body part is defined as a concentration of a substance comprised in the magnetic nanoparticles, in particular the magnetosomes, or compound, such as iron, oxygen or a substance comprised in the compound, which is lower, preferentially 1.001, 1.01, 1.1, 2, 5, 10, 10², 10⁵, 10¹⁰, or 10²⁰ times lower, in the body part before administration of the magnetic nanoparticles, in particular the magnetosomes, or without magnetic nanoparticles, in particular magnetosomes, in the body part than after administration of the magnetic nanoparticles, in particular the magnetosomes, or with magnetic nanoparticles, in particular magnetosomes, in the body part.

In an embodiment of the invention, a compound is bound or attached to the magnetic nanoparticle, in particular magnetosome, preferentially before the irradiation by the laser radiation of the magnetic nanoparticles, in particular the magnetosomes.

In one embodiment of the invention, compound(s) or the compound(s) represent(s) an assembly comprising more than 1, 10, 10², 10³, 10⁵, 10¹⁰, 10²⁰, or 10⁵⁰ compound(s).

In still another embodiment of the invention, compound(s) or the compound(s) represent(s) an assembly comprising less than 1, 10, 10², 10³, 10⁵, 10¹⁰, 10²⁰, or 10⁵⁰ compound(s).

In one embodiment of the invention, the laser radiation is a radiation, which is associated with, or linked with, or which induces, or produces, or results in, or is responsible for, or creates the movement, or vibration, or oscillation of the compound, preferentially after the dissociation of the compound from the magnetic nanoparticle, in particular the magnetosome.

In an embodiment of the invention, the compound is a therapeutic, immunogenic, metabolic, luminescent, fluorescent, radioactive, diagnostic, biologic, or chemical compound, or is a compound that triggers a therapeutic, immunogenic, metabolic, luminescent, fluorescent, radioactive, or diagnostic effect.

In one embodiment of the invention, the compound is part of the magnetic nanoparticle, in particular the magnetosome. In this case, it can be free iron or free oxygen, preferentially in the ionic form, which preferentially dissociates or leaks out or diffuses away from the magnetic nanoparticle, in particular the magnetosome, preferentially under or following the irradiation by the laser radiation of the magnetic nanoparticle, in particular the magnetosome, or body part, preferentially following dissolution of the magnetic nanoparticle, in particular magnetosome, preferentially following administration of the magnetic nanoparticle, in particular magnetosome, in/to the body part.

In one embodiment of the invention, the magnetic nanoparticles, in particular the magnetosomes, are administered to or in the body part, when they are directly administered to the body part or when they are administered close to the body part, preferentially less than 1, 10⁻¹, 10⁻², 10⁻³, 10⁻⁴, 10⁻⁵, 10⁻⁶, or 10⁻⁹ m away from the body part. In this case, the magnetic nanoparticles, in particular the magnetosomes, may not need to be transported or diffuse from the region where they are administered to the body part.

In another embodiment of the invention, the magnetic nanoparticles, in particular the magnetosomes, are administered to or in the body part, when they are administered far from the body part, preferentially more than 1, 10⁻¹, 10⁻², 10⁻³, 10⁻⁴, 10⁻⁵, 10⁻⁶, or 10⁻⁹ m away from the body part. In this case, the magnetic nanoparticles, in particular the magnetosomes, may be transported or diffuse from the region where they are administered to the body part.

In another embodiment of the invention, the magnetic nanoparticles, in particular the magnetosomes, are administered to or in the body part when they are injected in, or mixed with, or introduced in, or inserted in the body part.

In another embodiment of the invention, the magnetic nanoparticles, in particular the magnetosomes, are administered to or in the body part when they occupy more than 10⁻⁹, 10⁻⁷, 10⁻⁵, 10⁻³, 1, 10, 25, 50, or 75 % of the body part, where this percentage can be the ratio between the volume of the region occupied by the magnetic nanoparticles, in particular the magnetosomes, in the body part and the volume of the body part, preferentially comprising regions without magnetic nanoparticles, in particular magnetosomes. This occupation can correspond to that measured 10⁻⁵, 10⁻³, 10⁻¹, 1, 10, 10³, or 10⁵ minute(s) following administration of magnetic nanoparticles, in particular magnetosomes.

In one embodiment of the invention, the volume of the region occupied by the magnetic nanoparticles, in particular magnetosomes, in the body part is designated as region of magnetic nanoparticles, in particular magnetosomes. The region of magnetic nanoparticles, in particular magnetosomes, can be the volume occupied by an assembly of magnetic nanoparticles, in particular magnetosomes, in the body part, where the magnetic nanoparticles, in particular magnetosomes, are preferentially separated by less than 10⁹, 10⁶, 10³, or 10 nm. In some cases, the separating distance between the magnetic nanoparticles, in particular magnetosomes, within the assembly of magnetic nanoparticle, in particular magnetosome, can correspond to the average or maximum distance separating the magnetic nanoparticles, in particular magnetosomes, within this assembly. In some cases, the distribution in separating distances between magnetic nanoparticles, in particular magnetosomes, can highlight the presence of a minority of magnetic nanoparticle, in particular magnetosomes, *i.e.* preferentially less than 50, 10, 1, 10⁻², or 10⁻⁵ % of the total number of magnetic nanoparticles, in particular magnetosomes, in the individual, with either small separating distances, *i.e* separating distances preferentially lower than 10⁹, 10⁶, 10³, or 10 nm, or with large separating distances, *i.e.* separating distances preferentially larger than 10⁹, 10⁶, 10³, or 10 nm. In this case, the presence of this minority of magnetic nanoparticle, in particular magnetosomes, is preferentially not taken into consideration to estimate the average or maximum separating distance between the magnetic nanoparticles, in particular magnetosomes.

In another embodiment of the invention, the magnetic nanoparticles, in particular magnetosomes, are administered to or in the body part following at least one of the following administration routes: local, enteral, gastrointestinal, parenteral, topical, oral, by inhalation, intramuscular, subcutaneous, intra-tumor, in an organ, in a vein, in arteries, in blood, or in tissue.

In one embodiment of the invention, the body part or a portion of the body part is a pathological site. The pathological site can be defined as an unhealthy site, or a site that is in a different condition from a site of a healthy individual, or the site of an unhealthy individual. It can comprise pathological cells, such as tumor cells, bacteria, eukaryotic or prokaryotic cells, viruses or other pathological material. It can also comprise healthy cells, which are preferentially not arranged or working as they usual do in a healthy individual, most preferentially due to the presence of pathological cells in the pathological site. It can comprise a larger number of pathological than healthy cells. It can lead to a ratio between the number of pathological cells and number of healthy cells, which is preferentially larger than 2, 5, 10, 10³, 10⁶, 10⁹, 10²⁰, or 10⁵⁰.

In one embodiment of the invention, pathological cell(s) or the pathological cell(s) represent(s) an assembly comprising more than 1, 10, 10², 10³, 10⁵, 10¹⁰, 10²⁰, or 10⁵⁰ pathological cell(s).

In one embodiment of this invention, the body part is divided between a portion of the body part comprising the magnetic nanoparticles, in particular the magnetosomes, also preferentially designated as region of magnetic nanoparticles, in particular magnetosomes, and a portion of the body part not comprising the magnetic nanoparticles, in particular the magnetosomes, also preferentially designated as region of the body part outside of the region of magnetic nanoparticles, in particular magnetosomes. In some cases, the portion of the body part comprising the magnetic nanoparticles, in particular the magnetosomes, can absorb more than 10⁻⁹, 10⁻⁷, 10⁻⁵, 10⁻³, 10⁻¹, 1, 5, 10, 25, 50, 75, or 80% of the energy or power of the laser and the portion of the body part not comprising the magnetic nanoparticle, in particular the magnetosomes, can preferentially absorb less than 10⁻⁹, 10⁻⁷, 10⁻⁵, 10⁻³, 10⁻¹, 1, 5, 10, 25, 50, 75, or 80% of the energy or power of the laser. In some other cases, the portion of the body part comprising the magnetic nanoparticle, in particular the magnetosomes, can absorb less than 10⁻⁹, 10⁻⁷, 10⁻⁵, 10⁻³, 10⁻¹, 1, 5, 10, 25, 50, 75, or 80% of the energy of the laser and the portion of the body part not comprising the magnetic nanoparticles, in particular the magnetosomes, can preferentially absorb more than 10⁻⁹, 10⁻⁷, 10⁻⁵, 10⁻³, 10⁻¹, 1, 5, 10, 25, 50, 75, or 80% of the energy of the laser. In some cases, this percentage can represent the energy or power of the laser radiation absorbed by the magnetic nanoparticles, in particular magnetosomes, divided by the energy or power of the laser radiation generated by the laser equipment or laser apparatus. In some other cases, this percentage can represent the ratio between the energy or power of the laser radiation absorbed by the portion of the body part comprising the magnetic nanoparticles, in particular magnetosomes, divided by the energy or power of the laser radiation absorbed by the body part, preferentially comprising both the portion of the body part with the magnetic nanoparticles, in particular magnetosomes, and the portion of the body part without the magnetic nanoparticles, in particular magnetosomes.

In one embodiment of this invention, the body part is divided between a portion of the body part comprising the pathological site or the pathological cells and a portion of the body part not comprising the pathological site or the pathological cells.

In some cases, the body part can designate the portion of the body part.

In some cases, the portion of the body part can designate: i), the portion of the body part comprising the magnetic nanoparticles, in particular magnetosomes, ii), the region of the magnetic nanoparticles, in particular the magnetosomes, or iii), the portion of the body part comprising the pathological cells or pathological site.

In some other cases, the portion of the body part can designate: i), the portion of the body part not comprising the magnetic nanoparticles, in particular the magnetosomes, ii), the region outside of the region of magnetic nanoparticles, in particular magnetosomes, or iii), the portion of the body part not comprising the pathological cells or pathological site.

In this invention, the body part or magnetic nanoparticles, in particular magnetosomes, exposed to the laser radiation or irradiated by the laser radiation can mean that the laser radiation irradiates, covers, targets, is present in, is applied in or on, or is located in at least 10⁻⁹, 10⁻⁷, 10⁻⁵, 10⁻³, 10⁻¹, 1, 5, 10, 25, 50, 75, or 80% of the body part or magnetic nanoparticle(s), in particular magnetosome(s). This percentage can represent the number or volume of magnetic nanoparticle(s), in particular magnetosome(s), or of region of magnetic nanoparticle(s), in particular magnetosome(s), or body part, exposed to the laser radiation or irradiated by the laser radiation divided by the total number or volume of magnetic nanoparticle(s), in particular magnetosome(s), region of magnetic nanoparticles, in particular magnetosomes, or body part, which is(are) both exposed and not irradiated by laser radiation. In some cases, the laser radiation can also cover, target, be present, be applied in or on, or be located outside of the body part, or magnetic nanoparticle(s), in particular magnetosome(s), or region of magnetic nanoparticle(s), in particular magnetosome(s), preferentially when the laser radiation is of low enough power or energy not to induce toxicity towards healthy cells.

In one embodiment of the invention, healthy cell(s) or the healthy cell(s) represent(s) an assembly comprising more than 1, 10, 10², 10³, 10⁵, 10¹⁰, 10²⁰, or 10⁵⁰ healthy cell(s).

Furthermore, in some cases, the body part, or magnetic nanoparticle(s), in particular magnetosome(s), or the region of magnetic nanoparticles, in particular magnetosomes, can be exposed to the laser radiation or can be irradiated by the laser radiation when the laser radiation is applied on or the laser irradiates: i), the body part, or, ii), the magnetic nanoparticle(s), in particular magnetosome(s), or, iii), the region of magnetic nanoparticles, in particular magnetosomes. In some other cases, the body part, or magnetic nanoparticle(s), in particular magnetosome(s), or the region of magnetic nanoparticles, in particular magnetosomes, can be exposed to the laser radiation or can be irradiated when the body part, or magnetic nanoparticle(s), in particular magnetosome(s), or the region of magnetic nanoparticle(s), in particular magnetosome(s), is(are) irradiated or is(are) subjected to the application of the laser radiation or undergo(es) the effect(s) of the laser radiation or the disturbance created by the laser radiation.

In an embodiment of the invention, the body part comprises more than 1, 2, 5, 10, or 100 similar or different organism(s), apparatus, organ(s), tissue(s), cell(s), or biomolecule(s). The body part can be all or part of the head, neck, shoulder, arm, leg, knee, foot, hand, ankle, elbow, trunk, inferior members, or superior members, preferentially of the individual as defined in this patent application.

In one embodiment of the invention, the body part is or comprises water, an excipient, a solution, a suspension, at least one chemical element, organic material, or gel, which can be synthetic or produced by a living organism.

In an embodiment of the invention, the organ or body part belongs to the musculoskeletal, muscular, digestive, respiratory, urinary, female reproductive, male reproductive, circulatory, cardiovascular, endocrine, circulatory, lymphatic, nervous (peripheral or not), ventricular, enteric nervous, sensory, integumentary system, reproductive organ (internal or external), sensory organ, or endocrine glands. The organ or body part can be or belong to human skeleton, joints, ligaments, tendons, mouth, teeth, tongue, salivary glands, parotid glands, submandibular glands, sublingual glands, pharynx, esophagus, stomach, small intestine, duodenum, jejunum, ileum, large intestine, liver, gallbladder, mesentery, pancreas, nasal cavity, pharynx, larynx, trachea, bronchi, lungs, diaphragm, kidneys, ureters, bladder, urethra, ovaries, fallopian tubes, uterus, vagina, vulva, clitoris, placenta, testes, epididymis, vas deferens, seminal vesicles, prostate, bulbourethral glands, penis, scrotum, pituitary gland, pineal gland, thyroid gland, parathyroid glands, adrenal glands, pancreas, heart, arteries, veins, capillaries, lymphatic vessel, lymph node, bone marrow, thymus, spleen, gut-associated lymphoid tissue, tonsils, brain, cerebrum, cerebral hemispheres, diencephalon, brainstem, midbrain, pons, medulla, oblongata, cerebellum, spinal cord, choroid plexus, nerves, cranial nerves, spinal nerves, ganglia, eye, cornea, iris, ciliary body, lens, retina, ear, outer ear, earlobe, eardrum, middle ear, ossicles, inner ear, cochlea, vestibule of the ear, semicircular canals, olfactory epithelium, tongue, taste buds, mammary glands, or skin. The body part or organ can belong to the circulatory system.

In an embodiment of the invention, the body part designates the body part of an individual. In some cases, the individual is an organism, preferentially a living organism, a plant, a tree, a flour, a fungus, a mushroom, an archaea, a microbe, an animal, a mammal, a bird, a crustacean, a fish, a vertebrate animal, a bacterium, a human, a man, a woman, an elderly, or a child. In some cases, a distinction is made between the individual that is treated for a disease, preferentially also designated as treated individual, and the individual or organism that is responsible for the disease of the treated individual.

In some cases, the individual can be alive.

In some other cases, the individual can be dead, or be an inactivated or dead organism.

In some cases, the body part of the individual is an assembly of cells, preferentially of more than 1, 10, 10³, 10⁶, 10⁹, or 10²⁰ cell(s), that are preferentially comprised in the body part, extracted from the body part, issued from the body part, or resulting in or from the body part. In some cases, these cells can be cultivated and amplified to reach a certain number, preferentially more than 1, 10, 10³, 10⁶, 10⁹, or 10²⁰ cell(s).

In an embodiment of the invention, the magnetic nanoparticles, in particular the magnetosomes, according to the invention are drugs, medical devices, cosmetic products, biological products, products used for research purposes, or products used to determine the properties of biological or chemical samples.

In one embodiment of the invention, the compound can dissociate or is dissociated from the magnetic nanoparticles, in particular the magnetosomes, preferentially under the irradiation by the laser radiation. In some cases, the compound is dissociated from the magnetic nanoparticles, in particular the magnetosomes, when more than 10⁻⁵, 10⁻³, 10⁻¹, 1, 5, 10, 25, 50, 75, 85, or 90% of compounds are dissociated from the magnetic nanoparticles, in particular the magnetosomes, where this percentage can represent the percentage of dissociation.

In one embodiment of the invention, the percentage of dissociation represents the ratio between the quantity of compounds dissociated from the magnetic nanoparticles, in particular the magnetosomes, following or under the irradiation by the laser radiation and the quantity of compounds linked or bound to the magnetic nanoparticles, in particular the magnetosomes, before or without the irradiation by the laser radiation. In some cases, the number of compounds linked or bound to one magnetic nanoparticle, in particular one magnetosome, can be larger than 1, 2, 5, 10, 10³, 10⁵, or 10¹⁰. In some other cases, the number of compounds linked or bound to one magnetic nanoparticle, in particular one magnetosome, is lower than 2, 5, 10, 10³, 10⁵, or 10¹⁰. In still some other cases, the percentage of compounds dissociated can increase by a factor of at least 1.01, 1.1, 2, 5, 7, 10, 10², or 10⁵ between before and after the irradiation by the laser radiation.

In one embodiment of the invention, the compound can't dissociate or is not dissociated from the magnetic nanoparticle, in particular magnetosome, preferentially in the absence of irradiation by the laser radiation. Preferentially, less than 10⁻⁵, 10⁻³, 10⁻¹, 1, 5, 10, 25, 50, 75, 85, or 90% of compounds are dissociated from the magnetic nanoparticles, in particular the magnetosomes, when the compounds are not dissociated from the magnetic nanoparticles, in particular the magnetosomes.

In one embodiment of the invention, the compound is linked or bound to the magnetic nanoparticle, in particular magnetosome, or not dissociated from the magnetic nanoparticle, in particular magnetosome, when it is located at a distance of less than 10⁹, 10⁵, 10³, 10, or 1 nm from the magnetic nanoparticle, in particular magnetosome. Alternatively, the compound can in some cases be linked or bound to the magnetic nanoparticle, in particular magnetosome, or non-dissociated from the magnetic nanoparticle, in particular magnetosome, when the magnetic nanoparticle, in particular magnetosome, and the compound can both or together be attracted by a magnet or both or together move under the application of a magnetic field gradient, of strength per unit distance preferentially larger than 10⁻⁹, 10⁻⁶, 10⁻³, 10⁻¹, or 1 T/m or T/cm or T/mm, where the speed of motion of the compound attached or linked to the magnetic nanoparticle, in particular magnetosome, or non-dissociated from the magnetic nanoparticle, in particular magnetosome, is preferentially larger than 10⁻⁹, 10⁻⁶, 10⁻³, 10⁻¹, 1, 10, or 10³ nm/sec or nm/min or nm/hour or nm/day or m/sec or m/min or m/hour or m/day.

In one embodiment of the invention, the compound is not bound, not linked to the magnetic nanoparticle, in particular magnetosome, or is dissociated from the magnetic nanoparticle, in particular magnetosome, when it is located at a distance of more than 10⁹, 10⁵, 10³, 10, or 1 nm from the magnetic nanoparticle, in particular magnetosome. Alternatively, the compound can in some cases not be bound or linked to the magnetic nanoparticle, in particular magnetosome, or be dissociated from the magnetic nanoparticle, in particular magnetosome, when the compound can't be attracted by a magnet or move under the application of a magnetic field gradient, of strength per unit length preferentially larger than 10⁻⁹, 10⁻⁶, 10⁻³, 10⁻¹, or 1 T/m or T/cm or T/mm, where the speed of motion of the compound is preferentially lower than 10⁻⁹, 10⁻⁶, 10⁻³, 10⁻¹, 1, 10, or 10³ nm/sec or nm/min or nm/hour or nm/day or m/sec or m/min or m/hour or m/day, or where the speed of motion of the compound is preferentially lower, by a factor of more than 1.001, 1.01, 1.1, 2, 5, 10, 10³, 10⁶, 10⁹, 10¹⁵, or 10²⁰, from the speed of motion of the magnetic nanoparticle, in particular magnetosome, or of the compound linked or bound to the magnetic nanoparticle, in particular magnetosome.

In one embodiment of the invention, the percentage of dissociation is between 10⁻²⁰% and 10²⁰%, or between 10⁻¹⁰% and 10¹⁰%, or between 10⁻⁵% and 10⁵%, or between 10⁻³% and 10³%, or between 10⁻¹% and 10%. A percentage of dissociation larger than 100% can in some cases be reached, for example when a compound that dissociates from the magnetic nanoparticle, in particular magnetosome, transforms itself or results in several compounds.

In still another embodiment of the invention, the irradiation by the laser radiation designates the irradiation by the laser radiation of the magnetic nanoparticle(s), in particular the magnetosome(s), or region of the magnetic nanoparticle(s), in particular the magnetosome(s), or body part. In some cases, the irradiation by the laser radiation can also designate the exposure of the magnetic nanoparticle(s), in particular the magnetosome(s), body part, or region of magnetic nanoparticles, in particular magnetosomes, to the laser radiation.

In another embodiment of the invention, the ratio between the mass, number, or weight, of the compounds, preferentially linked to a single magnetic nanoparticle, in particular magnetosome, and the mas, number, or weight of a single magnetic nanoparticle, in particular magnetosome, is lower than 10²⁰, 10⁹, 10⁵, 10², 2, 1, 10⁻², 10⁻⁵, 10⁻⁹, or 10⁻²⁰.

In another embodiment of the invention, the ratio between the mass, number, or weight, of the compound, preferentially linked to a single magnetic nanoparticle, in particular magnetosome, and the mas, number, or weight of a single magnetic nanoparticle, in particular magnetosome, is larger than 10²⁰, 10⁹, 10⁵, 10², 1, 10⁻², 10⁻⁵, 10⁻⁹, or 10⁻²⁰.

In one embodiment of the invention, a suitable range of values for the number of compounds preferentially linked to a single magnetic nanoparticle, in particular magnetosome, is between 1 and 178, where this minimum value of 1 corresponds to the minimum number of compounds that can be linked to a single magnetosome and the maximum value of 178 corresponds to the number of RhB molecules that was linked to a single magnetosome in patent WO2017/068252 incorporated in reference, and which could dissociate at least in part from a single magnetic nanoparticle, in particular magnetosome. In some cases, this maximum value of 178 can be increased, preferentially by a factor of more than 5, 10, 10³, 10⁷, 10¹⁰, or 10²⁰, by: i), decreasing the size, or mass of the compound linked to the magnetic nanoparticle, in particular magnetosome, preferentially by a factor of more than 1.1, 2, 5, 10, 10³, 10⁷, 10¹⁰, or 10²⁰, ii), changing the type of bounds between the compounds and the magnetic nanoparticle, in particular magnetosome, or iii), changing the method for attaching or binding the compound to the magnetic nanoparticle, in particular magnetosome.

In an embodiment of this invention, the body part is first irradiated by the laser radiation in a first step. This preferentially means that the body part, preferentially the portion of the body part comprising the magnetic nanoparticle, in particular magnetosome, receives or absorbs the energy or power of the laser, or receives or absorbs at least 10⁻⁹, 10⁻⁷, 10⁻⁵, 10⁻³, 10⁻¹, 1, 5, 10, 25, 50, 75, or 80% of the energy or power of the laser radiation, preferentially emitted or generated by the laser equipment or apparatus. The energy or power of the laser, which is not absorbed by the portion of the body part comprising the magnetic nanoparticle, in particular magnetosome, can be absorbed by the portion of the body part not comprising the magnetic nanoparticle, in particular magnetosome.

In some cases, in a/the first step can mean or be equivalent to during a/the first step.

The first step is preferentially the step during or in which the body part or magnetic nanoparticle, in particular magnetosome, is irradiated by the laser radiation. Alternatively, the first step is preferentially the step during or in which the body part or magnetic nanoparticle(s), in particular magnetosome(s), is(are) irradiated by the laser radiation.

In some cases, preferentially in the first step but possibly also in the second step, the laser power is set at a value that enables to induce a temperature increase of the magnetic nanoparticles, in particular magnetosomes, region of magnetic nanoparticles, in particular magnetosomes, portion of the body part comprising the magnetic nanoparticles, in particular magnetosomes, and preferentially to prevent a temperature increase of the body part not comprising the magnetic nanoparticles, in particular magnetosomes.

In some cases, in a/the second step can mean or be equivalent to during a/the second step.

In some cases, preferentially in the first step but possibly also in the second step, the laser power or laser power density can be maintained below 10⁹, 10⁶, 10³, 10², 10, or 1 W or W/cm or W/cm² or W/cm³ or W per cm of body part or W per cm² of body part or W per cm³ of body part.

In some cases, preferentially in the first step but possibly also in the second step, the laser intensity can be maintained below 10²⁰, 10⁹, 10⁶, 10³, 10², 10, or 1 mA (milliampere).

In the experimental example, the laser intensity is fixed at 4500 A, and a power density of 2 W/cm² is used, which is sufficient to induce a temperature increase of the magnetic nanoparticles, in particular magnetosomes, and does not induce a temperature increase of water alone, not comprising the magnetic nanoparticles, in particular magnetosomes. In some cases, it is possible to use laser intensity, which is lower than 4500 mA, preferentially by a factor of more than 1.001, 1.01, 1.1, 1.5, 2, 5, 10, or 10³, and/or a power density, which is lower than 2 W/cm², preferentially by a factor of more than 1.001, 1.01, 1.1, 1.5, 2, 5, 10, or 10³, and still induce a temperature increase during the heating step. This can preferentially be achieved by: i), increasing the magnetic nanoparticle, in particular magnetosome, concentration, preferentially by a factor of more than 1.001, 1.01, 1.1, 1.5, 2, 5, 10, or 10³, ii), increasing the duration of the heating step, preferentially by a factor of more than 1.001, 1.01, 1.1, 1.5, 2, 5, 10, or 10³, or iii), by reduction the temperature increase or temperature gradient that one wants to achieve during the heating step, preferentially by a factor of more than 1.001, 1.01, 1.1, 1.5, 2, 5, 10, or 10³.

In some cases, the laser intensity can be the strength of the current that produces or induces laser radiation with a certain power. The laser intensity can in some cases be related to the laser power and a calibration curve can in some cases be established to relate the laser intensity to the laser power.

In some cases, a too high laser power, power density, or laser intensity, is preferentially avoided to prevent toxicity or overheating or a too large percentage of dissociation of compounds from the magnetic nanoparticle, in particular magnetosomes, or to enable cooling during the cooling step.

In some other cases, preferentially in the first step but possibly also in the second step, the laser power is maintained above 10⁻⁹, 10⁻⁶, 10⁻³, 10⁻¹, 1, 10, 10³, or 10⁵ W or W/cm or W/cm² or W/cm³ or W per cm of body part or W per cm² of body part or W per cm³ of body part.

In still some other cases, preferentially in the first step but possibly also in the second step, the laser intensity is maintained above 10⁻²⁰, 10⁻⁹, 10⁻⁶, 10⁻³, 10⁻¹, 1, 10, 10³, 10⁵, or 10¹⁰ mA

In the experimental example, the laser intensity is fixed at 4500 A, and a power density of 2 W/cm² is used, which is sufficient to induce a temperature increase of the magnetic nanoparticles, in particular magnetosomes, and does not induce a temperature increase of water alone, not comprising the magnetic nanoparticles, in particular magnetosomes. In some cases, it is possible to use a laser intensity, which is larger than 4500 mA, preferentially by a factor of more than 1.001, 1.01, 1.1, 1.5, 2, 5, 10, or 10³, and/or a power density, which is larger than 2 W/cm², preferentially by a factor of more than 1.001, 1.01, 1.1, 1.5, 2, 5, 10, or 10³, and induce a temperature increase of the magnetic nanoparticles, in particular magnetosomes, region of magnetic nanoparticles, in particular magnetosomes, or portion of the body part comprising the magnetic nanoparticles, in particular magnetosomes, preferentially without inducing a temperature increase of the portion of the body part not comprising the magnetic nanoparticles, in particular magnetosomes, or of the region outside of the region of the magnetic nanoparticles, in particular magnetosomes. This can preferentially be achieved by: i), decreasing the concentration of the magnetic nanoparticles, in particular magnetosomes, preferentially by a factor of more than 1.001, 1.01, 1.1, 1.5, 2, 5, 10, or 10³, ii), decreasing the duration of the heating step, preferentially by a factor of more than 1.001, 1.01, 1.1, 1.5, 2, 5, 10, or 10³, or iii), by increasing the temperature increase or temperature gradient that one wants to achieve during the heating step, preferentially by a factor of more than 1.001, 1.01, 1.1, 1.5, 2, 5, 10, or 10³.

In sill some other cases, a sufficiently high laser power or laser power density or laser intensity can be desired to be able to heat the magnetic nanoparticles, in particular the magnetosomes, or to produce a sufficiently large percentage of dissociation of the compounds from the magnetic nanoparticle, in particular magnetosomes, preferentially in the first step.

In an embodiment of the invention, the laser power or laser power density or laser intensity is maintained sufficiently large in the first step to produce a temperature increase of the portion of the body part comprising the magnetic nanoparticles, in particular magnetosomes. In some cases, the temperature increase of this body part can be larger than 10⁻⁶, 10⁻³, 10⁻¹, 1, 5, 10, 10², or 10³ °C. In some other cases, the temperature increase of this body part can be lower than 10⁶, 10³, 10, 1, or 10⁻¹ °C.

In one embodiment of the invention, the temperature increase designates the temperature increase of the body part, portion of the body part comprising the magnetic nanoparticles, in particular the magnetosomes, region of the magnetic nanoparticles, in particular the magnetosomes, region, or the magnetic nanoparticles, in particular the magnetosomes. The temperature increase can be assimilated to or correspond to the magnitude of the temperature increase occurring in the second step, which is preferentially the absolute value of the difference between the maximum and minimum temperature reached in the second step, which is also in some cases designated as temperature variation or temperature variation during or occurring in the first step.

In another embodiment of the invention, the laser power or power density or laser intensity is maintained sufficiently high in the first step to produce the dissociation of the compounds from the magnetic nanoparticles, in particular the magnetosomes. In some cases, the percentage of dissociation of the compounds from the magnetic nanoparticles, in particular the magnetosomes, can be larger than 10⁻⁵, 10⁻³, 10⁻¹, 1, 5, 10, 25, 50, 75, or 90 %. In some other cases, the percentage of dissociation of the compounds from the magnetic nanoparticles, in particular the magnetosomes, can be lower than 10⁻⁵, 10⁻³, 10⁻¹, 1, 5, 10, 25, 50, 75, 90, or 99 %.

In another embodiment of the invention, the laser power or laser power density or laser intensity is maintained sufficiently low in the first step to prevent a temperature increase of the portion of the body part not comprising the magnetic nanoparticles, in particular the magnetosomes.

In another embodiment of the invention, the laser power or laser power density or laser intensity is maintained sufficiently low in the first step to prevent the dissociation of the compounds from the magnetic nanoparticles, in particular the magnetosomes.

In some cases, the laser wavelength could be designated as or correspond to laser emission wavelength or laser radiation wavelength,

In still another embodiment of the invention, in the first step, the laser wavelength is lower or is maintained lower than 10²⁰, 10¹⁵, 10⁹, 10⁷, 10⁵, 10⁴, 1000, 900, 800, 700, 500, 100, 50, 20, 2, or 1 nm. In still another embodiment of the invention, in the first step, the laser wavelength is larger or is maintained larger than 10²⁰, 10¹⁵, 10⁹, 10⁷, 10⁵, 10⁴, 1000, 900, 800, 700, 500, 100, 50, 20, 2, or 1 nm. In still another embodiment of the invention, in the first step, the laser wavelength is between or is maintained between 1 nm and 100 000 nm, 1 nm and 10 000 nm, 20 nm and 5000 nm, 50 nm and 2000 nm, or between 100 nm and 1000 nm.

In one embodiment of the invention, the electromagnetic radiation, preferentially associated with or corresponding to laser radiation, comprises more than 1, 2, 5, 10, or 10³ different frequency(ies) of oscillation.

In still another embodiment of the invention, the electromagnetic radiation, preferentially associated with or corresponding to laser radiation, comprises less than 2, 5, 10, or 10³ different frequency(ies) of oscillation.

In still another embodiment of the invention, the electromagnetic radiation, preferentially associated with or corresponding to laser radiation, is different from or is not a magnetic field or an alternating magnetic field.

In still another embodiment of the invention, in the first step, the laser wavelength is fixed at a value that corresponds to or leads to or results in a strong absorption of light by the magnetic nanoparticle, in particular magnetosome, *i.e.* a wavelength that is preferentially lower than 10000, 5000, 2000, 1000, 900, 800, 700, 600, 500,400, 300, 200, 100, 50, 20, 10, 5, 2, or 1 nm.

In still another embodiment of the invention, preferentially in the first step, the laser wavelength is fixed at a value that corresponds to or leads to or results in a strong absorption or penetration of light in or to the body part or individual, *i.e.* a wavelength that is preferentially larger than 10000, 5000, 2000, 1000, 900, 800, 700, 600, 500, 400, 300, 200, 100, 50, 20, 10, 5, 2, or 1 nm, or a wavelength that is between 10000 and 1 nm, 5000 and 1 nm, 5000, 10 nm, 5000 and 100 nm, or between 2000 nm and 200 nm.

In still another embodiment of the invention, preferentially in the first step, the laser wavelength is fixed at a wavelength that enables to reach maximum coupling between the surface plasmon wave of the magnetic nanoparticle, in particular magnetosome, and the laser radiation.

In the experimental example, the laser wavelength is fixed at 808 nm. In some cases, this laser wavelength can be increased, preferentially by a factor of more than 1.001, 1.01, 1.1, 10, 10³, or 10⁶, preferentially to enhance absorption of the laser radiation by the body part, preferentially by a factor of more than 1, 2, 5, 10, 10³, or 10⁶. In some other cases, this laser wavelength can be decreased, preferentially by a factor of more than 1.001, 1.01, 1.1, 10, 10³, or 10⁶, preferentially to enhance absorption of the laser radiation by the magnetic nanoparticles, in particular magnetosomes, preferentially by a factor of more than 1.001, 1.01, 1.1, 10, 10³, or 10⁶.

In some cases, the percentage of dissociation of the compounds from the magnetic nanoparticles, in particular magnetosomes, reached in the first step can be larger than 10⁻⁵, 10⁻³, 10⁻¹, 1, 5, 10, 25, 50, 75, or 90 %.

In some other cases, the percentage of dissociation of the compounds from the magnetic nanoparticles, in particular magnetosomes, reached in the first step can be lower than 10⁻⁵, 10⁻³, 10⁻¹, 1, 5, 10, 25, 50, 75, 90, or 99 %.

In still some cases, the percentage of dissociation of the compounds from the magnetic nanoparticles, in particular magnetosomes, reached in the first step can be between 10⁻⁵ and 99%, 10⁻³ and 99%, 10⁻¹ and 99%, 1 and 99%, 10 and 99%, 10 and 90%, or between 25 and 75%.

The second step is preferentially the step during which or in which the body part, or magnetic nanoparticle, preferentially magnetosome, is irradiated by a laser radiation of lower power than in the first step. Alternatively, the second step is preferentially the step during which or in which the body part, or magnetic nanoparticle, preferentially magnetosome, is not irradiated by the laser radiation. Alternatively, the second step is preferentially the step during which or in which no laser irradiation of the magnetic nanoparticles, preferentially the magnetosomes, or body part, is performed.

In one embodiment of the invention, the laser power or laser power density or laser intensity is at least 1.1, 1.2, 1.5, 2, 5, 10, 10³, 10⁶, 10⁹, or 10²⁰ times lower in the second step than in the first step.

In the experimental example, the laser is switched off in the second step. It is however possible to maintain the laser switched on during the step, provided the laser power is sufficiently low, preferentially to avoid heating the magnetic nanoparticles, in particular the magnetosomes, or body part. This may in some cases be useful to avoid damaging the laser by switching it on and off a too large number of times.

In still another embodiment of the invention, in the second step, the laser wavelength is maintained at a value that differs by less than 100 000, 10 000, 1000, 900, 800, 700, 500, 100, 50, 20, 2, or 1 nm, from the laser wavelength used in the first step.

In still another embodiment of the invention, in the second step, the laser wavelength is maintained at a value that differs by more than 100 000, 10 000, 1000, 900, 800, 700, 500, 100, 50, 20, 2, or 1 nm, from the laser wavelength used in the first step.

In still another embodiment of the invention, in the second step, the laser wavelength is fixed at a value that corresponds to or leads to or results in weak absorption of light by the magnetic nanoparticles, in particular the magnetosomes, *i.e.* preferentially a wavelength that is larger than 1, 5, 10, 100, 200, 300, 400, 500, 600, 700, 800, 900, 1000, 2000, 5000, or 10000 nm.

In still another embodiment of the invention, in the second step, the laser wavelength is fixed at a wavelength that enables to reach minimum coupling between the surface plasmon waves of the magnetic nanoparticles, in particular magnetosomes, and the laser radiation.

In one embodiment of the invention, the first and/or second step(s) is(are) repeated at least 1, 2, 3, 4, 5, 10, 100, 10³, 10⁵, or 10¹⁰ time(s).

In another embodiment of the invention, the first and/or second step(s) is(are) repeated less than 1, 2, 3, 4, 5, 10, 100, 10³, 10⁵, or 10¹⁰ time(s).

In still another embodiment of the invention, the first and/or second step(s) is(are) repeated between 2 and 10¹⁰ times, between 2 and 10⁵ times, between 2 and 10⁵ times, between 2 and 10³ times, between 5 and 10³ times, or between 10 and 10³ time(s).

In one embodiment of the invention, the sequence of first step and second step is repeated at least 1, 2, 3, 4, 5, 10, 100, 10³, 10⁵, or 10¹⁰ times.

In another embodiment of the invention, the sequence of first step and second step is repeated less than 1, 2, 3, 4, 5, 10, 100, 10³, 10⁵, or 10¹⁰ time(s).

In still another embodiment of the invention, the sequence of first step and second step is repeated between 2 and 10¹⁰ times, between 2 and 10⁵ times, between 2 and 10⁵ times, between 2 and 10³ times, between 5 and 10³ times, or between 10 and 10³ time(s).

In some cases, the sequence of first step and second step is the first step followed by the second step. In still another embodiment of the invention, the number of sequences per unit time is larger than 10⁻⁵⁰, 10⁻²⁰, 10⁻¹⁰, 10⁻⁵, 10⁻³, 10⁻¹, 1, 10, or 10³ sequence(s)/sec.

In still another embodiment of the invention, the number of sequences per unit time is mower than 10⁵⁰, 10²⁰, 10¹⁰, 10⁵, 10³, 10, 1, 10⁻¹, 10⁻³ sequence(s)/sec.

In the experimental example, the number of sequences per unit time was: 8 10⁻³ sequences per sec. for 0.5 mg/mL of M-CMD, 10⁻² sequences per sec. for 1 mg of M-CMD, and 7 10⁻³ sequences per sec. for 1 mg of N-CMD. In some cases, it is possible to increase the number of sequences per unit time, preferentially by a factor of more than 1.001, 1.01, 1.1, 1.5, 2, 5, 10, or 10³, by: i), increasing the magnetic nanoparticle, in particular magnetosome, concentration, preferentially by a factor of more than 1.001, 1.01, 1.1, 1.5, 2, 5, 10, or 10³, ii), increasing the laser power during the heating step or decreasing the laser power during the cooling step, preferentially by a factor of more than 1.001, 1.01, 1.1, 1.5, 2, 5, 10, or 10³, or iii), reducing the temperature increase of the heating step or temperature decrease of the cooling step that one wants to achieve, preferentially by a factor of more than 1.001, 1.01, 1.1, 1.5, 2, 5, 10, or 10³. In some other cases, it is possible to decrease the number of sequences per unit time, preferentially by a factor of more than 1.001, 1.01, 1.1, 1.5, 2, 5, 10, or 10³, by: i), decreasing the magnetic nanoparticle, in particular magnetosome, concentration, preferentially by a factor of more than 1.001, 1.01, 1.1, 1.5, 2, 5, 10, or 10³, ii), decreasing the laser power during the heating step or increasing the laser power during the cooling step, preferentially by a factor of more than 1.001, 1.01, 1.1, 1.5, 2, 5, 10, or 10³, or iii), increasing the temperature increase of the heating step or temperature decrease of the cooling step that one wants to achieve, preferentially by a factor of more than 1.001, 1.01, 1.1, 1.5, 2, 5, 10, or 10³.

In still another embodiment of the invention, several sequences correspond to a treatment session. Treatment session can be separated by a duration, which is more than 1.001, 1.01, 1.1, 1.2, 1.5, 2, 5, 10, 10³, or 10⁵ times larger than t₁ₐ+t₂ₐ or t_{1b}+t_{2b}.

In one embodiment of the invention, the treatment session is repeated more than 2, 5, 10, 100, or 10⁴ times.

In another embodiment of the invention, the treatment session is repeated less than 5, 10, 100, 10⁴, or 10⁵ times.

In another embodiment of the invention, the number of treatment session and/or sequences is adjusted to reach maximum treatment efficacy, *i.e.* for example to reach the disappearance or destruction of a disease, of the portion of the body part comprising the pathological cells, or of a tumor.

In still another embodiment of the invention, the number of treatment session(s) and/or sequence(s) is adjusted to reach minimal treatment toxicity, *i.e.* for example to avoid the disappearance or destruction of the portion of the body part comprising the healthy cells.

The invention relates to magnetic nanoparticles, in particular magnetosomes, for use, wherein the first step is a heating step and/or a dissociation step.

In one embodiment of the invention, the heating step has a duration t₁ₐ, also designated as duration of the heating step or heating time.

In some cases, the duration of the heating step corresponds to the duration of the temperature increase of the body part, magnetic nanoparticle(s), in particular magnetosome(s), or the region of the magnetic nanoparticles, in particular magnetosomes. It is preferentially comprised between the time, tᵢ₁ₐ, of: i), laser start, ii), switching on of the laser equipment or apparatus, or iii), the beginning of irradiation by the laser radiation, preferentially of the body part, magnetic nanoparticle(s), in particular magnetosome,(s), or region of magnetic nanoparticles, in particular magnetosomes, and the time, t_{f1a}, of: i), laser stop, ii), switching off of the laser equipment or apparatus, iii), ending of irradiation by the laser radiation, or iv), irradiation by laser radiation with a lower power than at time t₁ₐ, preferentially of the body part, magnetic nanoparticle(s), in particular magnetosome(s), or region of magnetic nanoparticles, in particular magnetosomes.

In some cases, the magnetic nanoparticles, in particular the magnetosomes, will start to produce or induce an increase in temperature of the body part, magnetic nanoparticle(s), in particular magnetosome(s), or region of magnetic nanoparticles, in particular magnetosomes, a certain time Δtᵢ₁ₐ following tᵢ₁ₐ.

In some cases, the magnetic nanoparticles, in particular the magnetosomes, will stop producing or inducing an increase in temperature of the body part, magnetic nanoparticle(s), in particular magnetosome(s), or region of magnetic nanoparticles, in particular magnetosomes, a certain time Δt_{f1a} following t_{f1a}.

In some cases, Δtᵢ₁ₐ and/or Δt_{f1a} can be larger than 10⁻⁹, 10⁻⁶, 10⁻³, 10⁻¹, 1, or 10 second(s). This long delay may, in some cases, be due to the absorption of laser radiation by the body part not comprising the magnetic nanoparticles, in particular magnetosomes, or by other substances than the magnetic nanoparticles, in particular magnetosomes. This long delay may, in some other cases, be due to the absorption of laser radiation by the magnetic nanoparticles, in particular magnetosomes, or body part comprising the magnetic nanoparticles, in particular magnetosomes, which is not immediately transformed or transferred into heat.

In some other cases, Δtᵢ₁ₐ and/or Δt_{f1a} can be lower than 10⁻⁹, 10⁻⁶, 10⁻³, 10⁻¹, 1, or 10 second(s). This short delay may, in some cases, be due to the absorption of laser radiation by the magnetic nanoparticles, in particular magnetosomes, and to its rapid transformation or transfer into heat.

In still some other cases, the value(s) of Δtᵢ₁ₐ and/or Δt_{f1a} can depend upon organization of magnetic nanoparticles, in particular magnetosomes, transfer of heat from the magnetic nanoparticles, in particular magnetosomes, to the surrounding or environment of the magnetic nanoparticles, in particular magnetosomes, or to the intrinsic properties of the magnetic nanoparticles, in particular magnetosomes, such as size, ferrimagnetic properties, crystallization. In some cases, Δtᵢ₁ₐ and/or Δt_{f1a} can be decreased by improving the crystallization of magnetic nanoparticles, in particular magnetosomes, by increasing the size of magnetic nanoparticles, in particular magnetosomes, by reducing the size distribution of magnetic nanoparticles, in particular magnetosomes, or by improving the spatial distribution of magnetic nanoparticles, in particular magnetosomes, preferentially by reducing the aggregation of magnetic nanoparticles, in particular magnetosomes, preferentially in the body part.

In the experimental example, Δt_{f1a} is equal to 0 second for 0.5 and 1 mg/mL of M-CMD and between 6 and 16 seconds for N-CMD. It indicates that some nanoparticles, which are preferentially chemically synthesized or which are not well crystallized, continue to heat after the laser has been switched off. This is a property that should usually be avoided since it can yield overheating and prevent an accurate control over the temperature reached during the heating steps. It also indicates that some nanoparticles, which are preferentially produced biologically or by living organisms or are well crystallized, stop heating immediately after the laser has been switched off. This is a property that should usually be sought or looked after since it can prevent overheating and enable an accurate control over the temperature reached during the heating steps.

In one embodiment of the invention, the dissociation step has a duration t_{1b}, also designated as duration of the dissociation step or dissociation time.

In some cases, the duration of the dissociation step corresponds to the duration of the dissociation of the compound from the magnetic nanoparticle, in particular magnetosome. It is preferentially comprised between the time, t_{i1b}, of laser start, switching on of the laser equipment or apparatus, the beginning of irradiation by the laser radiation, preferentially of the body part, magnetic nanoparticle(s), in particular magnetosome(s), or region of magnetic nanoparticles, in particular magnetosomes, and the time, t_{f1b}, of laser stop, switching off of the laser equipment or apparatus, or the ending of irradiation by the laser radiation.

In some cases, the magnetic nanoparticles, in particular the magnetosomes, will start producing or inducing the dissociation of the compound from the magnetic nanoparticle, in particular magnetosome, a certain time Δt_{i1b} before or following t_{i1b}.

In some cases, the magnetic nanoparticle, in particular magnetosome, will stop to producing or inducing the dissociation of the compound from the magnetic nanoparticle, in particular magnetosome, a certain time Δt_{f1b} before or following t_{f1b}.

In some cases, Δt_{i1b} and/or Δt_{f1b} can be larger than 10⁻⁹, 10⁻⁶, 10⁻³, 10⁻¹, 1, or 10 second(s). This long delay may, in some cases, be due to the absorption of laser radiation by the body part not comprising the magnetic nanoparticles, in particular magnetosomes, or by other substances than the magnetic nanoparticle, in particular magnetosome. This long delay may, in some other cases, be due to the absorption of laser radiation by the magnetic nanoparticle, in particular magnetosome, or body part comprising the magnetic nanoparticles, in particular magnetosomes, which does not immediately induce the dissociation of the compound from the magnetic nanoparticle, in particular magnetosome.

In some other cases, Δt_{i1b} and/or Δt_{f1b} can be lower than 10⁻⁹, 10⁻⁶, 10⁻³, 10⁻¹, 1, or 10 second(s). This short delay may, in some cases, be due to the absorption of laser radiation by the magnetic nanoparticle, in particular magnetosome, which rapidly leads to the dissociation of the compound from the magnetic nanoparticle, in particular magnetosome.

In still some other cases, the value(s) of Δt_{i1b} and/or Δt_{f1b} can depend upon organization of the magnetic nanoparticle, in particular magnetosome, the strength of the link between the compound and the magnetic nanoparticle, in particular magnetosome, the distance between the compound and the magnetic nanoparticle, in particular magnetosome, the properties of the magnetic nanoparticle, in particular magnetosome, such as size, ferrimagnetic properties, or crystallization. In some cases, Δt_{i1b} and/or Δt_{f1b} can be decreased by improving crystallization of magnetic nanoparticle, in particular magnetosome, by increasing the size of magnetic nanoparticles, in particular magnetosomes, by reducing the size distribution of magnetic nanoparticles, in particular magnetosomes, by improving the spatial distribution of magnetic nanoparticles, in particular magnetosomes, preferentially by reducing the aggregation of magnetic nanoparticles, in particular magnetosomes, preferentially in the body part, by adjusting the strength or distance between the compound and the magnetic nanoparticle, in particular magnetosome, so that the irradiation by the laser radiation induces the dissociation of the compound from the magnetic nanoparticle, in particular magnetosome, rapidly after the irradiation by the laser radiation.

In another embodiment of the invention, the first step has a duration, which is equal to t₁ₐ or t_{1b}, or which is larger than t₁ₐ or t_{1b}, or which is lower than t₁ₐ+t_{1b}.

In one embodiment of the invention, the heating step is the step during which or in which the body part is heated, preferentially by more than 10⁻⁶, 10⁻³, 10⁻¹, 1, 2, 5, 10, or 15 °C, preferentially above physiological temperature or above the temperature of the body part not comprising the magnetic nanoparticle, in particular magnetosome, or above the temperature of the body part reached before the irradiation by the laser radiation.

In one embodiment of the invention, the dissociation step is the step during which or in which the compound dissociates from the magnetic nanoparticle, in particular magnetosome. In some cases, the compound dissociates from the magnetic nanoparticle, in particular magnetosome when more than 0.1, 1, 10, 25, 50, 75, or 90% of compounds are dissociated from the magnetic nanoparticle, in particular magnetosome.

Preferentially, the percentage of dissociation means or is the percentage of dissociation of the compounds from the magnetic nanoparticles, in particular magnetosomes, which may also be designated as the percentage of dissociation.

Preferentially, the variation of the percentage of dissociation is the variation with time, most preferentially during the dissociation or non-dissociation step, of the percentage of dissociation.

Preferentially, the gradient of the percentage of dissociation is the slope of the variation with time, most preferentially during the dissociation or non-dissociation step, of the percentage of dissociation. Most preferentially, this gradient is the absolute value of the slope of the variation with time of the percentage of dissociation.

In some cases, the percentage of dissociation of the compounds from the magnetic nanoparticle, in particular magnetosome, can correspond to or be the number of compounds not linked or not bound to the magnetic nanoparticle, in particular magnetosome, preferentially following the irradiation by the laser radiation, divided by the number of compounds linked or bound to the magnetic nanoparticle, in particular magnetosome, preferentially before or without the irradiation by the laser radiation.

In one embodiment of the invention, the compound comprises at least one substance capable of establishing weak interactions or covalent bonds with the magnetic nanoparticle, in particular magnetosome.

In one embodiment of the invention, the compound comprises at least one compound capable of being chemisorbed or physisorbed on the magnetic nanoparticle, in particular magnetosome, or on the surface of the magnetic nanoparticle, in particular magnetosome.

In one embodiment of the invention, the compound comprises at least one substance capable of establishing interactions or bonds with atoms, ions, molecules, which may be in or on the surface of the magnetic nanoparticle, in particular magnetosome.

In one embodiment of the invention, the compound comprises at least one substance, atom, ion, or chemical function such as an acid, carboxylic acid, phosphoric acid, or sulfonic acid function, wherein the substance, atom or ion included in the coating is capable of establishing interactions or bonds with the magnetic nanoparticle, in particular magnetosome.

In one embodiment of the invention, the percentage of dissociation is defined as the ratio between the number or mass of compounds that have dissociated following the irradiation by the laser radiation divided by the number or mass of compounds that are linked to the magnetic nanoparticle, in particular magnetosome, before the irradiation by the laser radiation.

In another embodiment of the invention, the compound is dissociated from the magnetic nanoparticle, in particular magnetosome, when the magnetic nanoparticle, in particular magnetosome, can be magnetically separated from the compound. In this case, a magnet can preferentially be used to attract the magnetic nanoparticle, in particular magnetosome, preferentially using a magnetic field whose intensity varies spatially, preferentially using a magnet with a lower strength than 10, 1, 10⁻¹, 10⁻³, or 10⁻⁹ T, where this strength is preferentially measured at the surface or near the magnet and decreases with increasing distance away from the magnet. By contrast to the magnetic nanoparticle, in particular magnetosome, the compound is preferentially not attracted by the magnet. Hence, by using magnetic separation, a certain distance, preferentially larger than 1, 10, 10³, or 10⁵ nm, can be created between the compound and the magnetic nanoparticle, in particular magnetosome, and the magnetic nanoparticle, in particular magnetosome, can then be separated or isolated from the compound.

In another embodiment of the invention, the compound is dissociated from the magnetic nanoparticle, in particular magnetosome, when the percentage of compounds associated or linked to the magnetic nanoparticle, in particular magnetosome, or located at a distance of less than 10, 1 , 10⁻¹, 10⁻³, 10⁻⁶, or 10⁻⁹ cm from the magnetic nanoparticle, in particular magnetosome, is lower than 99, 90, 75, 50, 30, 20, 10, 5, 2, or 1%, where this percentage can represent the ratio between the number or mass of compounds linked or associated to the magnetic nanoparticle, in particular magnetosome, before magnetic separation and the number or mass of compounds linked or associated to the magnetic nanoparticle, in particular magnetosome, after magnetic separation.

In another embodiment of the invention, the compound is not dissociated from the magnetic nanoparticle, in particular magnetosome, or non-dissociated, when the magnetic nanoparticle, in particular magnetosome, can't be magnetically separated from the compound, preferentially using a magnet with a strength that is lower than 10, 1, 10⁻¹, 10⁻³, or 10⁻⁹ T.

In still another embodiment of the invention, the compound is not dissociated from the magnetic nanoparticle, in particular magnetosome, or non-dissociated when the percentage of compounds associated or linked to the magnetic nanoparticle, in particular magnetosome, or located at a distance of less than 10, 1 , 10⁻¹, 10⁻³, 10⁻⁶, or 10⁻⁹ cm from the magnetic nanoparticle, in particular magnetosome, is larger than 99, 90, 75, 50, 30, 20, 10, 5, 2, or 1%, where this percentage can represent the ratio between the number or mass of compounds linked or associated to the magnetic nanoparticle, in particular magnetosome, before magnetic separation and the number or mass of compounds linked or associated to the magnetic nanoparticle, in particular magnetosome, after magnetic separation.

The invention relates to magnetic nanoparticles, in particular magnetosomes, for use, wherein the first step can be divided between a first sub-step during which or in which a first portion of the body part is irradiated by the laser radiation, and a second sub-step during which or in which temperature increase and/or dissociation of a compound from the magnetic nanoparticles, in particular magnetosomes, occurs in another portion of the body part, which has not been irradiated by the laser radiation during the first sub-step.

In one embodiment of this invention, the body part is divided between a portion of the body part irradiated by the laser radiation or irradiated and a portion of the body part not irradiated by the laser radiation or not irradiated.

In still another embodiment of the invention, the portion of the body part irradiated by the laser radiation, also designated in some cases as body part or portion of body part, is the region where a first portion of magnetic nanoparticles, in particular magnetosomes, is located preferentially in the body part or individual. It can also be designated in some cases as first region of magnetic nanoparticles, in particular magnetosomes. In some cases, the first region of magnetic nanoparticles, in particular magnetosomes, corresponds to or is the volume comprising less than 99, 90, 80, 70, 50, 40, 20, 10, 5, or 1% of the magnetic nanoparticles, in particular magnetosomes, which have been administered to the individual or body part or are comprised in the body part or individual. This percentage can represent the ratio between the quantity of magnetic nanoparticles, in particular magnetosomes, comprised in the first region of magnetic nanoparticles, in particular magnetosomes, and the quantity of magnetic nanoparticles, in particular magnetosomes, administered to or comprised in the individual or body part.

In still another embodiment of this invention, the portion of the body part not irradiated by the laser radiation or not irradiated, also designated in some cases as body part or portion of body part, is the region were a second portion of magnetic nanoparticles, in particular magnetosomes, is located preferentially in the individual. It can also be designated in some cases as second region of magnetic nanoparticles, in particular magnetosomes. In some cases, the second region of magnetic nanoparticles, in particular magnetosomes, corresponds to or is the volume comprising more than 99, 90, 80, 70, 50, 40, 20, 10, 5, 1, 10⁻³, or 10⁻⁶ % of magnetic nanoparticles, in particular magnetosomes, which are not located in the first region of magnetic nanoparticles, in particular magnetosomes, or which are located outside of the first region of magnetic nanoparticles, in particular magnetosomes, or which are located in a different region from the first region of magnetic nanoparticles, in particular magnetosomes. This percentage can represent the ration between the quantity of magnetic nanoparticles, in particular magnetosomes, which are located outside of the first region of magnetic nanoparticles, in particular magnetosomes, or which are located in the second region of magnetic nanoparticles, in particular magnetosomes, divided by the quantity of magnetic nanoparticles, in particular magnetosomes, comprised in or administered to the individual or body part.

In still another embodiment of the invention, the duration between the first and second sub-steps is lower than 1, 10⁻³, 10⁻⁶, or 10⁻⁹ second(s).

In still another embodiment of the invention, the mechanism by which the magnetic nanoparticles, in particular the magnetosomes, located in the second region of magnetic nanoparticles, in particular magnetosomes, can be heated or prone to the dissociation of the compound can involve conduction of heat or conduction or transmission of an electromagnetic wave between the first and second regions of magnetic nanoparticles, in particular magnetosomes. In case of large concentrations of magnetic nanoparticles, in particular magnetosomes, or magnetic nanoparticles, in particular magnetosomes, connected to each other, preferentially in chains, or densely packed, heat conduction and/or transmission or conduction of the electromagnetic wave can be favored. In some cases, heat conduction and/or transmission or conduction of the electromagnetic wave can take place between the magnetic nanoparticles, in particular magnetosomes, between the magnetic nanoparticles, in particular the magnetosomes, and tissues or water comprised in the body part, or between water molecules in the body part, preferentially between moving water molecules, where the movement of water molecules is preferentially increased by heat.

The invention also relates to magnetic nanoparticles, in particular magnetosomes, for use, wherein the second step is a cooling step and/or a non-dissociation step.

In one embodiment of the invention, the cooling step has a duration t₂ₐ, also designated as duration of the cooling step or cooling time.

In some cases, the duration of the cooling step corresponds to the duration of the temperature decrease of the body part, magnetic nanoparticle(s), in particular magnetosome(s), or region of magnetic nanoparticles, in particular magnetosomes. It is preferentially comprised between the time, tᵢ₂ₐ, of laser stop, switching off of the laser equipment or apparatus, decrease of the laser power, laser power density, or laser intensity, or the end of irradiation by the laser radiation, and the time, t_{f2a}, of laser start, switching on of the laser equipment or apparatus, or the beginning of irradiation by the laser radiation.

In one embodiment of the invention, the temperature decrease designates the temperature decrease of the body part, portion of the body part comprising the magnetic nanoparticles, in particular the magnetosomes, region of the magnetic nanoparticles, in particular the magnetosomes. The temperature decrease can be assimilated to or correspond to the magnitude of the temperature decrease occurring in the second step, which is preferentially the absolute value of the difference between the maximum and minimum temperature reached in the second step, which is also in some cases designated as temperature variation or temperature variation during or occurring in the second step.

In some cases, the magnetic nanoparticles, in particular the magnetosomes, will start producing or inducing a decrease in temperature of the body part, magnetic nanoparticle(s), in particular magnetosome(s), or region of magnetic nanoparticles, in particular magnetosomes, a certain time Δtᵢ₂ₐ following or preceding tᵢ₂ₐ.

In some cases, the magnetic nanoparticles, in particular magnetosomes, will stop producing or inducing a decrease in temperature of the body part, magnetic nanoparticle(s), in particular magnetosome(s), or region of magnetic nanoparticles, in particular magnetosomes, a certain time Δt_{f2a} following or preceding t_{f2a}.

In some cases, Δtᵢ₂ₐand/or Δt_{f2a} can be larger than 10⁻⁹, 10⁻⁶, 10⁻³, 10⁻¹, 1, or 10 second(s). This long delay may, in some cases, be due to the slow or inefficient release or heat transfer from the magnetic nanoparticles, in particular magnetosomes, to the environment or medium surrounding the magnetic nanoparticles, in particular magnetosomes, or to the body part not comprising the magnetic nanoparticles, in particular magnetosomes. In some cases, this release or transfer of heat can be slower than 10⁻²⁰, 10⁻¹⁰, 10⁻⁴, 10⁻², 10⁻¹, 1, 10, 10², 10⁵, 10¹⁰, or 10²⁰ °C per second.

In some other cases, Δtᵢ₂ₐand/or Δt_{f2a} can be lower than 10⁻⁹, 10⁻⁶, 10⁻³, 10⁻¹, 1, or 10 second(s). This short delay may, in some cases, be due to the fast or efficient release or transfer of heat from the magnetic nanoparticle, in particular magnetosome, to the environment or medium surrounding the magnetic nanoparticle, in particular magnetosome, or to the body part not comprising the magnetic nanoparticle, in particular magnetosome. In some cases, this release or transfer of heat can be faster than 10⁻²⁰, 10⁻¹⁰, 10⁻⁴, 10⁻², 10⁻¹, 1, 10, 10², 10⁵, 10¹⁰, or 10²⁰ °C per second.

In still some other cases, the value(s) of Δtᵢ₂ₐand/or Δt_{f2a} can depend upon organization of magnetic nanoparticle, in particular magnetosome, heat transfer from the magnetic nanoparticle, in particular magnetosome, to the surrounding or environment of the magnetic nanoparticle, in particular magnetosome, or to the body part not comprising the magnetic nanoparticle, in particular magnetosome. It can also depend on the properties of the magnetic nanoparticle, in particular magnetosome, such as size, ferrimagnetic properties, crystallization. In some cases, Δtᵢ₂ₐand/or Δt_{f2a} can be decreased by improving crystallization of magnetic nanoparticle, in particular magnetosome, increasing the size of magnetic nanoparticles, in particular magnetosomes, reducing the size distribution of magnetic nanoparticles, in particular magnetosomes, improving the spatial distribution of magnetic nanoparticles, in particular magnetosomes, preferentially by reducing aggregation of magnetic nanoparticles, in particular magnetosomes, preferentially in the body part.

In the experimental example, Δt_{f2a} is equal to 0 second for 0.5 and 1 mg/mL of M-CMD and between 6 and 25 seconds for N-CMD. It indicates that some nanoparticles, which are preferentially chemically synthesized or which are not well crystallized, do not cool down within the same period of time as that of laser switch off. This is a property that should usually be avoided since it can yield overcooling and prevents an accurate control over the temperature reached during the cooling steps. It also indicates that some nanoparticles, which are preferentially produced biologically or by living organisms or are well crystallized, cool down within the same period of time as that of laser switch off. This is a property that should usually be sought after since it can prevent overcooling and enable an accurate control over the temperature reached during the cooling steps.

In one embodiment of the invention, the non-dissociation step has a duration t_{2b}, also designated as duration of the non-dissociation step or non-dissociation time.

In some cases, the duration of the non-dissociation step corresponds to the duration of the non-dissociation of the compound from the magnetic nanoparticle, in particular magnetosome. It is preferentially comprised between the time, t_{i2b}, of laser stop, switching off of the laser equipment or apparatus, the end of irradiation by the laser radiation, and the time, t_{f2b}, of laser start, switching on of the laser equipment or apparatus, the beginning of irradiation by the laser radiation.

In some cases, the magnetic nanoparticle, in particular magnetosome, will start producing or inducing the non-dissociation of the compound from the magnetic nanoparticle, in particular magnetosome, a certain time Δt_{i2b} following or preceding t_{i2b}.

In some cases, the magnetic nanoparticle, in particular magnetosome, will stop producing or inducing the non-dissociation of the compound from the magnetic nanoparticle, in particular magnetosome, a certain time Δt_{f2b} following or preceding t_{f2b}.

In some cases, Δt_{i2b} and/or Δt_{f2b} can be larger than 10⁻⁹, 10⁻⁶, 10⁻³, 10⁻¹, 1, or 10 second(s). This long delay may for Δt_{i2b} be due to the continuation of the dissociation of the compound from the magnetic nanoparticle, in particular magnetosome, a long time after the laser has been switched off or reduced in power. This long delay may for Δt_{f2b} be due to the beginning of the dissociation of the compound a long time after the laser has been switched on.

In some other cases, Δt_{i2b} and/or Δt_{f2b} can be lower than 10⁻⁹, 10⁻⁶, 10⁻³, 10⁻¹, 1, or 10 second(s). This short delay may for Δt_{i2b} be due to the end of the dissociation of the compound from the magnetic nanoparticle, in particular magnetosome, a short time after the laser has been switched off or reduced in power. This sort delay may for Δt_{f2b} be due to the beginning of the dissociation of the compound a short time after the laser has been switched on.

In still some other cases, the value(s) of Δt_{i2b} and/or Δt_{f2b} can depend upon the organization of magnetic nanoparticles, in particular magnetosomes, the strength of the link between the compound and the magnetic nanoparticle, in particular magnetosome, the distance between the compound and the magnetic nanoparticle, in particular magnetosome, the properties of the magnetic nanoparticle, in particular magnetosome, such as size, ferrimagnetic properties, or crystallization. In some cases, Δt_{i2b} and/or Δt_{f2b} can be decreased by improving crystallization of magnetic nanoparticles, in particular magnetosomes, increasing the size of magnetic nanoparticles, in particular magnetosomes, reducing the size distribution of magnetic nanoparticles, in particular magnetosomes, improving the spatial distribution of magnetic nanoparticles, in particular magnetosomes, preferentially by reducing aggregation of magnetic nanoparticles, in particular magnetosomes, preferentially in the body part, by adjusting the strength or distance between the compound and the magnetic nanoparticle, in particular magnetosome, so that the end of irradiation by the laser radiation stops the dissociation or induces the non-dissociation of the compound from the magnetic nanoparticle, in particular magnetosome, a short time after the end of irradiation by the laser radiation.

In another embodiment of the invention, the second step has a duration, which is equal to t₂ₐ or t_{2b}, or which is larger than t₂ₐ or t_{2b}, or which is lower than t₂ₐ+t_{2b}.

In one embodiment of the invention, the cooling step is the step during which or in which the body part is cooled, preferentially by more than 10⁻⁶, 10⁻³, 10⁻¹, 1, 2, 5, 10, or 15 °C, preferentially below the maximum temperature reached during the heating step.

In one embodiment of the invention, the non-dissociation step is the step during which or in which the compound does not dissociate from the magnetic nanoparticle, in particular magnetosome. In some cases, the compound does not dissociate from the magnetic nanoparticle, in particular magnetosome, when the percentage of dissociation is lower than 0.1, 1, 10, 25, 50, 75, or 90%.

In another embodiment of the invention, the time t₁ₐ, t_{1b}, t₂ₐ, or t_{2b}, is shorter than 10⁻³, 10⁻², 10⁻¹, 1, 10, 10², or 10³ minute(s). In some cases, t₁ₐ, t_{1b}, t₂ₐ, or t_{2b}, is shorter than 10⁻⁹, 10⁻⁷, 10⁻⁵, 10⁻³, 10⁻¹, 1, 10, 10³, 10⁵, 10⁷, or 10⁹ seconds.

In still another embodiment of the invention, the time t₁ₐ, t_{1b}, t₂ₐ, or t_{2b}, is longer than 10⁻³, 10⁻², 10⁻¹, 1, 10, 10², or 10³ minute(s). In some cases, t₁ₐ, t_{1b}, t₂ₐ, or t_{2b}, is longer than 10⁻⁹, 10⁻⁷, 10⁻⁵, 10⁻³, 10⁻¹, 1, 10, 10³, 10⁵, 10⁷, or 10⁹ second(s).

In one embodiment of the invention, the time t₁ₐ, t_{1b}, t₂ₐ, or t_{2b}, is shorter than the time of a laser pulse, preferentially by a factor of at least 1.1, 1.5, 2, 5, 10, 10², 10³, 10⁵, 10⁷, 10⁹, 10¹², 10¹⁵, or 10²⁰.

In still another embodiment of the invention, the time t₁ₐ, t_{1b}, t₂ₐ, or t_{2b}, is longer than the time of a laser pulse, preferentially by a factor of at least 1.1, 1.5, 2, 5, 10, 10², 10³, 10⁵, 10⁷, 10⁹, 10¹², 10¹⁵, or 10²⁰.

In still another embodiment of the invention, the pulse is defined as the irradiation by the laser radiation, preferentially on magnetic nanoparticle(s), in particular magnetosome,(s), body part, or region of magnetic nanoparticles, in particular magnetosomes, during a time shorter than 10⁶, 10³, 1, 10⁻³, 10⁻⁶, 10⁻⁹, 10⁻¹², 10⁻¹⁵, or 10⁻²⁰ second(s).

In one embodiment of the invention, the ratio t₁ₐ/t₂ₐ or t_{1b}/t_{2b} is smaller than 10⁻⁹, 10⁻⁶, 10⁻³, 10⁻¹, 1, 10, 10³, 10⁶, or 10⁹.

In still another embodiment of the invention, the ratio t₁ₐ/t₂ₐ or t_{1b}/t_{2b} is larger than 10⁻⁹, 10⁻⁶, 10⁻³, 10⁻¹, 1, 10, 10³, 10⁶, or 10⁹.

In the experimental example, t₁ₐ is comprised between 35 sec. and 150 sec. for 0.5 mg/mL of M-CMD, 21 sec. and 80 sec. for 1 mg/mL M-CMD, and 22 sec. and 45 sec. for N-CMD. In some cases, it is possible to decrease t₁ₐ, preferentially by a factor of more than 1.001, 1.01, 1.1, 1.5, 2, 5, 10, or 10³, and still induce a temperature increase during the heating step. This can preferentially be achieved by: i), increasing the concentration of magnetic nanoparticles, in particular magnetosomes, preferentially by a factor of more than 1.001, 1.01, 1.1, 1.5, 2, 5, 10, or 10³, ii), increasing the laser power, laser power density, or laser intensity, preferentially by a factor of more than 1.001, 1.01, 1.1, 1.5, 2, 5, 10, or 10³, or iii), by reducing the temperature increase or temperature gradient that one wants to achieve during the heating step, preferentially by a factor of more than 1.001, 1.01, 1.1, 1.5, 2, 5, 10, or 10³. In still some other cases, it is possible to increase t₁ₐ, preferentially by a factor of more than 1.001, 1.01, 1.1, 1.5, 2, 5, 10, or 10³, and induce a temperature increase during the heating steps, preferentially without inducing a temperature increase of the body part not comprising the magnetic nanoparticles, in particular magnetosomes. This can preferentially be achieved by: i), decreasing the concentration of the magnetic nanoparticles, in particular magnetosomes, preferentially by a factor of more than 1.001, 1.01, 1.1, 1.5, 2, 5, 10, or 10³, ii), decreasing the laser power, laser power density, or laser intensity, preferentially by a factor of more than 1.001, 1.01, 1.1, 1.5, 2, 5, 10, or 10³, or iii), by increasing the temperature increase or temperature gradient that one wants to achieve during the heating step, preferentially by a factor of more than 1.001, 1.01, 1.1, 1.5, 2, 5, 10, or 10³.

In the experimental example, t₂ₐ is comprised between 60 sec. and 75 sec. for 0.5 mg/mL of M-CMD, 65 sec. and 90 sec. for 1 mg/mL of M-CMD, and 89 sec. and 105 sec. for N-CMD. In some cases, it is possible to decrease t₂ₐ, preferentially by a factor of more than 1.001, 1.01, 1.1, 1.5, 2, 5, 10, or 10³, and still induce a temperature decrease during the cooling step. This can preferentially be achieved by: i), decreasing the concentration of magnetic nanoparticle, in particular magnetosome, preferentially by a factor of more than 1.001, 1.01, 1.1, 1.5, 2, 5, 10, or 10³, ii), decreasing the laser power, laser power density, or laser intensity, preferentially by a factor of more than 1.001, 1.01, 1.1, 1.5, 2, 5, 10, or 10³, or iii), by reducing the temperature decrease or temperature gradient that one wants to achieve during the cooling step, preferentially by a factor of more than 1.001, 1.01, 1.1, 1.5, 2, 5, 10, or 10³. In still some other cases, it is possible to increase t₂ₐ, preferentially by a factor of more than 1.001, 1.01, 1.1, 1.5, 2, 5, 10, or 10³, and induce a temperature decrease during the cooling steps, preferentially without inducing a temperature decrease of the body part not comprising the magnetic nanoparticles, in particular magnetosomes. This can preferentially be achieved by: i), increasing the concentration of magnetic nanoparticles, in particular magnetosomes, preferentially by a factor of more than 1.001, 1.01, 1.1, 1.5, 2, 5, 10, or 10³, ii), increasing the laser power, laser power density, or laser intensity, preferentially by a factor of more than 1.001, 1.01, 1.1, 1.5, 2, 5, 10, or 10³, or iii), by decreasing the temperature decrease or temperature gradient that one wants to achieve during the cooling steps, preferentially by a factor of more than 1.001, 1.01, 1.1, 1.5, 2, 5, 10, or 10³.

In the experimental example, not taking into consideration the first sequence, t₁ₐ/t₂ₐ is comprised between 0.6 and 0.7 for 0.5 mg of M-CMD, equal to 0.3 for 1 mg of M-CMD, and comprised between 0.2 and 0.4 for 1 mg of N-CMD. In some cases, the ratio t_{1a/}t₂ₐ can be increased, preferentially by a factor larger than 1.001, 1.01, 1.1, 1.5, 2, 5, 10, or 10³, preferentially by: i), decreasing the concentration of magnetic nanoparticles, in particular magnetosomes, preferentially by a factor of more than 1.001, 1.01, 1.1, 1.5, 2, 5, 10, or 10³, ii), decreasing the laser power, preferentially by a factor of more than 1.001, 1.01, 1.1, 1.5, 2, 5, 10, or 10³. In some other cases, the ratio t₁ₐ/t₂ₐ can be decreased, preferentially by a factor larger than 1.001, 1.01, 1.1, 1.5, 2, 5, 10, or 10³, preferentially by: i), increasing the concentration of magnetic nanoparticles, in particular magnetosomes, preferentially by a factor of more than 1.001, 1.01, 1.1, 1.5, 2, 5, 10, or 10³, ii), increasing the laser power, preferentially by a factor of more than 1.001, 1.01, 1.1, 1.5, 2, 5, 10, or 10³.

In still another embodiment of the invention, the cooling time is more than 1.001, 1.01, 1.1, 1.5, 2, 5, 10, or 10³ larger in the absence than in the presence of the magnetic nanoparticles, in particular magnetosomes.

In still another embodiment of the invention, the heating time is more than 1.001, 1.01, 1.1, 1.5, 2, 5, 10, or 10³ larger in the presence than in the absence of the magnetic nanoparticles, in particular magnetosomes.

The invention also relates to magnetic nanoparticles, in particular magnetosomes, for use, wherein the heating step produces a temperature increase of more than 1 °C of the body part.

In one embodiment of the invention, the heating step produces a temperature increase of/in the body part, magnetic nanoparticle, in particular magnetosome, or region of magnetic nanoparticle, in particular magnetosome, of more than 10⁻⁹, 10⁻⁶, 10⁻³, 10⁻¹, 1, 2, 5, or 10 °C, preferentially above the physiological temperature, or preferentially above the temperature of the body part before or without irradiation by the laser radiation.

In the experimental example, apart from the first sequence, the temperature increase reached during the second to eight heating steps, which is equal to the maximum temperature minus the minimum temperature of the heating steps, is equal to an average of: i), 8 °C for 0.5 and 1 mg/mL of M-CMD, and ii) 12 °C for 1 mg/mL of N-CMD, where M-CMD and N-CMD are exposed to a laser power density of 2 W/cm². In some cases, it is possible to reach larger values of temperature increase, preferentially by a factor of more than 1.001, 1.01, 1.1, 1.5, 2, 5, 10, or 10³, by: i), increasing the concentration of magnetic nanoparticles, in particular magnetosomes, preferentially by a factor of more than 1.001, 1.01, 1.1, 1.5, 2, 5, 10, or 10³, ii), increasing the duration of the heating step, preferentially by a factor of more than 1.001, 1.01, 1.1, 1.5, 2, 5, 10, or 10³, iii), by increasing the temperature increase or temperature gradient that one wants to achieve during the heating step, preferentially by a factor of more than 1.001, 1.01, 1.1, 1.5, 2, 5, 10, or 10³, or, iv), by using magnetic nanoparticles, in particular magnetosomes, that continue to heat, preferentially by more than 10⁻⁹, 10⁻⁶, 10⁻³, 10⁻¹, 1, 10, 10³, or 10⁶ °C, after the laser has been switched off, preferentially more 10⁻⁹, 10⁻⁶, 10⁻³, 10⁻¹, 1, 10, 10³, or 10⁶ second(s) after the laser has been switched off.

In one embodiment of the invention, the heating step produces a temperature gradient of/in the body part preferentially comprising the magnetic nanoparticles, in particular magnetosomes, measured at any given time of the heating step, (ΔT/δt)t₁ₐ, which is larger than 10⁻⁹, 10⁻⁶, 10⁻³, 10⁻¹, 1, 2, 5, or 10 °C/sec, or which is preferentially larger, most preferentially larger by a factor of more than 1.1, 1.2, 2, 5, 10, 50, 10², 10³, or 10⁵, than any temperature gradient occurring in the body part without the magnetic nanoparticles, in particular magnetosomes. In some cases, (ΔT/δt)t₁ₐ can be defined as the slope of the tangent of the temperature variation with time occurring during the heating step.

In one embodiment of the invention, (ΔT/δt)t₁ₐ and (ΔT/δt)t₂ₐ are the absolute values of the slopes of the tangent of the temperature variation with time, measured at t₁ₐ and t_{1b}, respectively.

In one embodiment of the invention, (ΔT/δt)t₁ₐ decreases, preferentially by a factor of more than 1.001, 1.01, 1.1, 1.5, 2, 5, 10, or 100, when t₁ₐ increases, preferentially by a factor of more than 1.001, 1.01, 1.1, 1.5, 2, 5, 10, or 100. This can mean that (ΔT/δt)t₁ₐ is larger at the beginning than at the end of the first step. This can mean that the initial slope of the temperature variation with time, measured at the beginning of the heating step, is the largest value of (ΔT/δt)t₁ₐ. In some cases, the laser power, laser power density, laser intensity, and/or concentration of magnetic nanoparticles, in particular magnetosomes, can be increased, preferentially by a factor of more than 1.001, 1.01, 1.1, 1.2, 1.5, 2, 5, 10, or 100, to decrease the variation between the different values of (ΔT/δt)t₁ₐ, measured during the heating step. In some cases, (ΔT/δt)t₁ₐ varies by less than 99, 90, 85, 75, 60, 50, 30, 20, 10, 5, 2, 1, or 10⁻¹ %. This percentage can be equal to [(ΔT/δt)t₁ₐ₁-(ΔT/δt)t₁ₐ₂]/(ΔT/δt)t₁ₐ₁, where (ΔT/δt)t₁ₐ₁ and (ΔT/δt)t₁ₐ₂ are the temperature gradients measured at two different times t₁ₐ₁ and t₁ₐ₂ of the heating step.

In still another embodiment of the invention, the average temperature gradient of the heating step is the average value of (ΔT/δt)t₁ₐ measured at different times t₁ₐ, where the different values of t₁ₐ used to measure this average value are preferentially separated by similar durations, or by durations that are longer than 10⁻⁹, 10⁻⁶, 10⁻³, 10⁻¹, 1, 5, 10, 50, or 100 second(s). The average value of (ΔT/δt)t₁ₐ is designated as Av[(ΔT/δt)t₁ₐ]. In some cases, Av[(ΔT/δt)t₁ₐ] is larger than 10⁻⁹, 10⁻⁶, 10⁻³, 10⁻¹, 1, 2, 5, or 10 °C/sec. In some other cases, Av[(ΔT/δt)t₁ₐ] is lower than 10⁶, 10³, 10, 5, 2, 1, or 10⁻³ °C/sec.

In the experimental example, the average temperature gradient of the heating step is comprised between 0.13 and 0.21 °C/sec. for 0.5 mg of M-CMD, 0.22 and 0.4 °C/sec. for 1 mg of M-CMD, and 0.33 and 0.56 °C/sec. for N-CMD. In some cases, the temperature gradient or average temperature gradient of the heating step can be increased, preferentially by a factor of more than 1.001, 1.01, 1.1, 1.5, 2, 5, 10, or 10³, by: i), increasing the concentration of the magnetic nanoparticle, in particular magnetosome, preferentially by a factor of more than 1.001, 1.01, 1.1, 1.5, 2, 5, 10, or 10³, or ii), increasing the laser power or laser power density, preferentially by a factor of more than 1.001, 1.01, 1.1, 1.5, 2, 5, 10, or 10³. In some other cases, the temperature gradient or average temperature gradient of the heating step can be decreased, preferentially by a factor of more than 1.001, 1.01, 1.1, 1.5, 2, 5, 10, or 10³, by: i), decreasing the concentration of the magnetic nanoparticle, in particular magnetosome, preferentially by a factor of more than 1.001, 1.01, 1.1, 1.5, 2, 5, 10, or 10³, or ii), decreasing the laser power or laser power density, preferentially by a factor of more than 1.001, 1.01, 1.1, 1.5, 2, 5, 10, or 10³.

The invention also relates to magnetic nanoparticles, in particular magnetosomes, for use, wherein the cooling step produces a temperature decrease of more than 1 °C of the body part.

In one embodiment of the invention, the cooling step produces a temperature decrease of more than 10⁻⁹, 10⁻⁶, 10⁻³, 10⁻¹, 1, 2, 5, or 10 °C of the body part, magnetic nanoparticle(s), in particular magnetosome(s), or region of magnetic nanoparticles, in particular magnetosomes, preferentially below the maximum temperature reached during the heating step.

In the experimental example, the temperature decrease reached during the cooling steps, which is equal to the absolute value of the minimum temperature minus the maximum temperature of the cooling steps, is equal to an average of: i), 8 °C for 0.5 and 1 mg/mL of M-CMD, and ii) 12 °C for 1 mg/mL of N-CMD, where M-CMD and N-CMD are not irradiated by the laser radiation during the cooling steps. In some cases, it is possible to reach larger values of temperature decrease, preferentially by a factor of more than 1.001, 1.01, 1.1, 1.5, 2, 5, 10, or 10³, by: i), increasing or decreasing the concentration of the magnetic nanoparticles, in particular magnetosomes, preferentially by a factor of more than 1.001, 1.01, 1.1, 1.5, 2, 5, 10, or 10³, ii), increasing the duration of the cooling step, preferentially by a factor of more than 1.001, 1.01, 1.1, 1.5, 2, 5, 10, or 10³, or iii), by increasing the temperature decrease or temperature gradient that one wants to achieve during the cooling step, preferentially by a factor of more than 1.001, 1.01, 1.1, 1.5, 2, 5, 10, or 10³.

In one embodiment of the invention, the cooling step produces a temperature gradient of/in the body part, measured at any given time of the cooling step, (ΔT/δt)t₂ₐ or the absolute value of (ΔT/δt)t₂ₐ, which is larger than 10⁻⁹, 10⁻⁶, 10⁻³, 10⁻¹, 1, 2, 5, or 10 °C/sec, or which is preferentially larger, most preferentially larger by a factor of more than 1.1, 1.2, 2, 5, 10, 50, 10², 10³, or 10⁵, than any temperature gradient occurring in the body part without the magnetic nanoparticles, in particular magnetosomes. In some cases, (ΔT/δt)t₂ₐ can be defined as the slope or the absolute value of the slope of the tangent of the temperature variation with time occurring during the cooling step.

In one embodiment of the invention, (ΔT/δt)t₂ₐ decreases, preferentially by a factor of more than 1.001, 1.01, 1.1, 1.5, 2, 5, 10, or 100, when t₂ₐ increases, preferentially by a factor of more than 1.001, 1.01, 1.1, 1.5, 2, 5, 10, or 100. This can mean that (ΔT/δt)t₂ₐ is larger at the beginning than at the end of the second or cooling step. This can mean that the initial slope of the temperature variation with time, measured at the beginning of the cooling step, is the largest value of (ΔT/δt)t₂ₐ. In some cases, the laser power, laser power density, laser intensity, and/or concentration of magnetic nanoparticles, in particular magnetosomes, can be increased, preferentially by a factor of more than 1.001, 1.01, 1.1, 1.2, 1.5, 2, 5, 10, or 100, to decrease the variation between the different values of (ΔT/δt)t₂ₐ, measured during different times of the cooling step. In some cases, (ΔT/δt)t₂ₐ varies by less than 99, 90, 85, 75, 60, 50, 30, 20, 10, 5, 2, 1, or 10⁻¹ %. This percentage can be equal to [(ΔT/δt)t₂ₐ₁-(ΔT/δt)t₂ₐ₂]/(ΔT/δt)t₂ₐ₁, where (ΔT/δt)t₂ₐ₁ and (ΔT/δt)t₂ₐ₂ are the temperature gradients measured at two different times t₂ₐ₁ and t₂ₐ₂ of the cooling step.

In still another embodiment of the invention, the average temperature gradient of the cooling step is the average value of (ΔT/δt)t₂ₐ measured at different times t₂ₐ, where the different values of t₂ₐ used to measure this average value are preferentially separated by similar durations, or by durations that are longer than 10⁻⁹, 10⁻⁶, 10⁻³, 10⁻¹, 1, 5, 10, 50, or 100 second(s). The average value of (ΔT/δt)t₂ₐ is designated as Av[(ΔT/δt)t₂ₐ]. In some cases, Av[(ΔT/δt)t₂ₐ] is larger than 10⁻⁹, 10⁻⁶, 10⁻³, 10⁻¹, 1, 2, 5, or 10 °C/sec. In some other cases, Av[(ΔT/δt)t₂ₐ] is lower than 10⁶, 10³, 10, 5, 2, 1, or 10⁻³ °C/sec.

In the experimental example, the average temperature gradient of the cooling step is comprised between 0.11 and 0.13 °C/sec. for 0.5 mg of M-CMD, 0.09 and 0.12 °C/sec. for 1 mg of M-CMD, and 0.11 and 0.14 °C/sec. for N-CMD. In some cases, the temperature gradient or average temperature gradient of the cooling step can be increased, preferentially by a factor of more than 1.001, 1.01, 1.1, 1.5, 2, 5, 10, or 10³, by: i), decreasing the concentration of magnetic nanoparticles, in particular magnetosomes, preferentially by a factor of more than 1.001, 1.01, 1.1, 1.5, 2, 5, 10, or 10³, or ii), decreasing the laser power or laser power density, preferentially by a factor of more than 1.001, 1.01, 1.1, 1.5, 2, 5, 10, or 10³. In some other cases, the temperature gradient or average temperature gradient of the heating step can be decreased, preferentially by a factor of more than 1.001, 1.01, 1.1, 1.5, 2, 5, 10, or 10³, by: i), increasing the concentration of magnetic nanoparticles, in particular magnetosomes, preferentially by a factor of more than 1.001, 1.01, 1.1, 1.5, 2, 5, 10, or 10³, or ii), increasing the laser power or laser power density, preferentially by a factor of more than 1.001, 1.01, 1.1, 1.5, 2, 5, 10, or 10³.

In one embodiment of the invention, the magnitudes of the temperature increase reached during the heating step, ΔT_{HS}, and the magnitude of the temperature decrease reached during the cooling step, ΔT_{CS}, differ by less than 10⁶, 10³, 10², 10, or 1 %, where this percentage can be equal to the absolute values of (ΔT_{HS}-ΔT_{CS})/ΔT_{HS} or (ΔT_{CS}-ΔT_{HS})/ΔT_{CS}.

In still another embodiment of the invention, the largest temperature gradient is that reached at the beginning of the heating or cooling step.

The invention also relates to magnetic nanoparticles, in particular magnetosomes, for use, wherein the dissociation step produces a percentage of dissociation of the compounds from the magnetic nanoparticles, in particular magnetosomes, which is larger than 10⁻⁴ %.

In one embodiment of the invention, the dissociation step produces a percentage of dissociation of the compounds from the magnetic nanoparticles, in particular magnetosomes, which is larger than 10⁻²⁰, 10⁻¹⁰, 10⁻⁴, 10⁻¹, 1, 5, 10, or 50 %.

In one embodiment of the invention, the dissociation step produces a gradient of the percentage of dissociation, preferentially of/in the body part comprising the magnetic nanoparticles, in particular magnetosomes, measured at any given time of the dissociation step, (ΔD/δt)t_{1b}, which is larger than 10⁻⁹, 10⁻⁶, 10⁻³, 10⁻¹, 1, 2, 5, or 10 %./sec. In some cases, (ΔD/δt)t_{1b} can be defined as the slope of the tangent of the variation with time of the percentage of dissociation occurring during the dissociation step.

In one embodiment of the invention, (ΔD/δt)t_{1b} decreases, preferentially by a factor of more than 1.001, 1.01, 1.1, 1.5, 2, 5, 10, or 100, when t_{1b} increases, preferentially by a factor of more than 1.001, 1.01, 1.1, 1.5, 2, 5, 10, or 100. This can mean that (ΔD/δt)t_{1b} is larger at the beginning than at the end of the dissociation step. This can mean that the initial slope of the variation of the percentage of dissociation with time, measured at the beginning of the dissociation step, is the largest value of (ΔD/δt)t_{1b}. In some cases, the laser power, laser power density, laser intensity, and/or concentration of the magnetic nanoparticles, in particular magnetosomes, can be increased, preferentially by a factor of more than 1.001, 1.01, 1.1, 1.2, 1.5, 2, 5, 10, or 100, to decrease the variation between the different values of (ΔD/δt_{1b}, measured at different times of the dissociation step. In some cases, (ΔD/δt)t_{1b} varies by less than 99, 90, 85, 75, 60, 50, 30, 20, 10, 5, 2, 1, or 10⁻¹ %. This percentage can be equal to [(ΔD/δt)t_{1b1}-(ΔD/δt)t_{1d2}]/(ΔD/δt)t_{1b1}, where (ΔD/δt)t_{1b1} and (ΔD/δt)t_{1b2} are the gradients in the percentages of dissociation measured at two different times t_{1b1} and t_{1b2} of the dissociation step.

In still another embodiment of the invention, the average gradient of the percentage of dissociation, measured during the dissociation step, is the average value of (ΔD/δt)t_{1b} measured at different times t_{1b}, where the different values of t_{1b} used to measure this average value are preferentially separated by similar durations, or by durations that are longer than 10⁻⁹, 10⁻⁶, 10⁻³, 10⁻¹, 1, 5, 10, 50, or 100 second(s). The average value of (ΔD/δt)t_{1b} is designated as Av[(ΔD/δt)t_{1b}]. In some cases, Av[(ΔD/δt)t_{b}] is larger than 10⁻⁹, 10⁻⁶, 10⁻³, 10⁻¹, 1, 2, 5, or 10 %/sec. In some other cases, Av[(ΔD/δt)t_{1b}] is lower than 10⁶, 10³, 10, 5, 2, 1, or 10⁻³ %/sec.

The invention also relates to magnetic nanoparticles, in particular magnetosomes, for use, wherein the non-dissociation step produces a percentage of dissociation of the compounds from the magnetic nanoparticles, in particular magnetosomes, which is lower than 99%.

In one embodiment of the invention, the non-dissociation step produces a percentage of dissociation of the compounds from the magnetic nanoparticles, in particular magnetosomes, which is lower than 99.9, 99, 90, 80, 50, 40, 20, 10, 5, 2, or 1 %.

In one embodiment of the invention, the non-dissociation step produces a gradient of the percentage of dissociation, preferentially of/in the body part comprising the magnetic nanoparticles, in particular magnetosomes, measured at any given time of the non-dissociation step, (ΔD/δt)t_{2b}, which is lower than 10⁻⁹, 10⁻⁶, 10⁻³, 10⁻¹, 1, 2, 5, or 10 %/sec. In some cases, (ΔD/δt)t_{2b} can be defined as the slope of the tangent of the variation with time of the percentage of dissociation occurring during the non-dissociation step.

In still another embodiment of the invention, the average gradient of the percentage of dissociation, measured during the non-dissociation step, is the average value of (δD/δt)t_{2b} measured at different times t_{2b}, where the different values of t_{2b} used to measure this average value are preferentially separated by similar durations, or by durations that are longer than 10⁻⁹, 10⁻⁶, 10⁻³, 10⁻¹, 1, 5, 10, 50, or 100 second(s). The average value of (ΔD/δt)t_{2b} is designated as Av((ΔD/δt)t_{2b}]. In some cases, Av[(ΔD/δt)t_{2b}] can be lower than 10⁻⁹, 10⁻⁶, 10⁻³, 10⁻¹, 1, 2, 5, or 10 %/sec. In some other cases, Av[(ΔD/δt)t_{2b}] can be lower than 10⁶, 10³, 10, 5, 2, 1, or 10⁻³ %/sec.

In one embodiment of the invention, the percentage of dissociation reached during the dissociation step, P_{DS}, and the percentage of dissociation reached during the non-dissociation step, P_{NDS}, differ by more than 10⁻⁶, 10⁻³, 10⁻², 10⁻¹, or 1 %, where this percentage can be equal to the absolute values of (P_{DS} - P_{NDS})/ P_{DS} or (P_{NDS} - P_{DS}) / P_{NDS}.

The invention also relates to magnetic nanoparticles, in particular magnetosomes, for use, wherein the first and/or second step(s) is/are ended when a given temperature and/or percentage of dissociation is/are reached.

In one embodiment of the invention, the first and/or second step(s) is/are ended when a temperature larger than -273, -270, -150, -100, -50, -20, 0, 5, 10, 15, 20, 37, 40, 41, 43, 45, 50, 55, 60, 70, 80, 90, 100, 200, 500, or 1000 °C, is reached.

In another embodiment of the invention, the first and/or second step(s) is/are ended when a temperature lower than -273, -270, -150, -100, -50, -20, 0, 5, 10, 15, 20, 37, 40, 41, 43, 45, 50, 55, 60, 70, 80, 90, 100, 200, 500, or 1000 °C, is reached.

In one embodiment of the invention, the first and/or second step(s) is/are ended when a percentage of dissociation larger than 10⁻²⁰, 10⁻¹⁰, 10⁻⁵, 10⁻³, 10⁻¹, 1, 5, 10, 10³, 10⁵, 10¹⁰, or 10²⁰ %, is reached.

In one embodiment of the invention, the first and/or second step(s) is/are ended when a percentage of dissociation lower than 10⁻²⁰, 10⁻¹⁰, 10⁻⁵, 10⁻³, 10⁻¹, 1, 5, 10, 10³, 10⁵, 10¹⁰, or 10²⁰ %, is reached.

In one embodiment of the invention, the first and/or second step(s) is/are ended: i), by switching off or stop the laser or laser power or laser intensity or laser equipment or laser apparatus, or, ii), by decreasing the laser power or laser intensity by a factor, which is preferentially larger than 1.001, 1.01, 1.1, 1.5, 2, 5, 10, 50, 10², 10³, 10⁶, or 10⁹, preferentially between the first and second step.

The invention also relates to magnetic nanoparticles, in particular magnetosomes, for use, wherein the concentration of the magnetic nanoparticles, in particular magnetosomes, and/or the duration(s) of the first and/or second step(s) and/or the laser power or laser power density or laser intensity is(are) adjusted to reach the desired variation(s) or gradient(s) of temperature and/or percentage of dissociation occurring during the first and/or second step(s).

In one embodiment of the invention, the concentration of the magnetic nanoparticle, in particular magnetosome, preferentially in the body part, is adjusted to a value above 10⁻⁹, 10⁻⁶, 10⁻³, 10⁻², 10⁻¹, 1, 10, 100, or 10³ mg of magnetic nanoparticles, in particular magnetosomes, per cm³ or mg of magnetic nanoparticle, in particular magnetosome, per cm³ of body part. A sufficiently large concentration of magnetic nanoparticle, in particular magnetosome, can enable to heat locally the body part, preferentially at the nanometer scale.

In the experimental example, for M-CMD, the increase in concentration from 0.5 to 1 mg per mL produces an increase of the average temperature gradient during the heating step by a factor comprised between 1.6 and 2.2. In some case, it is possible to reduce the concentration of the magnetic nanoparticles, in particular magnetosomes, preferentially by a factor of more than 1.001, 1.01, 1.1, 2, 5, 10, or 10³, and reach an average temperature gradient during the heating step, which is decreased, preferentially by a factor a factor of more than 1.001, 1.01, 1.1, 2, 5, 10, or 10³. In some other cases, it is possible to increase the concentration of magnetic nanoparticles, in particular magnetosomes, preferentially by a factor of more than 1.001, 1.01, 1.1, 2, 5, 10, or 10³, and reach an average temperature gradient during the heating step, which is increased, preferentially by a factor a more than 1.001, 1.01, 1.1, 2, 5, 10, or 10³.

In the experimental example, for M-CMD, the increase in concentration from 0.5 to 1 mg per mL produces a decrease of the average temperature gradient during the cooling step by a factor comprised between 1.1 and 1.3. In some case, it is possible to reduce the magnetic nanoparticle, in particular magnetosome, concentration, preferentially by more than a factor of 1.001, 1.01, 1.1, 2, 5, 10, or 10³, and reach an average temperature gradient during the cooling step, which is increased, preferentially by a factor a factor of 1.001, 1.01, 1.1, 2, 5, 10, or 10³. In some other cases, it is possible to increase the magnetic nanoparticle, in particular magnetosome, concentration, preferentially by more than a factor of 1.001, 1.01, 1.1, 2, 5, 10, or 10³, and reach an average temperature gradient during the cooling step, which is decreased, preferentially by a factor a factor of 1.001, 1.01, 1.1, 2, 5, 10, or 10³. In one embodiment of the invention, a sufficiently large magnetic nanoparticle, in particular magnetosome, concentration can be looked for to reach a sufficiently large magnetic nanoparticle, in particular magnetosome, absorption rate, SAR_{M}.

In some cases, SAR_{M} can be larger than 10⁻²⁰, 10⁻¹⁰, 10⁻⁵, 10⁻², 10⁻¹, 1, 5, 10, 10², or 10³ Watt per gram of magnetic nanoparticle, in particular magnetosome, or Watt per gram of iron oxide comprised in the magnetic nanoparticle, in particular magnetosome, or Watt per gram of iron comprised in the magnetic nanoparticle, in particular magnetosome.

In still another embodiment of the invention, SAR_{M} does not vary or decrease or increase by more than 10⁵, 500, 90, 70, 50, 25, 10, 5, or 2 % between different concentrations of magnetic nanoparticles, in particular magnetosomes, where this percentage can represent SAR_{MC2}-SAR_{MC1}/SAR_{MC1}, where SAR_{MC1} and SAR_{MC2} are two different concentrations of magnetic nanoparticles, in particular magnetosomes. In some cases, this situation can occur when the concentration of magnetic nanoparticle, in particular magnetosome, is between 10⁻⁶ and 10⁶, 10⁻⁵ and 10⁵, 10⁻³ and 10³, or between 10⁻² and 10² mg per mL or mg per cm³ of body part. In some other cases, this situation can occur when the concentration of magnetic nanoparticle, in particular magnetosome, is lower than 1000, 100, 10, 1, 0.1, or 0.01 mg per mL or mg per cm³ of body part. In still some other cases, this situation can occur when the concentration of magnetic nanoparticles, in particular magnetosomes, is larger than 1000, 100, 10, 1, 0.1, or 0.01 mg per mL or mg per cm³ of body part.

In one embodiment of the invention, SAR_{M} is the specific absorption rate of the magnetic nanoparticles, in particular magnetosomes, comprised, mixed or inserted in the body part. It can be expressed in a power unit such as Watt divided by a mass unit such as gram or in a power unit divided by a length, surface area, or volume unit such as cm, cm², or cm³. SAR_{M} is preferentially measured under the application of a radiation that produces a temperature increase, preferentially in the presence of the magnetic nanoparticle, in particular magnetosome,s. In some cases, this radiation can be a laser, preferentially of power or power density larger than 10⁻⁹, 10⁻⁵, 10⁻³, 10⁻¹, 1, 10, or 10³ W or W/cm or W/cm² or W/cm³. In some other cases, this radiation can be an acoustic wave, preferentially of power or power density larger than 10⁻⁹, 10⁻⁵, 10⁻³, 10⁻¹, 1, 10, or 10³ W/cm or W/cm² or W/cm³, preferentially of frequency larger than 10⁻⁶, 10⁻³, 10⁻¹, 1, 10, 10³, or 10⁶ MHz. In still some other cases, this radiation can be an alternating magnetic field, preferentially of frequency larger than 10⁻⁹, 10⁻⁶, 10⁻³, 1, 10³, 10⁶, or 10⁹ kHz, preferentially of strength larger than 10⁻⁹, 10⁻⁶, 10⁻³, 10⁻¹, 1, 10, 10³, or 10⁶ mT. SAR_{M} is preferentially measured in adiabatic conditions or in conditions in which heat exchanges are minimized, preferentially between: i), the portion of the body part comprising the magnetic nanoparticles, in particular magnetosomes, and the portion of the body part not comprising the magnetic nanoparticles, in particular the magnetosomes, or the region outside the body part comprising the magnetic nanoparticles, in particular the magnetosomes, or ii), the container or tube containing the magnetic nanoparticles, in particular the magnetosomes, and the exterior of this container or tube. Heat exchanges are minimized when they produce a temperature decrease of less than 75, 60, 50, 25, 10, 5, 2, 1, or 0.1 °C. In some cases, SAR_{M} can be proportional to the difference between the initial slope of the temperature variation with time of the body part, medium, water, tissue comprising the magnetic nanoparticles, in particular magnetosomes, (ΔT/δt)_{(M)}, minus the initial slope of the temperature variation with time of body part, medium, water, tissue, not comprising the magnetic nanoparticles, in particular magnetosomes, (ΔT/δt)_{(WM)}, where (ΔT/δt)_{(M)} and (ΔT/δt)_{(WM)} are preferentially estimated in °C/sec. SAR_{M} is preferentially estimated using to the formula: SAR_{M}=αᵣₑₐₗ[(ΔT/δt)_{(M)}-(ΔT/δt)_{(WM)}], where αᵣₑₐₗ is a proportionality coefficient. In some cases, SAR_{M} = [(ΔT/δt)_{(M)}-(ΔT/δt)_{(WM)}].Cᵥ/C_{mag}, where Cᵥ is the specific heat capacity, preferentially of the body part, tissue, water, medium comprising the magnetic nanoparticle, in particular magnetosomes, and C_{mag} is the concentration of magnetic nanoparticles, in particular magnetosomes, or quantity or number of magnetic nanoparticles, in particular magnetosomes, preferentially comprised in the body part.

In another embodiment of the invention, SAR_{M} is the specific absorption rate of the magnetic nanoparticles, in particular magnetosomes, estimated without subtracting (ΔT/δt)_{(WM)} to (ΔT/δt)_{(M)}. This can be the case when (ΔT/δt)_{(WM)} is small, preferentially smaller than 10⁻⁹, 10⁻⁶, 10⁻³, 10⁻¹, 1, 10, or 20 °C/sec. In some cases, SAR_{M} can be proportional to the initial slope of the temperature variation with time of the magnetic nanoparticles, in particular magnetosomes, comprised in the body part, (ΔT/δt)_{(M)}, preferentially estimated in °C/sec, preferentially leading to the formula: SAR_{M}=α_{M}.(ΔT/δt)_{(M)}, where α_{M} is a proportionality coefficient. In some other cases, SAR_{M}=(ΔT/δt)_{(M)}.Cᵥ/C_{mag}, where Cᵥ is the specific heat capacity, preferentially of the body part, tissue, water, medium comprising the magnetic nanoparticles, in particular magnetosomes, and C_{mag} is the concentration of the magnetic nanoparticles, in particular magnetosomes, or quantity or number of magnetic nanoparticles, in particular magnetosomes, preferentially comprised in the body part.

In still another embodiment of the invention, the SAR measured by applying the laser on the magnetic nanoparticles, in particular magnetosomes, or body part, designated as SARₗₐₛₑᵣ, is different from the SAR measured by applying an alternating magnetic field on the magnetic nanoparticles, in particular magnetosomes, or body part, designated as SAR_{AMF}. In some cases SARₗₐₛₑᵣ differs from SAR_{AMF} by at least 10⁻⁹, 10⁻⁷, 10⁻⁵, 10⁻³, 10⁻¹, 1, 10, 10³, 10⁵, 10⁷, or 10⁹ %, where this percentage can be equal to (SARₗₐₛₑᵣ-SAR_{AMF})/SARₗₐₛₑᵣ, where this percentage is preferentially measured at a given concentration of magnetic nanoparticles, in particular magnetosomes. In some cases, the concentration of magnetic nanoparticles, in particular magnetosomes, is comprised between 10⁻⁹ and 10⁹, 10⁻⁷ and 10⁷, 10⁻⁵ and 10⁵, 10⁻³ and 10³, or between 10⁻¹ and 10 mg of magnetic nanoparticles, in particular magnetosomes, per mL or between 10⁻¹ and 10 mg of magnetic nanoparticles, in particular magnetosomes, per cm³ of body part. In some other cases, the concentration of the magnetic nanoparticles, in particular magnetosomes, is lower than 10⁹, 10⁷, 10⁵, 10³, 10, 1, 10⁻³, 10⁻⁵, 10⁻⁷, or 10⁻⁹ mg of magnetic nanoparticles, in particular magnetosomes, per mL or mg of magnetic nanoparticle, in particular magnetosomes, per cm³ of body part. In still some other cases, the concentration of magnetic nanoparticles, in particular magnetosomes, is larger than 10⁹, 10⁷, 10⁵, 10³, 10, 1, 10⁻³, 10⁻⁵, 10⁻⁷, or 10⁻⁹ mg of magnetic nanoparticles, in particular magnetosomes, per mL or 10⁻⁹ mg of magnetic nanoparticles, in particular magnetosomes, per cm³ of body part.

In another embodiment of the invention, the concentration of magnetic nanoparticles, in particular magnetosomes, preferentially in the body part, is adjusted to a value below 10⁹, 10⁶, 10³, 10², 1, 10⁻¹, or 10⁻³ mg of magnetic nanoparticles, in particular magnetosomes, per cm³ or mg of magnetic nanoparticles, in particular magnetosomes, per cm³ of body part. A sufficiently low concentration of magnetic nanoparticles, in particular magnetosomes, can be sought after or looked for to avoid toxicity of magnetic nanoparticles, in particular magnetosomes.

In another embodiment of the invention, a too low concentration of magnetic nanoparticles, in particular magnetosomes, may prevent sufficient local heating and possibly result in macroscopic heating, *i.e.* heating at a scale that is preferentially larger than 1, 10, 10³, 10⁶, or 10⁹ nm. In this case, a too low concentration of magnetic nanoparticles, in particular magnetosomes, may be avoided.

In one embodiment of the invention, local heating or local temperature increase is defined as heating at a local scale or heating that can be measured at a local scale or locally, where a local scale can designate a scale that is below 10⁹, 10⁷, 10⁵, 10³, 10, 1, 10⁻¹, or 10⁻³ nm, or a scale that is close to the nanometer scale or to the size of a single magnetic nanoparticle, in particular magnetosome, or to the size of an assembly of magnetic nanoparticles, in particular magnetosomes.

In one embodiment of the invention, macroscopic heating or macroscopic temperature increase is defined as heating at a macroscopic scale or heating that can be measured at a macroscopic scale or macroscopically, where a macroscopic scale can designate a scale that is above 10⁹, 10⁷, 10⁵, 10³, 10, 1, 10⁻¹, or 10⁻³ nm, or a scale that is above the nanometer scale or above the size of a single magnetic nanoparticle, in particular magnetosome, or of the size of an assembly of magnetic nanoparticles, in particular magnetosomes.

In one embodiment of the invention, an increase in concentration of magnetic nanoparticles, in particular magnetosomes, preferentially by a factor of more than 1.001, 1.01, 1.1, 1.2, 1.5, 2, 5, 10, 10³, or 10⁶, or preferentially above 10⁻⁶, 10⁻³, 10⁻¹, 1, 5, or 10 mg of magnetic nanoparticle(s), in particular magnetosome(s), per cm³ of body part, can increase the temperature gradient, average temperature gradient, or temperature variation, occurring during the first and/or second step(s), preferentially by a factor of more than 1.001, 1.01, 1.1, 1.2, 1.5, 2, 5, 10, 10³, or 10⁶, or preferentially by more than 10⁻⁶, 10⁻³, 10⁻¹, 1, 5, 10, or 10³ °C.

In another embodiment of the invention, an increase in concentration of magnetic nanoparticles, in particular magnetosomes, by a factor of more than 1.001, 1.01, 1.1, 1.2, 1.5, 2, 5, 10, 10³, or 10⁶, or preferentially above 10⁻⁶, 10⁻³, 10⁻¹, 1, 5, or 10 mg of magnetic nanoparticle(s), in particular magnetosome,(s), per cm³ of body part, can increase the percentage of dissociation occurring in the first step, preferentially by a factor of more than 1.001, 1.01, 1.1, 1.2, 1.5, 2, 5, 10, 10³, or 10⁶, or preferentially above 10⁻⁶, 10⁻³, 10⁻¹, 1, 5, 10, 25, 50, 75, 80, or 90%.

In another embodiment of the invention, a decrease in t₁ₐ and/or t₂ₐ, preferentially by a factor of more than 1.001, 1.01, 1.1, 1.2, 1.5, 2, 5, 10, 10³, or 10⁶, or preferentially below 10²⁰, 10¹⁰, 10⁵, 10³, 10², 50, 10, or 1 second(s), can increase the average temperature gradient, the temperature gradient, or the temperature variation, occurring during the first and/or second step(s), preferentially by a factor of more than 1.001, 1.01, 1.1, 1.2, 1.5, 2, 5, 10, 10³, or 10⁶, or preferentially by more than 10⁻⁶, 10⁻³, 10⁻¹, 1, 5, 10, or 10³ °C.

In an embodiment of the invention, a decrease in t_{1b} and/or t_{2b} by a factor of more than 1.001, 1.01, 1.1, 1.2, 1.5, 2, 5, 10, 10³, or 10⁶, or preferentially below 10²⁰, 10¹⁰, 10⁵, 10³, 10², 50, 10, or 1 second(s), can increase the percentage of dissociation, the average percentage of dissociation, the gradient of the percentage of dissociation, the average gradient of the percentage of dissociation, occurring during the first and/or second steps(s), preferentially by a factor of more than 1.001, 1.01, 1.1, 1.2, 1.5, 2, 5, 10, 10³, or 10⁶, or preferentially above 10⁻⁶, 10⁻³, 10⁻¹, 1, 5, 10, 25, 50, 75, 80, or 90%.

In some cases, the first step can be carried out to reach a maximum temperature, *i.e.* a temperature that is preferentially above the temperature reached before or without irradiation by the laser radiation, preferentially by more than 10⁻³, 1, 5, 10, or 50 °C. In some other cases, the first step can be carried out to reach a maximum percentage of dissociation, *i.e.* a percentage of dissociation that is preferentially above, preferentially by more than 10⁻³, 10⁻¹, 1, 10, or 25 %, the percentage of dissociation reached before or without the irradiation by the laser radiation.

In some cases, the maximum temperature that one desires to reach in the first step is larger than -273, - 150, -100, -50, -20, -10, -5, -3, -1, 5, 10, 50, 10², or 10³ °C.

In some other cases, the maximum percentage of dissociation that one desires to reach in the first step is larger than 90, 80, 70, 50, 25, 10, 5, 2, or 1 %.

In some cases, the maximum temperature that one desires to reach in the first step is lower than -273,-150, -100, -50, -20, -10, -5, -3, -1, 5, 10, 50, 10², or 10³ °C.

In some other cases, the maximum percentage of dissociation that one desires to reach in the first step is lower than 90, 80, 70, 50, 25, 10, 5, 2, or 1 %.

In some cases, the second step can be carried out to reach a minimum temperature, *i.e.* a temperature that is preferentially below, preferentially by more than 10⁻³, 1, 5, 10, or 50 °C, the maximum temperature reached in the first step. In some other cases, the second step is carried out to reach a minimum percentage of dissociation, *i.e.* a percentage of dissociation that is preferentially below, preferentially by more than 10⁻³, 10⁻¹, 1, 10, or 25 %, the percentage of dissociation reached in the first step.

In some cases, the minimum temperature that one desires to reach in the second step is at lease 1, 5, 10, 50, 100, 10³, or 10⁵ °C lower than the maximum temperature that one desires to reach in the first step.

In some other cases, the minimum percentage of dissociation that one desires to reach in the second step is at least 1, 5, 10, 50, 75, 80, or 90 % lower than the maximum percentage of dissociation that one desires to reach in the first step.

In some cases, the minimum temperature that one desires to reach in the second step is lower than 273, -150, -100, -50, -20, -10, -5, -3, -1, 5, 10, 50, 10², or 10³ °C.

In some other cases, the minimum percentage of dissociation that one desires to reach in the second step is lower than 1, 5, 10, 50, 75, 80, or 90 %.

In still some other cases, the maximum or minimum temperature or percentage of dissociation that one desires to reach during the first or second step can be the same as the maximum or minimum temperature or percentage of dissociation that is reached during the first or second step.

In still some other cases, the maximum or minimum temperature or percentage of dissociation that one desires to reach during the first or second step can be different from the maximum or minimum temperature or percentage of dissociation that is reached during the first or second step, preferentially by a factor of more than 1.001, 1.01, 1.1, 1.5, 2, 5, 10, or 10³.

In still another embodiment of the invention, the absolute value of the variation or gradient of temperature occurring in the first step does not differ by a factor of more than 1.1, 1.2, 1.5, 2, 5, 10, or 10³, from the absolute value of the variation or gradient of temperature occurring in the second step.

In still another embodiment of the invention, the percentage of dissociation occurring in the first step differs by a factor of more than 1.1, 1.2, 1.5, 2, 5, 10, or 10³, from the percentage of dissociation occurring in the second step.

The invention also relates to magnetic nanoparticles, in particular magnetosomes, for use, wherein the duration(s) of the first and/or second step(s) is/are between 10⁻⁹ seconds and 10³ hours.

In one embodiment of the invention, the duration of the first and/or second step(s) is between 10⁻²⁰ seconds and 10²⁰ hours, or between 10⁻⁹ seconds and 10¹⁰ hours, or between 10⁻³ seconds and 1 hour, or between 10⁻³ seconds and 10³ seconds, or between 10⁻² seconds and 10³ seconds, or between 10⁻¹ seconds and 10 seconds, or between 1 second and 10³ seconds, or between 1 second and 10² seconds, or between 1 second and 50 seconds.

The invention also relates to magnetic nanoparticles, in particular magnetosomes, for use, wherein the ratio between the duration of the first step and the duration of the second step is between 10⁻²⁰ and 10²⁰.

In one embodiment of the invention, the ratio between the duration of the first step and the duration of the second step is between 10⁻²⁰ and 10²⁰, 10⁻¹⁰ and 10¹⁰, 10⁻⁵ and 10⁵, 10⁻³ and 10³, 10⁻¹ and 10, 0.5 and 5, or between 0.5 and 2.

The invention also relates to magnetic nanoparticles, in particular magnetosomes, for use, wherein the application of sequences enables to decrease diffusion of magnetic nanoparticles, in particular magnetosomes, outside of the portion of the body part comprising the magnetic nanoparticles, in particular the magnetic nanoparticles, in particular magnetosomes.

In one embodiment of the invention, the portion of the body part comprising the magnetic nanoparticles, in particular the magnetosomes, is the portion of the body part that has received the magnetic nanoparticle, in particular the magnetosomes, more than 10⁻⁹, 10⁻⁶, 10⁻³, 1, 10³, 10⁶, or 10⁹ second(s) following administration in the body part of magnetic nanoparticles, in particular magnetosomes.

In another embodiment of the invention, the portion of the body part comprising the magnetic nanoparticles, in particular the magnetosomes, is the portion of the body part that has received the magnetic nanoparticle, in particular the magnetosomes, less than 10⁻⁹, 10⁻⁶, 10⁻³, 1, 10³, 10⁶, or 10⁹ second(s) following administration in the body part of the magnetic nanoparticles, in particular magnetosomes.

In another embodiment of the invention, the portion of the body part comprises pathological cells, or a majority of pathological cells, or more than 10⁻²⁰, 10⁻¹⁰, 10⁻⁵, 10⁻³, 10⁻¹, 1, 5, 10, 25, 50, 75, or 90 % of pathological cells, where this percentage represents the ratio between the number of pathological cells comprised in the body part and the total number of cells or the sum of the numbers of healthy and pathological cells comprised in the body part.

In one embodiment of the invention, the body part is or comprises or includes the portion of the body part. In some cases, the portion of the body part is the portion of the body part comprising the magnetic nanoparticle, in particular magnetosome,s. In some other cases, the portion of the part is the portion of the body part not comprising the magnetic nanoparticle, in particular magnetosome,s. In still some other cases, the portion of the body parts is the portion of the body part comprising pathological cells. In still some other cases, the portion of the body part is the portion of the body part not comprising the pathological cells. In still some other cases, the portion of the body part is the portion of the body part not comprising the healthy cells. In still some other cases, the portion of the body part is the portion of the body part not comprising the healthy cells.

In one embodiment of the invention, the application of sequences enables to reach a percentage of magnetic nanoparticles, in particular magnetosomes, diffusing outside of the portion of the body part comprising the magnetic nanoparticles, in particular magnetosomes, which is lower than 95, 90, 80, 70, 50, 30, 20, 10, 5, 2, or 1%. Considering a portion of the body part with numbers of magnetic nanoparticles, in particular magnetosomes, N₁ at a time t₁ and N₂ at a time t₂, preferentially following administration in the body part of magnetic nanoparticles, in particular magnetosomes, this percentage can be equal to (N₂-N₁)/N₁.

In one embodiment of the invention, the application of sequences enables to heat the magnetic nanoparticles, in particular magnetosomes, or to dissociate the compound from the magnetic nanoparticle, in particular magnetosome, during an overall time, which preferentially includes the duration of all sessions, which is 2, 5, 10, or 100 times longer than without the application of sequences.

In another embodiment of the invention, the application of sequences enables to heat the magnetic nanoparticles, in particular magnetosomes, or to dissociate the compound from the magnetic nanoparticles, in particular magnetosomes, during more than 1 minute, 1 hour, 1 day, 1 week, 1 month, or 1 year, following administration, preferentially in the body part, of the magnetic nanoparticles, in particular magnetosomes.

The invention also relates to magnetic nanoparticles, in particular magnetosomes, for use, wherein the application of sequences enables to increase the percentage of dissociation of the compound from the magnetic nanoparticle, in particular magnetosome, and/or to increase the number of temperature gradients or variations and/or to decrease the average temperature reached during treatment.

In one embodiment of the invention, the application of sequences enables to decrease the average temperature reached during treatment, preferentially by a factor of more than 1.001, 1.01, 1.1, 1.2, 1.5, 2, 5, 10, 10³, or 10⁶, preferentially below 10⁶, 10³, 100, 90, 70, 50, 49, 48, 47, 46, 45, 44, 43, 42, 41, 40, 39, 38, 37, 35, 20, 10, 5, 2 °C.

In still another embodiment of the invention, the application of sequences enables to reach an average temperature reached during treatment, which is lower than 10⁶, 10³, 100, 90, 70, 50, 49, 48, 47, 46, 45, 44, 43, 42, 41, 40, 39, 38, 37, 35, 20, 10, 5, 2 °C.

In the experimental example of the invention, the average temperature reached during treatment can be equal to (45+37)/2 = 41 °C for 0.5 and 1 mg/mL of M-CMD, and to (49+37)/2 = 43 °C for 1 mg/mL of N-CMD. In some cases, it is possible to decrease the average temperature reached during treatment, preferentially by a factor of more than 1.001, 1.01, 1.1, 1.5, 2, 5, 10, or 10³, by decreasing the temperature increase and/or temperature decrease that one wants to achieve during the heating and/or cooling step(s), respectively, preferentially by a factor of more than 1.001, 1.01, 1.1, 1.5, 2, 5, 10, or 10³. In some other cases, it is possible to increase the average temperature reached during treatment, preferentially by a factor of more than 1.001, 1.01, 1.1, 1.5, 2, 5, 10, or 10³, by increasing the temperature increase and/or temperature decrease that one wants to achieve during the heating and/or cooling step(s), respectively, preferentially by a factor of more than 1.001, 1.01, 1.1, 1.5, 2, 5, 10, or 10³.

In one embodiment of the invention, the average temperature reached during treatment is an average value of the maximum and minimum temperatures reached in the first step, or is the average value of the maximum and minimum temperature reached in the second step. In some cases, the average temperature reached during treatment can be the average value of the two average values estimated for the different heating and cooling steps. In some other cases, the average temperature reached during treatment can be the average of the average values estimated for each of the various sequences or sessions.

In still another embodiment of the invention, the average temperature reached during treatment is the temperature that is reached during the longest period of time of the treatment. In some cases, the saturating temperature or the temperature that reaches a plateau during the heating or cooling step, *i.e.* the temperature that varies by less than 10⁶, 10³, 10, 5, 2, 1, 10⁻³, 10⁻⁶, 10⁻⁹, or 10⁻²⁰ °C/sec could be considered as the average temperature reached during treatment.

In some other cases, the average temperature reached during treatment can be deduced from the average temperature variations of the heating and cooling steps, being preferentially the average value of the temperature variations of the heating and cooling steps divided by a certain factor, preferentially a factor of 2.

In some other cases, the average temperature reached during treatment can be deduced from the average temperature gradients of the heating and cooling steps.

In one embodiment of the invention, the application of sequences enables to increase, preferentially by a factor of more than 1.1, 1.2, 1.5, 2, 5, 10, 10³, or 10⁵, the percentage of dissociation and/or the number of temperature gradients or variations, compared with a continuous application of the laser, *i.e.* an irradiation by the laser radiation without sequences or sessions or with less than 10³, 10², 10, 5, or 2 sequences or sessions. A continuous application can be defined as the continuous irradiation by the laser radiation during a sufficiently long time, preferentially during more than 10⁻³, 1, 10³, 10⁶, or 10⁹ second(s), without switching off the laser or without reducing the laser power or laser power density or laser intensity by a factor of more than 1.1, 1.2, 1.5, 2, 5, 10, 10³, or 10⁶, or below 10²⁰, 10¹⁰, 10⁵, 10³, 10, 1, 10⁻³, or 10⁻⁵ W or W/cm or W/cm² or W/cm³, or below 10²⁰, 10¹⁰, 10⁵, 10³, 10, 1, 10⁻³, or 10⁻⁵ mA

The invention also relates to magnetic nanoparticles, in particular magnetosomes, for use, in which the sequences are carried out in such a way that: i), the maximum temperature or maximum percentage of dissociation that one desires to reach in the first step and the minimum temperature or minimum percentage of dissociation that one desires to reach in the second step are determined, ii), a parameter of the laser is set or fixed at a first value to reach the maximum temperature and/or maximum percentage of dissociation in the first step and then a parameter of the laser is set or fixed at a second value to reach the minimum temperature and/or minimum percentage of dissociation in the second step, optionally, iii), the duration(s) of the first and/or second step(s) required to reach the maximum or minimum temperature and/or the maximum or minimum percentage of dissociation are measured, and optionally, iv), the first and/or second step(s) is(are) repeated during the measured duration(s) of the first and/or second step(s).

In one embodiment of the invention, the maximum temperature and/or maximum percentage of dissociation that one wants to reach in the first step is(are) set or fixed at one or several given value(s), designated as gₘₐₓ.

In some cases, the maximum temperature and/or maximum percentage of dissociation that one wants to reach in the first step is(are) different from the maximum temperature and/or maximum percentage of dissociation reached in the first step by a factor of more than 1.1, 1.5, 10, 10³, 10⁶, 10⁹, or 10²⁰.

In some other cases, the maximum temperature and/or maximum percentage of dissociation that one wants to reach in the first step is/are different from the maximum temperature and/or maximum percentage of dissociation reached in the first step by a factor of less than 1.1, 1.5, 10, 10³, 10⁶, 10⁹, or 10²⁰.

In one embodiment of the invention, the minimum temperature and/or minimum percentage of dissociation that one wants to reach in the second step is(are) set at one or several given value(s), designated as gₘᵢₙ.

In some cases, the minimum temperature and/or minimum percentage of dissociation that one wants to reach in the second step is(are) different from the minimum temperature and/or minimum percentage of dissociation reached in the first step by a factor of more than 1.1, 1.5, 10, 10³, 10⁶, 10⁹, or 10²⁰.

In some other cases, the minimum temperature and/or minimum percentage of dissociation that one wants to reach in the second step is(are) different from the minimum temperature and/or minimum percentage of dissociation reached in the first step by a factor of less than 1.1, 1.5, 10, 10³, 10⁶, 10⁹, or 10²⁰.

In one embodiment of the invention, gₘₐₓ, gₘᵢₙ, the duration of the first or second step, the number of sequences or sessions, or the laser parameter, is chosen to reach the maximum laser treatment efficacy or minimum laser treatment toxicity.

In some cases, maximum laser treatment efficacy and/or minimum laser treatment toxicity can be reached when the treatment involves direct effects, where direct effects can be defined as effects that cause the destruction or disappearance of the disease or pathological cells without involving substances such as immune cells, immune-attractants, T cells, B Cells, cytokines, which are preferentially different from the magnetic nanoparticle, in particular magnetosome, or the compound. For example, a direct effect can be the destruction of a pathological cell by the heat produced by the magnetic nanoparticle, in particular magnetosome, or by the toxicity of the compound towards pathological cells.

In some other cases, maximum laser treatment efficacy and/or minimum laser treatment toxicity can be reached when the treatment involves indirect effects, where indirect effects can be defined as effects that cause the destruction or disappearance of the disease or pathological cells by involving substances such as immune cells, immune-attractants, T cells, B Cells, cytokines, which are different from the magnetic nanoparticle, in particular magnetosome, or the compound. For example, an indirect effect can be the destruction of a pathological cell by an immune cell, or the destruction of a pathological cell at a distance from the magnetic nanoparticles, in particular magnetosomes, or body part comprising the magnetic nanoparticles, in particular magnetosomes, which is preferentially larger than 1, 5, 10, 100, 10³, 10⁵, 10⁷, or 10⁹ nm. An indirect effect is preferentially an effect that is not a direct effect.

In one embodiment of the invention, the parameter of the laser used to reach the maximum temperature and/or maximum percentage of dissociation in the first step is different, preferentially by a factor of more than 1.1, 1.5, 2, 5, 10, 10³, 10⁶, or 10¹⁰, from the parameter of the laser used to reach the minimum temperature and/or minimum percentage of dissociation in the second step.

In another embodiment of the invention, the duration(s) of the first and/or second step(s) required to reach maximum or minimum temperature and/or maximum or minimum percentage of dissociation are measured. In some cases, the duration of the first step is measured by preferentially starting from the minimum temperature or minimum percentage of dissociation, preferentially reached at the beginning of the first step, by irradiating magnetic nanoparticle, in particular magnetosome, or body part, with laser radiation and by measuring the time that it takes for magnetic nanoparticles, in particular magnetosomes, or body part to reach the maximum temperature or maximum percentage of dissociation, preferentially reached at the end of the first step. In some other cases, the duration of the second step is measured by preferentially starting from the maximum temperature or maximum percentage of dissociation, preferentially reached at the beginning of the second step, by not irradiating by laser radiation or by irradiating with laser radiation of lower power than in the first step, and by measuring the time it takes to reach the minimum temperature or minimum percentage of dissociation, preferentially reached at the end of the second step. In some cases, the duration(s) of the first and/or second step(s) is(are) measured for magnetic nanoparticles, in particular magnetosomes, comprised in the body part. In some other cases, the durations of the first and/or second step(s) is(are) measured for magnetic nanoparticles, in particular magnetosomes, comprised outside of the body part, for example for magnetic nanoparticles, in particular magnetosomes, mixed in water or in a matrix or medium that preferentially mimics the body part. In still some other cases, the duration(s) of the first and/or second step(s) is(are) measured during the first sequences, preferentially during less than 2, 5, 10, 10², 10³, or 10⁵ sequences that preferentially belong to a treatment session. In still some other cases, the duration(s) of the first and/or second step(s) is(are) measured during the first sessions, preferentially during less than 2, 5, 10, 10², 10³, or 10⁵ sessions. In still some other cases, the duration(s) of the first and/or second step(s) is(are) measured during the first sequences or sessions and the average value(s) of the duration(s) of this/these step(s) is/are estimated. This/these average value(s) is/are preferentially used to carry out sequences or sessions that follow the first sequences or sessions, preferentially without measuring or without needing to measure: i), the duration(s) of the first and/or second step(s), ii), the temperature, or iii), the percentage of dissociation.

In one embodiment of the invention, the first and second steps are repeated, preferentially more than 2, 5, 10, 10², 10³, or 10⁶ times, preferentially during the duration(s) of the first and/or second step(s), which have been measured.

In another embodiment of the invention, the first and second steps are repeated until a therapeutic effect, such as the destruction of the body part or pathological cells, is achieved.

The invention also relates to magnetic nanoparticles, in particular magnetosomes, for use, wherein the parameter of the laser is selected from the group consisting of laser power, power density, intensity, amplitude, strength, frequency, and pulsation time.

In some cases, the laser power corresponds to the power of laser radiation generated by the equipment or apparatus generating laser radiation before laser radiation reaches or travels through the body part. In still some other cases, the laser power corresponds to the power of laser radiation generated by the equipment or apparatus generating laser radiation after or while laser radiation reaches or travels through the body part.

In one embodiment of the invention, the laser power is larger than 10⁻²⁰, 10⁻¹⁰, 10⁻⁵, 10⁻², 10⁻¹, 1, 5, 10, 10², 10³, or 10⁵ W (Watt).

In another embodiment of the invention, the laser power is lower than 10⁻²⁰, 10⁻¹⁰, 10⁻⁵, 10⁻², 10⁻¹, 1, 5, 10, 10², 10³, or 10⁵ W (Watt).

In still another embodiment of the invention, the laser power density is the ratio between the laser power and the volume, surface, length of body part through which laser radiation travels or of body part that laser radiation reaches.

In one embodiment of the invention, the laser power density is larger than 10⁻²⁰, 10⁻¹⁰, 10⁻⁵, 10⁻², 10⁻¹, 1, 5, 10, 10², 10³, or 10⁵ W per cm³ of body part.

In another embodiment of the invention, the laser intensity is lower than 10⁻²⁰, 10⁻¹⁰, 10⁻⁵, 10⁻², 10⁻¹, 1, 5, 10, 10², 10³, 10⁵, 10¹⁰, or 10¹⁰ mA

In one embodiment of the invention, the laser intensity is larger than 10⁻²⁰, 10⁻¹⁰, 10⁻⁵, 10⁻², 10⁻¹, 1, 5, 10, 10², 10³, 10⁵, 10¹⁰ mA

In some cases, a similar temperature increase or percentage of dissociation can be reached by increasing the laser power, laser power density, or laser intensity, preferentially by a factor of more than 1.001, 1.01, 1.1, 1.2, 1.5, 2, 5, 10, 10³, 10⁶, or 10⁹, while decreasing the magnetic nanoparticle, in particular magnetosome, concentration, preferentially in the body part, preferentially by a factor of more than 1.001, 1.01, 1.1, 1.2, 1.5, 2, 5, 10, 10³, 10⁶, or 10⁹.

In some other cases, a similar temperature increase or percentage of dissociation can be reached by decreasing the laser power, laser power density, or laser intensity, preferentially by a factor of more than 1.001, 1.01, 1.1, 1.2, 1.5, 2, 5, 10, 10³, 10⁶, or 10⁹, while increasing the concentration of the magnetic nanoparticle, in particular magnetosome, preferentially in the body part, preferentially by a factor of more than 1.001, 1.01, 1.1, 1.2, 1.5, 2, 5, 10, 10³, 10⁶, or 10⁹.

In some cases, the laser amplitude can be or be proportional to the amplitude of the laser radiation or of the electromagnetic wave associated with the laser radiation, preferentially measured at a given time of the oscillation of this wave or of laser radiation, or preferentially measured at a given wavelength of the laser radiation. In some cases, the laser amplitude can correspond to or be or be proportional to the laser intensity or laser power.

In some cases, the laser frequency can be the frequency of oscillation of the electromagnetic wave associated with the laser radiation, or can be the frequency at which the laser radiation is generated, or is generated with sufficient intensity, power, or amplitude. The laser frequency is preferentially associated with a temporal or spatial laser frequency. In some cases, the laser frequency can be lower than 10²⁰, 10¹⁵, 10¹², 10¹⁰, 10⁸, 10⁶, 10⁵, 10³, 10², 10, or 1 Hz. In some other cases, the frequency can be larger than 10²⁰, 10¹⁵, 10¹², 10¹⁰, 10⁸, 10⁶, 10⁵, 10³, 10², 10, or 1 Hz. In still some other cases, the frequency can be between 1 and 10²⁰, 10³ and 10¹⁵, or between 10⁸ and 10¹² Hz.

In some cases, the laser pulsation time can be the time of pulsation of the laser radiation. In some cases, it can be lower than 10³, 10⁻¹, 10⁻¹, 10⁻³, 10⁻⁵, 10⁻¹⁰, 10⁻¹², 10⁻¹⁵, 10⁻²⁰, 10⁻⁴⁰ second(s). In some other cases, it can be larger than 10³, 10⁻¹, 10⁻¹, 10⁻³, 10⁻⁵, 10⁻¹⁰, 10⁻¹², 10⁻¹⁵, 10⁻²⁰, 10⁻⁴⁰ second(s). In still some other cases, it can be between 10⁻⁴⁰ and 10³ sec., between 10⁻²⁰ and 10 sec., between 10⁻²⁰ and 10⁻³ sec., or between 10⁻¹⁰ and 10⁻²⁰ sec.

The invention also relates to magnetic nanoparticles, in particular magnetosomes, for use, for the prevention or treatment of a disease selected from a cancer, a tumor, and an infection.

In one embodiment of the invention, a disease is a health condition of an individual in which the body part comprises more than 1, 10, 10³, 10⁶, 10⁹, 10¹⁵, or 10²⁰ pathological cells or less than 1, 10, 10³, 10⁶, 10⁹, 10¹⁵, or 10²⁰ healthy cells. In some cases, a disease can be a health condition of an individual in which the body part malfunctions or does not work or function as in a healthy individual. In still some other cases, a disease can be characterized by an increase of the number of pathological cells in the individual by a factor of more than 1.001, 1.1, 1.2, 1.5, 10, 10³, 10⁶, 10⁹, or 10²⁰, compared with the number of pathological cells in this individual before he had the disease or in another individual without the disease.

In one embodiment of the invention, the disease is a disorder or malfunction of the body part, of an infectious disease, an auto-immune disease, a neuropathology, a cancer, a cutaneous condition, an endocrine disease, an eye disease or disorder, an intestinal disease, a communication disorder, a genetic disorder, a neurological disorder, a voice disorder, a vulvovaginal disorder, a liver disorder, a heart disorder, a heating disorder, a mood disorder, or a personality disorder.

In one embodiment of the invention, the disease is a brain tumor, cervical cancer, colorectal cancer, cutaneous tumor, endometrial cancer, stomach cancer, liver cancer, gastrointestinal stromal tumor, malignant hemopathy, leukemia, multiple myeloma, lymphoma, Hodgkin's disease, non-Hodgkin's lymphoma, hepatocellular carcinoma, Kaposi's sarcoma, laryngeal cancer, mesothelioma, cancer of the esophagus, osteosarcoma, ovarian cancer, pancreatic cancer, skin cancer, oral cancer, lung cancer, small cell lung carcinoma, prostate cancer, rhabdomyosarcoma, kidney cancer, breast cancer, testicular cancer, thyroid cancer, soft tissue sarcoma, bladder carcinoma, myeloma (bone cancer), plasmacytoma, myeloma, germ cell cancer, neuroblastoma, osteosarcoma, retinoblastoma, cancer of the central nervous system, wilms tumor or nephroblastoma.

The invention also relates to magnetic nanoparticles, in particular magnetosomes, for use in a cosmetic composition or cosmetic treatment.

In one embodiment of the invention, a cosmetic composition is a composition that is used in a cosmetic treatment.

In some cases, a cosmetic treatment is used to repair or replace damaged body part, or a body part that comprises more than 1, 10, 10³, 10⁶, or 10⁹ pathological cells.

In some cases, a cosmetic treatment is a treatment that yields a percentage of pathological cells in the body part, which is lower than 10⁻⁶, 10⁻³, 1, 5, 10, 25, 50, 75, or 90 %. This percentage is preferentially the ratio between the number of pathological cells and the total number of the cells in the body part.

In some cases, a cosmetic treatment is a treatment that changes or modifies the color of the body part, for example by making it darker or lighter, by making the body part color switch from an orange, yellow, white, gray, green, blue, or red color, or a mixture of any of these colors without the magnetic nanoparticles, in particular magnetosomes, to another color, orange, yellow, white, gray, green, blue, red color, or a different mixture of these colors with the magnetic nanoparticles, in particular magnetosomes. In some cases, this change in color can be accentuated or more pronounced or different in the presence than in the absence of the laser. In still some other cases, this change in color or can be absent without laser irradiation or without the heat or compound dissociation that it induces and occur in the presence of laser irradiation or in the presence of heat or dissociation of the compound that laser induces.

### DESCRIPTION OF THE FIGURE

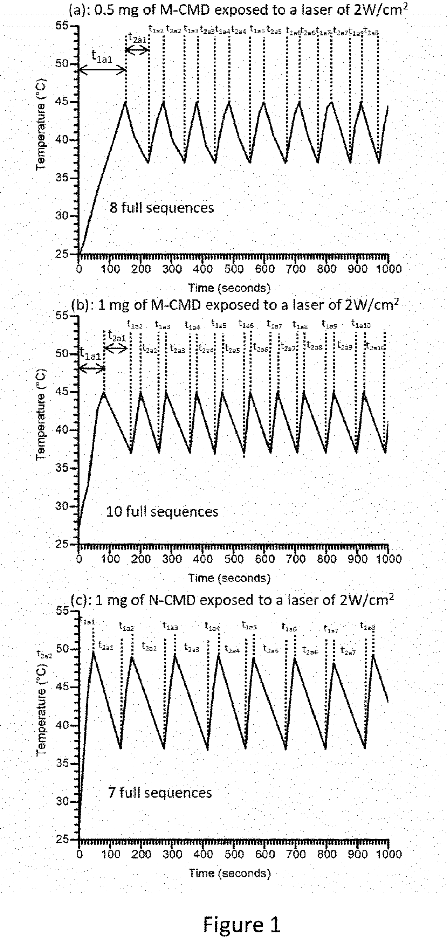

Figure 1: Temperature variations as a function of time of suspensions comprising 0.5 mg of M-CMD, (a), 1 mg of M-CMD, (b), or 1 mg of N-CMD, (c), which are exposed to a laser power density of 2 W/cm² during heating steps of durations t₁ₐᵢ (1<i<10) and not exposed to the laser during cooling steps of duration t₂ₐⱼ (1<j<10).

### EXAMPLE:

### Example 1: Sequential application of a laser on suspensions comprising CMD coated magnetic nanoparticle, in particular magnetosome, minerals and CMD coated chemically synthesized nanoparticles.

### Material and method:

Magnetosome minerals coated with carboxymethyldextran (M-CMD) were prepared according to a protocol slightly modified from that of example 8 of patent PCT/FR2016/000095 incorporated in reference. In this example, the word naked indicates that the nanoparticles are devoid of most carbonaceous material and mainly comprise iron oxide in the form of maghemite.

Briefly, to fabricate M-CMD and N-CMD, we first prepared: i), powder 1 comprising naked magnetosome minerals, *i.e.* comprising the central part of the magnetosomes with a low percentage of carbonaceous material surrounding the magnetosome, mineral core (also designated as magnetosome, central part) as defined and described in patent PCT/FR2016/000095 incorporated in reference (example 4), ii), powder 2 comprising naked chemical iron oxide nanoparticles purchased from Sigma Aldrich (reference Sigma: 637106), iii), a solution 2 containing carboxy-methyl-dextran (ref SIGMA ALDRICH: 86524-50G-F; lot #BCBQ7420V). The suspensions 1 and 2 were mixed, using an Iron to CMD molar ratio of 20.

First, we collected 40 mg of naked dried magnetosome mineral powder and naked dried chemical nanoparticle powder (ref Sigma: 637106) using a balance, where these weighted powders comprise 20 mg of iron.

Then, we harvested 400 mg of CMD powder using a balance (corresponding to a mass of CMD 20 times larger than the mass of iron in the nanoparticle powders) and we mixed these 400 mg of CMD in 20 ml of Milli-Q water.

Afterwards, we mixed the two different naked dried nanoparticle powders with CMD solutions and we adjusted the pH of the two resulting suspensions to 4.65 with a 2M hydrochloric acid solution. We sonicated these suspensions using a sonic bath (reference Transsonic T460: FY 7864596; frequency: 50-60 Hz; power: 67 W) during 15 hours.

After sonication, we did a first washing by centrifugation in eppendorf tubes (14500 rpm) for 10 minutes. Then, we removed with a pipette the coating in excess from the eppendorf, contained in the supernatant of coated nanoparticles. We added 2 ml of Milli Q water to the pellet contained the coated nanoparticles and repeated centrifugation again (14500 rpm), removal of supernate, and re-suspension in water. We then obtained M-CMD and N-CMD suspensions.

Finally, we used an iron dosage to estimate the iron concentration of the N-CMD and M-CMD suspensions. The concentration of the two suspensions of CMD coated nanoparticles mixed in water was adjusted to 0.5 mg/mL and 1 mg/mL for M-CMD and 1 mg/mL for N-CMD.

N-CMD and M-CMD were determined to be mainly composed of a core of maghemite, to have an average size between 35 and 41 nm, respectively, to have a distribution in sizes comprised between 10 and 75 nm for N-CMD and between 20 and 75 nm for M-CMD and to have a stability of 100% for both types of nanoparticles (table 1). To determine the stability of both types of nanoparticles, we measured the percentage of absorption decrease, measured at 480 nm, of 1 mL of suspensions comprising 1 mg/mL of both types of nanoparticles between the time of nanoparticle homogenization by hand checking and 12 hours afterwards.

The suspensions of N-CMD (100 µg of N-CMD mixed in water introduced in a 100 µl well) and M-CMD (50 µg or 100 µg of M-CMD mixed in water introduced in a 100 µL well) were exposed (or not) to a laser (reference: SHANGHAI DREAM LASER TECHNOLOGY : SDL-LM-5000T), which was connected to an optical fiber (diameter: 0.5 mm, reference: SMA905/LD80) that transported laser radiation from the laser equipment or unit to a fixed position, which is 1 cm away from the bottom of the well comprising the nanoparticle suspensions. The power of the laser was measured using a power meter (Spectra Physics: Model 407A) with a distance of 1 cm between the fiber end and the power meter detector surface. We made a calibration curve to relate the laser intensity to the laser power, measured 1 cm away from the fiber end. The power density of the laser was measured by dividing the power of the laser estimated 1 cm away from the fiber end and the surface of illumination of the laser, which was estimated by eyes as corresponding to an illuminated surface area of 1 cm². For the experiments, the laser power density was estimated as 2 W/cm² when the laser was switched on. This laser power density was chosen because it produced a temperature increase in the presence of M-CMD (0.5 and 1 mg/mL) and N-CMD (1 mg/mL), while it did induce a low temperature increase in the absence of the nanoparticles (∼ 0.007 °C/ sec.)

To irradiate (or not) the nanoparticle suspensions with laser radiation, we prepared suspensions comprising 100 µL of N-CMD or M-CMD at 1 mg/ml in iron that we inserted in wells of 96 microwell plate. We measured the temperature within the wells using an infrared camera and the duration of the different steps using a timer.

To irradiate (or not) the nanoparticle suspensions with laser radiation, we prepared suspensions comprising 100 µL of N-CMD or M-CMD at 1 mg/ml in iron that we inserted in wells of 96 microwell plate. We measured the temperature within the wells using an infrared camera and the duration of the different steps using a timer.

We carried out a series of heating steps by exposing the suspension of M-CMD and N-CMD to the laser of power density 2 W/cm². We started the heating steps by switching on the laser (the laser intensity is set at 4500 mA). We ended the heated steps by switching off the laser (the laser intensity is set at 0 A) when the temperature reached 45 °C. For N-CMD, when we switched off the laser, the temperature continued to increase up to 49 °C. The heating steps were followed by cooling steps in which suspensions of N-CMD and M-CMD were not exposed to the laser. We started the cooling steps by switching off the laser (the laser intensity is set at 0 A). We ended the cooling steps by switching on the laser (the laser intensity is set at 4500 mA) when the temperature reached 37 °C.

### Results and discussion:

Within a lapse of time of 1000 seconds, we have been able to carry out 8 and 10 sequences, using 0.5 mg/mL and 1 mg/mL of M-CMD, respectively. By increasing the concentration of M-CMD from 0.5 mg/mL to 1 mg/mL, we were able to increase the number of sequences from 8 to 10 (Figure 1). Within a lapse of time of 1000 seconds, we have been able to carry out 10 sequences with 1 mg of M-CMD and 7 sequences by using N-CMD. By using the magnetic nanoparticle, in particular magnetosome, minerals coated with CMD we were able to increase the number of sequences compared with chemically synthesized nanoparticles coated with M-CMD (Figure 1).

Considering the heating steps following the first one, for the concentration of 0.5 mg/mL of M-CMD, the heating times were between 40 sec. and 50 sec., while the cooling times were between 60 sec. and 75 sec (table 2). For the first sequence, the heating step lasts 150 sec., twice longer than the duration of the cooling step of 75 sec. (table 2). For the other sequences, the heating times were shorter than the cooling times, with a ratio between the cooling and the heating times comprised between 1.33 and 1.71 (table 2).

For the concentration of 1 mg/mL of M-CMD, the heating times were between 20 sec. and 80 sec (table 2). They were between 1.6 and 2.2 times shorter than the heating times measured for 0.5 mg of M-CMD at the same sequence numbers (table 2). The cooling times were between 65 sec. and 90 sec., while the cooling times were between 1 min and 1 min 15 s. By increasing the concentration of M-CMD from 0.5 mg/mL to 1 mg/mL, we: i), decreased the duration of the heating steps by a factor comprised between 1.6 and 2.2 depending on the heating step, and ii), increased the duration of the cooling steps by a factor comprised between 1.01 and 1.2.

For the concentration of 1 mg/mL of N-CMD, the heating times were between 22 sec. and 45 sec., while the cooling times were between 89 sec. and 105 sec (table 2). Compared with 1 mg of M-CMD, the heating times of N-CMD were either shorter or longer than the heating time of M-CMD depending on the sequence with a ratio between the heating times of N-CMD and those of M-CMD comprised between 0.6 and 1.8 (table 2). Compared with 1 mg of M-CMD, the cooling times were globally larger with 1 mg of N-CMD with a ratio between the cooling times of N-CMD and those of M-CMD comprised between 0.99 and 1.5 (table 2). The longer cooling times observed with N-CMD could explain why within we could carry out a lower number of sequences per unit time (1000 seconds for example) with N-CMD than with M-CMD.

For the concentration of 0.5 mg/mL of M-CMD, the ratio between the temperature increase of the heating step and the heating time was comprised between 0.14 °C/sec. and 0.24 °C/sec, while the ratio between the temperature decrease of the cooling step and the cooling time was comprised between 0.11 °C/sec. and 0.13 °C/sec (table 3). This suggests that the average rate at which the temperature increases during the heating step or the average temperature gradient of the heating step is larger than the average rate at which temperature decreases during the cooling step or the average temperature gradient of the cooling step.

For the concentration of 1 mg/mL of M-CMD, the ratio between the temperature increase of the heating step and the heating time was comprised between 0.22 °C/sec. and 0.4 °C/sec, while the ratio between the temperature decrease of the cooling step and the cooling time was comprised between 0.09 °C/sec. and 0.12 °C/sec (table 3). Compared with 0.5 mg/mL of M-CMD, the ratio between the temperature increase of the heating step and the heating time increased by a factor comprised between 1.6 and 2 for 1 mg/mL of M-CMD. This suggests that the rate at which temperature increases during the heating step is more important at 1 mg than at 0.5 mg of M-CMD. Compared with 0.5 mg/mL of M-CMD, the ratio between the temperature decrease of the cooling step and the cooling time decreased by a factor of 1.1-1.2 (table 3). This suggests that the rate at which temperature decreases is more important at 0.5 mg than at 1 mg of M-CMD.

For the concentration of 1 mg/mL of N-CMD, the ratio between the temperature increase of the heating step and the heating time was comprised between 0.33 °C/sec. and 0.56 °C/sec, while the ratio between the temperature decrease of the cooling step and the cooling time was comprised between 0.11 °C/sec. and 0.14 °C/sec (table 3). Between 1 mg of M-CMD and 1 mg of N-CMD, the ratio between the temperature increase of the heating step and the heating time increased (except for the third step) by a factor comprised between 1.02 and 2.3. This increase could be explained by the maximum temperature reached at the end of each heating step of 49 °C, which is 4°C above the desired temperature of 45 °C (table 6 and Figure 1). Between 1 mg of M-CMD and 1 mg of N-CMD, the ratio between the temperature decrease of the cooling step and the cooling time did not change very significantly as a whole (table 3). Even so there is an increase in the rate of temperature increase for 1 mg/mL of N-CMD, it does not result as a whole in a larger number of sequences since the maximum temperature reached during treatment is higher than the desired temperature.

Interesting, the heating times were the same as the durations of irradiation by the laser radiation during the heating steps and the cooling times were the same as the durations of non-irradiation by the laser radiation during the cooling steps for 0.5 mg/mL and 1 mg/mL of M-CMD (tables 2 and 4). By contrast, for 1 mg/mL of N-CMD, the heating times were larger by a factor comprised between 1.4 and 1.8 than the durations of irradiation by the laser radiation during the heating steps and the cooling times were larger by a factor comprised between 1.1 and 1.3 than the durations of non-irradiation by the laser radiation during the cooling steps (tables 2 and 4).

Consequently, for 0.5 mg and 1 mg of M-CMD, we found that: i), the ratio between the temperature increases during the heating steps and the heating times were the same as the ratio between the temperature increases during the heating steps and the duration of irradiation by the laser radiation during the various heating sequences, and ii), the ratio between the temperature decreases during the cooling steps and the cooling times were the same as the ratio between the temperature decreases during the cooling steps and the duration of non-irradiation by the laser radiation during the various cooling sequences, (tables 3 and 5). By contrast, for 1 mg/mL of N-CMD, we found that: i), the ratio between the temperature increases during the heating steps and the heating times were lower than the the ratio between the temperature increases during the heating steps and the duration of irradiation by the laser radiation during the various heating sequences, and ii), the ratio between the temperature decreases during the cooling steps and the cooling times were larger than the ratio between the temperature decreases during the cooling steps and the duration of non-irradiation by the laser radiation during the various cooling sequences. The behavior observed with N-CMD could be explained by the fact that N-CMD continue to produce heat after the laser has been switched off at the end of each heating step, hence increasing the heating time and decreasing the cooling time.

### We can draw the following conclusions from this example:

i) By increasing the concentration of M-CMD irradiated by laser radiation from 0.5 mg/mL to 1 mg/mL, we could increase the number of sequences from 8 to 10 within 1000 sec. of laser radiation application. This suggests that the number of sequences per unit time can be adjusted by varying magnetic nanoparticle, in particular magnetosome, concentration, preferentially can be increased by increasing magnetic nanoparticle, in particular magnetosome, concentration.
ii) At equivalent concentration of 1 mg/mL and for the same laser power density of 2 W/cm², M-CMD led to a larger number of sequences of 10 within 1000 seconds compared with N-CMD that resulted in 7 sequences within 1000 seconds of irradiation by laser radiation. This suggests that M-CMD can produce a larger number of sequences per unit time than N-CMD.
iii) Increasing the concentration of M-CMD irradiated by laser radiation of 2 W/cm² from 0.5 mg/mL to 1 mg/mL resulted in a decrease in heating time by a factor comprised between 1.6 and 2.2 and in an increase in cooling time by a factor comprised between 1.01 and 1.2. This suggests that the heating and cooling times can be tuned by varying the magnetic nanoparticle, in particular magnetosome, concentration, and that optimal magnetic nanoparticle, in particular magnetosome, concentration can correspond to a concentration resulting in the smallest heating times while possibly avoiding a too large increase in cooling times.
iv) The rate at which temperature increases during the heating steps is larger for 1 mg/mL than for 0.5 mg/mL of M-CMD irradiated by the laser radiation, while the rate at which temperature decreases during the cooling steps is larger for 0.5 mg/mL than for 1 mg/mL of M-CMD irradiated by the laser radiation. This suggests that increasing the magnetic nanoparticle, in particular magnetosome, concentration decreases the heating time and increases the cooling time, possibly due a different mechanism of heat transfer during the heating and cooling steps. During the heating steps, laser radiations can be directly or immediately coupled to or absorbed by magnetosomes, resulting in immediate heating. It can result to a rate of temperature increase that increases with increasing magnetic nanoparticle, in particular magnetosome, concentration. During the cooling steps, heat may be transferred from the magnetosomes to their surrounding such as body part at a rate or speed that decreases with increasing magnetosome concentration, possibly due to the fact that with increasing magnetosome concentration, heat is maintained for a longer period of time in the magnetosomes without being transferred to the surrounding medium or body part of the magnetosomes.
v) Interestingly, for both 0.5 mg/mL and 1 mg/mL of M-CMD, the heating and cooling times were the same as the durations of application and non-irradiation by the laser radiation, respectively. The desired maximum temperature of the heating steps and minimum temperature of the cooling steps were also the same as the temperatures reached during the heating and cooling steps, respectively. This suggests an optimal coupling between laser radiation and the magnetosomes. This is an advantageous property for the laser medical treatment. On the one hand it enables to reach a maximum temperature during the heating step and minimum temperature during the cooling step that are the same as those that one wants to reach. On the other hand it prevents overheating or overcooling and potential side effects that can result from them.
vi) By contrast, for 1 mg/mL of N-CMD, the heating and cooling times were different from the durations of applications and non-irradiation by the laser radiation, respectively. The desired maximum temperatures of the heating steps (45 °C) were also different from the maximum temperatures reached during the heating steps (49 °C). This suggests non-optimal coupling between laser radiation and the N-CMD. This is a disadvantageous property for the laser medical treatment. On the one hand, it does not enable to reach a maximum temperature during the heating step that is the same as the temperature that one wants to reach. On the other hand, it leads to overheating and potential side effects that can result from it.
vii) For the second to tenth sequence, the duration of sequences (heating step + cooling step), was between 1.3 to 1.5 longer for 1mg/mL of N-CMD than for 1 mg/mL of M-CMD. This explains why we could carry out a lower number of sequences per unit time for N-CMD than for M-CMD (Fig. 1).
viii) The better match between desired and reached maximum/minimum temperatures of the heating/cooling steps, the smaller heating times observed for M-CMD compared with N-CMD could be explained by the better crystallization, more homogenous organization, better faceted, higher coercivity or remanent magnetization, larger size, or lower size distribution, of M-CMD compared with N-CMD. This could also be explained by the arrangement in chains, which is present in M-CMD and absent in N-CMD.

### DESCRIPTION OF THE TABLES

**Table 1:** Properties of N-CMD (Sigma nanoparticles, reference 637106, coated with CMD) and M-CMD (magnetosome minerals coated with CMD) mixed in water; endotoxin concentration in Endotoxin unit (EU) per mL of suspension per mg of nanoparticles in iron; coating thickness; size distribution; mean size of N-CMD and M-CMD in nm as determined by Transmission electron microscopy; hydrodynamic sizes of N-CMD and M-CMD determined by dynamic light scattering measurements; Isoelectric point and zeta potential of N-CMD and M-CMD mixed in suspension; stability in suspension of N-CMD and M-CMD determined by measuring the percentage of decrease of the absorption at 480 nm of suspensions comprising 1 mg/mL of N-CMD and M-CMD, between the time just after homogenization of these suspensions and 12 hours afterwards.
**Table 2:** Heating times of heating steps and (t_{1ai,} 1 < i < 10) cooling times of cooling steps (t₂ₐᵢ, 1 < i < 10), measured for suspensions comprising 0.5 mg of M-CMD, 1 mg of M-CMD, or 1 mg of N-CMD, which are irradiated by the laser radiation of power density 2 W/cm² during 10 heating steps and not irradiated by the laser radiation during 10 cooling steps.
**Table 3:** Ratio between the variations of temperature of the heating steps and the heating times (t₁ₐᵢ, 1 < i < 10) as well as ratio between the variations of temperature of the cooling step and the cooling times (t₂ₐᵢ, 1 < i < 10), measured for suspensions comprising 0.5 mg of M-CMD, 1 mg of M-CMD, or 1 mg of N-CMD, which are irradiated by the laser radiation of power density 2 W/cm² during 10 heating steps and not irradiated by the laser radiation during 10 cooling steps.
**Table 4:** Durations of irradiation by the laser radiation with power density of 2 W/cm² during the various heating steps (t_{ON-OFFi}, 1 < i < 10), as well as durations of the non-irradiation by the laser radiation during the various cooling steps (t_{OFF-ONi}, 1 < i < 10), measured for suspensions comprising 0.5 mg of M-CMD, 1 mg of M-CMD, or 1 mg of N-CMD, which are irradiated by the laser radiation of power density 2 W/cm² during 10 heating steps and not irradiated by the laser radiation during 10 cooling steps.
**Table 5:** Ratio between the variations of temperature of the heating steps and the durations of irradiation by the laser radiation with power density of 2 W/cm² during the various heating steps (t_{ON-OFFi}, 1 < i < 10). as well as ratio between the variations of temperature of the cooling steps and durations of the non-irradiation by the laser radiation during the various cooling steps (t_{OFF-ONi}, 1 < i < 10), measured for suspensions comprising 0.5 mg of M-CMD, 1 mg of M-CMD, or 1 mg of N-CMD, which are irradiated by the laser radiation of power density 2 W/cm² during 10 heating steps and not irradiated by the laser radiation during 10 cooling steps.
**Table 6:** Desired temperature and average temperature reached during the various steps for suspensions comprising 0.5 mg of M-CMD, 1 mg of M-CMD, or 1 mg of N-CMD, which are irradiated by the laser radiation of power density 2 W/cm².

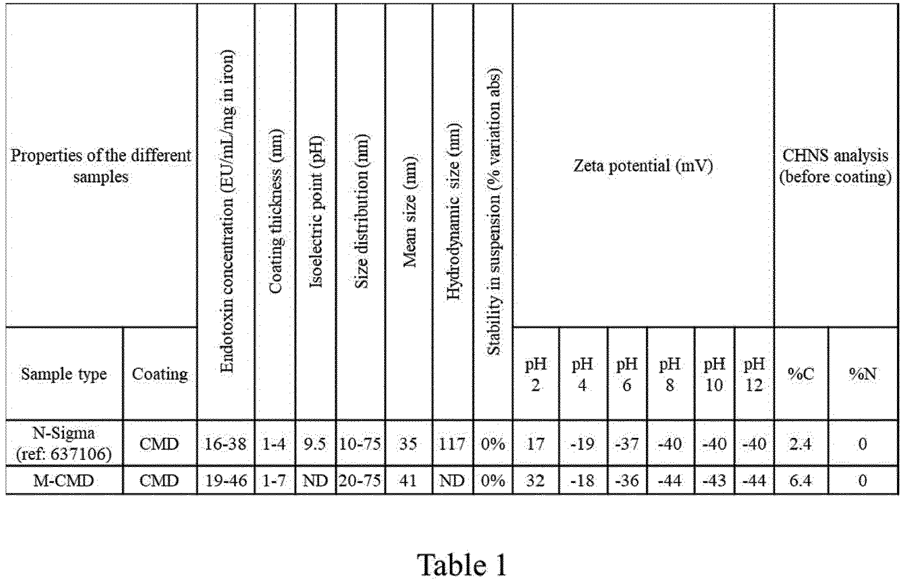

**Table 2**

| | **M-CMD (0.5 mg)** | **M-CMD (1 mg)** | **N-CMD (1 mg)** |
|---|---|---|---|
| t₁ₐ₁ | 150 s | 80 s | 45 s |
| t₂ₐ₁ | 75 s | 90s | 89 s |
| t₁ₐ₂ | 48 s | 30 s | 36 s |
| t₂ₐ₂ | 69 s | 80 s | 105 s |
| t₁ₐ₃ | 39 s | 22 s | 35 s |
| t₂ₐ₃ | 60 s | 77 s | 105 s |
| t₁ₐ₄ | 45 s | 21 s | 35 s |
| t₂ₐ₄ | 69 s | 70 s | 90 s |
| t₁ₐ₅ | 45 s | 24 s | 25 s |
| t₂ₐ₅ | 69 s | 72 s | 105 s |
| t₁ₐ₆ | 45 s | 20 s | 28 s |
| t₂ₐ₆ | 60 s | 65 s | 100 s |
| t₁ₐ₇ | 45 s | 22 s | 27 s |
| t₂ₐ₇ | 60s | 65 s | 100 s |
| t₁ₐ₈ | 35 s | 21s | 26 s |
| t₂ₐ₈ | 60 s | 71s | 101 s |
| t₁ₐ₉ | 37 s | 25 s | 22 s |
| t₂ₐ₉ | 60 s | 75 s | 103 s |
| t₁ₐ₁₀ | 41 s | 22 s | 23 s |
| t₂ₐ₁₀ | 60 s | 70 s | 100 s |

**x)**

**Table 3**

| | **M-CMD (0.5 mg)** | **M-CMD (1 mg)** | **N-CMD (1 mg)** |
|---|---|---|---|
| ΔT/t₁ₐ₁ | 0.14 | 0.22 | 0.51 |
| ΔT/t₂ₐ₁ | 0.11 | 0.09 | 0.14 |
| ΔT/t₂ₐ₂ | 0.17 | 0.27 | 0.33 |
| ΔT/t₂ₐ₂ | 0.11 | 0.1 | 0.11 |
| AT/t₁ₐ₃ | 0.21 | 0.36 | 0.35 |
| ΔT/t₂ₐ₃ | 0.13 | 0.1 | 0.14 |
| ΔT/t₁ₐ₄ | 0.18 | 0.38 | 0.47 |
| ΔT/t₂ₐ₄ | 0.12 | 0.11 | 0.11 |
| ΔT/t₁ₐ₅ | 0.18 | 0.33 | 0.43 |
| ΔT/t₂ₐ₅ | 0.12 | 0.11 | 0.12 |
| ΔT/t₁₈₆ | 0.18 | 0.4 | 0.41 |
| ΔT/t₂ₐ₆ | 0.13 | 0.12 | 0.11 |
| ΔT/t₁ₐ₇ | 0.18 | 0.36 | 0.47 |
| ΔT/t₂ₐ₇ | 0.13 | 0.12 | 0.12 |
| ΔT/t₁ₐ₈ | 0.24 | 0.38 | 0.56 |
| ΔT/t₂ₐ₈ | 0.13 | 0.11 | 0.12 |
| ΔT/t₁ₐ₉ | 0.22 | 0.37 | 0.53 |
| ΔT/t₂ₐ₉ | 0.13 | 0.11 | 0.12 |
| ΔT/t₁ₐ₁₀ | 0.18 | 0.36 | 0.53 |
| ΔT/t₂ₐ₁₀ | 0.13 | 0.11 | 0.11 |

**Table 4**

| | **M-CMD (0.5 mg)** | **M-CMD (1 mg)** | **N-CMD (1 mg)** |
|---|---|---|---|
| t_{ON-OFF1} | 150 s | 80 s | 30 s |
| t_{OFF-ON1} | 75 s | 90 s | 104 s |
| t_{ON-OFF2} | 48 s | 30 s | 20 s |
| t_{OFF-ON2} | 69 s | 80 s | 119 s |
| t_{ON-OFF3} | 39 s | 22 s | 20 s |
| t_{OFF-ON3} | 60 s | 77 s | 120 s |
| t_{ON-OFF4} | 45 s | 21 s | 20 s |
| t_{OFF-ON4} | 69 s | 70 s | 115 s |
| t_{ON-OFF5} | 45 s | 24 s | 15 s |
| t_{OFF-ON5} | 69 s | 72 s | 115 s |
| t_{ON-OFF6} | 45 s | 20 s | 18 s |
| t_{OFF-ON6} | 60s | 65 s | 110 s |
| t_{ON-OFF7} | 45 s | 22 s | 17 s |
| t_{OFF-ON7} | 60 s | 65 s | 110 s |
| t_{ON-OFF8} | 35 s | 21 s | 16 s |
| t_{OFF-ON8} | 60 s | 71 s | 111 s |
| t_{ON-OFF9} | 37 s | 25 s | 16 s |
| t_{OFF-ON9} | 60 s | 75 s | 109 s |
| t_{ON-OFF10} | 41 s | 22 s | 15 s |
| t_{OFF-ON10} | 60 s | 70 s | 108 s |

**Table 5**

| | **M-CMD (0.5 mg)** | **M-CMD (1 mg)** | **N-CMD (1 mg)** |
|---|---|---|---|
| ΔT/t_{ON-OFF1} | 0.14 | 0.22 | 0.61 |
| ΔT/t_{OFF-ON1} | 0.11 | 0.09 | 0.08 |
| ΔT/t_{ON-OFF2} | 0.17 | 0.27 | 0.4 |
| ΔT/t_{OFF-ON2} | 0.11 | 0.1 | 0.07 |
| ΔT/t_{ON-OFF3} | 0.21 | 0.36 | 0.4 |
| ΔT/t_{OFF-ON3} | 0.13 | 0.1 | 0.07 |
| ΔT/t_{ON-OFF4} | 0.18 | 0.38 | 0.4 |
| ΔT/t_{OFF-ON4} | 0.12 | 0.11 | 0.08 |
| ΔT/t_{ON-OFF5} | 0.18 | 0.33 | 0.5 |
| ΔT/t_{OFF-ON5} | 0.12 | 0.11 | 0.07 |
| ΔT/t_{ON-OFF6} | 0.18 | 0.4 | 0.44 |
| ΔT/t_{OFF-ON6} | 0.13 | 0.12 | 0.08 |
| ΔT/t_{ON-OFF7} | 0.18 | 0.36 | 0.47 |
| ΔT/t_{OFF-ON7} | 0.13 | 0.12 | 0.07 |
| ΔT/t_{ON-OFF8} | 0.24 | 0.38 | 0.5 |
| ΔT/t_{OFF-ON8} | 0.13 | 0.11 | 0.07 |
| ΔT/t_{ON-OFF9} | 0.22 | 0.37 | 0.5 |
| ΔT/t_{OFF-ON9} | 0.13 | 0.11 | 0.07 |
| ΔT/t_{ON-OFF10} | 0.18 | 0.36 | 0.5 |
| ΔT/t_{OFF-ON10} | 0.13 | 0.11 | 0.07 |

**Table 6**

| | **Steps** | **Desired temperature (°C)** | **Reached temperature (°C) (averaged over the different steps)** |
|---|---|---|---|
| 0.5 mg M-CMD | Heating steps | 45 °C | 45 °C |
| | Cooling steps | 37 °C | 37°C |
| 1 mg M-CMD | Heating steps | 45 °C | 45 °C |
| | Cooling steps | 37 °C | 37 °C |
| 1 mg N-CMD | Heating steps | 45 °C | 49 °C |
| | Cooling steps | 37 °C | 37 °C |

## Claims

1. Magnetosomes for use in a laser radiation medical treatment, wherein the magnetosomes are administered to a body part of an individual and:
- In a first step, the magnetosomes are irradiated by a laser radiation, and
- In a second step, the magnetosomes are irradiated by a laser radiation of lower power than in the first step or no laser irradiation of the magnetosomes is performed, and
the sequence of the first step and the second step is repeated at least once.

2. Magnetosomes for use according to claim **1,** wherein the first step is a heating step and/or a dissociation step.

3. Magnetosomes for use according to claim **1** or **2,** wherein the first step can be divided between a first sub-step during which a first portion of the body part is irradiated by the laser radiation, and a second sub-step during which temperature increase and/or dissociation of a compound from the magnetosomes occurs in another portion of the body part, which has not been irradiated by the laser radiation during the first sub-step.

4. Magnetosomes for use according to any one of claims **1** to **3,** wherein the second step is a cooling step and/or a non-dissociation step.

5. Magnetosomes for use according to any one of claims **1** to **4,** wherein the heating step produces a temperature increase of more than 1 °C of the body part.

6. Magnetosomes for use according to any one of claims **1** to **5,** wherein the cooling step produces a temperature decrease of more than 1 °C of the body part.

7. Magnetosomes for use according to any one of claims **1** to **6,** wherein the dissociation step produces a percentage of dissociation of the compounds from the magnetosomes, which is larger than 10⁻⁴%.

8. Magnetosomes for use according to any one of claims **1** to **7,** wherein the non-dissociation step produces a percentage of dissociation of the compounds from the magnetosomes, which is lower than 99%.

9. Magnetosomes for use according to any one of claims **1** to **8,** wherein the first and/or second step(s) is/are ended when a given temperature and/or percentage of dissociation is/are reached.

10. Magnetosomes for use according to any one of claims **1** to **9,** wherein the magnetosome concentrations and/or duration(s) of the first and/or second step(s) and/or the laser power or laser power density or laser intensity is/are adjusted to reach the desired variation(s) or gradient(s) of temperature and/or percentage of dissociation occurring during the first and/or second step(s).

11. Magnetosomes for use according to any one of claims **1** to **10,** wherein the duration(s) of the first and/or second step(s) is/are between 10⁻⁹ seconds and 10³ hours.

12. Magnetosomes for use according to any one of claims **1** to **11,** wherein the ratio between the duration of the first step and the duration of the second step is between 10⁻²⁰ and 10²⁰.

13. Magnetosomes for use according to any one of claims **1** to **12,** wherein the application of sequences enables to decrease magnetic nanoparticle diffusion outside of the portion of the body part comprising the magnetosomes.

14. Magnetosomes for use according to any one of claims **1** to **13,** wherein the application of sequences enables to increase the percentage of dissociation of the compound from the magnetosomes and/or to increase the number of temperature gradients or variations and/or to decrease the average temperature reached during treatment.

15. Magnetosomes for use according to any one of claims **1** to **14,** in which the sequences are carried out in such a way that: i), the maximum temperature or maximum percentage of dissociation that one desires to reach in the first step and the minimum temperature or minimum percentage of dissociation that one desires to reach in the second step are determined, ii), a parameter of the laser is set or fixed at a first value to reach the maximum temperature and/or maximum percentage of dissociation in the first step and then a parameter of the laser is set or fixed at a second value to reach the minimum temperature and/or minimum percentage of dissociation in the second step, optionally, iii), the duration(s) of the first and/or second step(s) required to reach these maximum or minimum temperature and/or the maximum or minimum percentage of dissociation are measured, and optionally, iv), the first and/or second step(s) is(are) repeated during the measured duration(s) of the first and/or second step(s).
